# EUROPEAN PATENT APPLICATION

(11) **EP 1 145 713 A1**
(43) Date of publication of application: **17.10.2001**
(21) Application number: 00900346.8
(22) Date of filing: 12.01.2000
(51) Int. Cl.: A61K 31/409, A61K 31/454, A61K 31/5377, A61P 43/00, A61P 35/00, C07D 487/22

(54) **TELOMERASE INHIBITORS**

(30) Priority: 12.01.1999 JP 481399
(71) Applicant: KYOWA HAKKO KOGYO CO., LTD., Chiyoda-ku, Tokyo 100-8185 (JP)
(72) Inventor: YAMASHITA, Yoshinori, Tokyo Res. Lab., Kyowa Hakko, Machida-shi, Tokyo 194-8533 (JP); ASAI, Akira, Tokyo Res. Lab., Kyowa Hakko, Machida-shi, Tokyo 194-8533 (JP); HASEGAWA, Atsuhiro, Tokyo Res. Lab., Kyowa Hakko, Machida-shi, Tokyo 194-8533 (JP); AKINAGA, Shiro, Phar. Res. Inst., Kyowa Hakko, Sunto-gun, Shizuoka 411-8731 (JP); ISHIDA, Hiroyuki, Phar. Res. Inst., Kyowa Hakko, Sunto-gun, Shizuoka 411-8731 (JP); MIZUKAMI, T., Head Office, Kyowa Hakko, Chiyoda-ku, Tokyo 100-8185 (JP); YAMAGUCHI, Hiroyuki, Tokyo Res. Lab., Kyowa Hakko, Machida-shi, Tokyo 194-8533 (JP); AKAMA, Tsutomu, Tokyo Res. Lab., Kyowa Hakko, Machida-shi, Tokyo 194-8533 (JP); ENDO, Kaori, Tokyo Res. Lab., Kyowa Hakko, Machida-shi, Tokyo 194-8533 (JP); NARA, Shinji, Phar. Res. Inst., Kyowa Hakko, Sunto-gun, Shizuoka 411-8731 (JP); KANDA, Yutaka, Phar. Res. Inst., Kyowa Hakko, Sunto-gun, Shizuoka 411-8731 (JP)
(74) Representative: VOSSIUS & PARTNER
(86) International application number: JP0000105
(87) International publication number: WO0041689

(57) **Abstract**

Telomerase inhibitors containing as the active ingredient porphyrin derivatives represented by general formula (I), N-(lower alkyl)-substituted derivatives thereof (wherein the lower alkyl is located on the nitrogen atom of the pyrrole ring of the porphyrin ring) or metal coordinates thereof or pharmacologically acceptable salts thereof: (I) wherein R¹, R², R³, R⁴, R⁵, R⁶, R⁷ and R⁸ are the same or different and each represents hydrogen, formyl, optionally substituted lower alkyl, optionally substituted lower alkenyl, carboxy, optionally substituted lower alkoxycarbonyl, CONR⁹R¹⁰ (wherein R⁹ and R¹⁰ are the same or different and each represents hydrogen or optionally substituted lower alkyl), sulfo, lower alkoxysulfonyl or cyano.

## Description

### TECHNICAL FIELD

The present invention relates to telomerase inhibitors and antitumor agents comprising, as an active ingredient, a porphyrin derivative, an *N*-lower alkyl-substituted compound or metal-coordinated compound thereof, or a pharmaceutically acceptable salt thereof. Furthermore, the present invention relates to porphyrin derivatives having a telomerase inhibitory activity or an antitumor activity, *N*-lower alkyl-substituted compounds or metal-coordinated compounds thereof, or pharmaceutically acceptable salts thereof.

### BACKGROUND ART

A telomere present at the end of the chromosome of eucaryote is known to be an important domain for stabilizing the chromosome, and in a human case, the sequence comprises a repeating sequence of TTAGGG from 5'-end. With a few exceptions, the telomere is usually reduced in a normal cell depending on the division times and when it is shortened to a certain length, the cell becomes a senescence cell and terminates the division (M1 stage). However, when the division terminating mechanism does not normally work owing to the mutation of a tumor suppressor gene such as p53 gene and the like, the cell further repeats the division. As a result, the telomere is shortened to the utmost limit, the chromosome becomes instable, and the cell finally dies (M2 stage) (*Proc. Natl. Acad. Sci. USA, 89:* 10114-10118 (1992) and *Trends in Cell Biology, 5:* 293-297 (1995)).

On the other hand, in 80% or more of cancer cells, an enzyme, telomerase which lengthens telomeres is expressed *(Journal of the NCI, 87*: 884-894 (1995)). The enzyme is a reverse transcriptase which lengthens the telomeres using an RNA as a template, and is composed of RNA (hTR), i.e., the template and a protein (hTERT), i.e., a catalyst subunit. Therefore, in the cancer cells, it is considered that the shortening of the telomeres is inhibited by the action of the telomerase and the telomeres are kept stable, so that the infinite proliferation of the cancer cells as immortal cells becomes possible.

This concept, i.e., "telomere hypothesis" was proposed by Calvin Harley *et al.* in 1992 (*Proc. Natl. Acad. Sci. USA*, *89*: 10114-10118 (1992)). As the experimental results for proving the telomere hypothesis, it has been reported that the expression of an antisense toward hTR (*Science*, *269:* 1236-1240 (1995)) and that of a dominant negative variant hTERT inhibiting wildtype telomerase *(Genes & Development, 13:* 2388-2399 (1999) and *Nature* *Medicine, 5*: 1164-1170 (1999)) induce cell death of cancer cells along with the shortening of telomeres. Accordingly, the substance selectively inhibiting telomerase is considered to be a new type of an antitumor agent which imparts a limited life span to cancer cells through the induction of the shortening of telomere. Furthermore, since telomerase is expressed only in cancer tissues with a few exceptions such as germ cells, the substance is expected to be a low toxic antitumor agent which hardly affects normal tissues.

As low molecular weight substances inhibiting telomerase *in vitro,* nucleic acid analogs such as AZTTP, ddGTP (*Mol*. *Cell. Biol., 16:* 53-65 (1996)), 7-deaza-dGTP (*Biochemistry, 35:* 15611-15617 (1996)) and the like, pyridothiophene compounds and the like (U.S. Patents 5,760,062, 5,767,278, 5,770,613, 5,703,116, and 5,656,638) are known. Moreover, it is reported that catechins, i.e., components of tea, exhibit the telomerase inhibitory activity and induce the shortening of telomeres in cancer cells (*Biochem. Biophys. Res. Commun., 249:* 391-396 (1998)). Furthermore, 2,6-diamidoanthraquinone (*J. Med. Chem*., *40*: 2113-2116 (1997)) and TMPyP4 having pyridine rings at α-, β-, γ- and δ-positions of a porphyrin ring (*J. Am. Chem. Soc., 120:* 3261-3262 (1998) and WO98/33503) are reported. It is reported that the latter inhibits telomerase by selectively binding to a unique high-order structure (quadruple strand structure) which telomere DNA has. However, since TMPyP4 inhibits telomerase inside cancer cells but does not induce the shortening of telomeres owing to the low activity (*J*. *Am. Chem. Soc., 121:* 3561-3270 (1999) and WO98/33503), it is unclear whether the compound exhibits medicinal effects as an antitumor agent acting on telomerase which induces the shortening of telomeres and inhibits the proliferation of cancer cells.

In addition, an antitumor agent comprising a porphyrin derivative as the active ingredient is disclosed in Japanese Published Examined Patent Application No. 52124/85 and Japanese Published Unexamined Patent Application No. 109929/78.

### DISCLOSURE OF THE INVENTION

An object of the present invention is to provide telomerase inhibitors and antitumor agents comprising, as an active ingredient, a porphyrin derivative, an *N*-lower alkyl-substituted compound or metal-coordinated compound thereof, or a pharmaceutically acceptable salt thereof. Furthermore, another object is to provide porphyrin derivatives having a telomerase inhibitory activity or an antitumor activity, *N*-lower alkyl-substituted compounds or metal-coordinated compounds thereof, or pharmaceutically acceptable salts thereof.

The present invention relates to telomerase inhibitors comprising, as an active ingredient, a porphyrin derivative represented by formula (I): 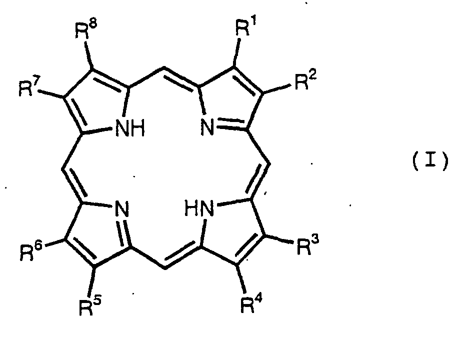 (wherein R¹, R², R³, R⁴, R⁵, R⁶, R⁷ and R⁸ are the same or different and each represents hydrogen, formyl, substituted or unsubstituted lower alkyl, substituted or unsubstituted lower alkenyl, carboxy, substituted or unsubstituted lower alkoxycarbonyl, CONR⁹R¹⁰ (wherein R⁹ and R¹⁰ are the same or different and each represents hydrogen or substituted or unsubstituted lower alkyl), sulfo, lower alkoxysulfonyl or cyano (hereinafter, the compound represented by formula (I) is referred to as Compound (I); Compounds of other formula numbers are also called similarly)), an *N*-lower alkyl-substituted compound thereof (the lower alkyl of the *N*-lower alkyl-substituted compound is present on the nitrogen atom of the pyrrole ring in the porphyrin ring) or metal-coordinated compound thereof, or a pharmaceutically acceptable salt thereof.

Moreover, the present invention provides antitumor agents comprising, as an active ingredient, Compound (I), an *N*-lower alkyl-substituted compound thereof (the lower alkyl of the *N*-lower alkyl-substituted compound is present on the nitrogen atom of the pyrrole ring in the porphyrin ring) or metal-coordinated compound thereof, or a pharmaceutically acceptable salt thereof.

Among the above telomerase inhibitors or antitumor agents, preferred are telomerase inhibitors or antitumor agents comprising, as an active ingredient, an *N*-lower alkyl-substituted compound (the lower alkyl of the *N*-lower alkyl-substituted compound is present on the nitrogen atom of the pyrrole ring in the porphyrin ring) or metal-coordinated compound of Compound (I), or a pharmaceutically acceptable salt thereof.

From another aspect, among compounds (I), the present invention relates to porphyrin derivatives represented by formula (II): 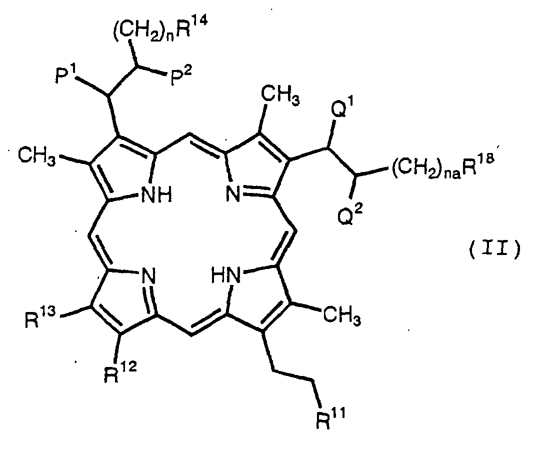 (wherein
n represents an integer of 0 to 10;
na has the same meaning as n described above;
P¹, P², Q¹ and Q² each represents a hydrogen atom, P¹ and P² are combined to represent a bond, or Q¹ and Q² are combined to represent a bond;
R¹¹ represents formyl, CO₂R¹⁵ (wherein R¹⁵ represents hydrogen, substituted or unsubstituted lower alkyl, substituted or unsubstituted lower alkenyl or substituted or unsubstituted aralkyl), CONR¹⁶R¹⁷ (wherein R¹⁶ represents hydrogen, substituted or unsubstituted lower alkyl, substituted or unsubstituted lower alkenyl, substituted or unsubstituted aralkyl, substituted or unsubstituted lower alkoxy, substituted or unsubstituted aryl or substituted or unsubstituted heteroaryl; R¹⁷ represents lower alkyl, substituted or unsubstituted lower alkenyl or substituted or unsubstituted aralkyl, or R¹⁶ and R¹⁷ are combined with the adjacent N to form a heterocyclic group), CH₂NR^{16b}R^{17b} (wherein R^{16b} and R^{17b} are combined with the adjacent N to form a heterocyclic group) or CH₂OR¹⁹ (wherein R¹⁹ represents hydrogen, substituted or unsubstituted lower alkyl, substituted or unsubstituted aralkyl, substituted or unsubstituted lower alkanoyl, substituted or unsubstituted aryl, substituted or unsubstituted aroyl, SO₂R²⁰ (wherein R²⁰ represents substituted or unsubstituted lower alkyl or substituted or unsubstituted aryl) or Si(R²¹)₃ (wherein R²¹'s are the same or different and each represents substituted or unsubstituted lower alkyl or substituted or unsubstituted aryl));
R¹² represents methyl and R¹³ represents (CH₂)₂R²² (wherein R²² has the same meaning as R¹¹ described above), or R¹² represents (CH₂)₂R²³ (wherein R²³ has the same meaning as R¹¹ described above) and R¹³ represents methyl;
R¹⁴ represents formyl, CONR²⁴R²⁵ (wherein R²⁴ and R²⁵ have the same meanings as R¹⁶ and R¹⁷ described above, respectively) or CH₂OR²⁶ (wherein R²⁶ represents substituted or unsubstituted lower alkyl, substituted or unsubstituted aralkyl, substituted or unsubstituted lower alkanoyl, substituted or unsubstituted aryl, substituted or unsubstituted aroyl, SO₂R²⁷ (wherein R²⁷ has the same meaning as R²⁰ described above) or Si(R²⁸)₃ (wherein R²⁸ has the same meaning as R²¹ described above));
R¹⁸ has the same meaning as R¹⁴ described above; and
   1) when R¹¹ represents CONR¹⁶R¹⁷ or CH₂OR¹⁹, and R²² represents CONR¹⁶R¹⁷ or CH₂OR¹⁹, or
      when R¹¹ represents CONR¹⁶R¹⁷ or CH₂OR¹⁹, and R²³ represents CONR¹⁶R¹⁷ or CH₂OR¹⁹,
      R¹⁴ and R¹⁸ are the same or different, and may represent CH₂OH, CO₂R³² (wherein R³² has the same meaning as R¹⁵ described above) or substituted or unsubstituted aryl, and
   2) when R¹¹ represents CO₂R¹⁵, and P¹, P², Q¹ and Q² each represents hydrogen, and R²² represents CO₂R¹⁵, or
      when R¹¹ represents CO₂R¹⁵, and P¹, P², Q¹ and Q² each represents hydrogen, and R²³ represents CO₂R¹⁵,
      R¹⁴ and R¹⁸ are the same or different, and may represent substituted or unsubstituted aryl);
      *N*-lower alkyl-substituted compounds thereof (the lower alkyl of the *N*-lower alkyl-substituted compounds is present on the nitrogen atom of the pyrrole ring in the porphyrin ring) or metal-coordinated compounds thereof, or pharmaceutically acceptable salts thereof.

Furthermore, among Compounds (II), other embodiments include porphyrin derivatives represented by formula (IIA): 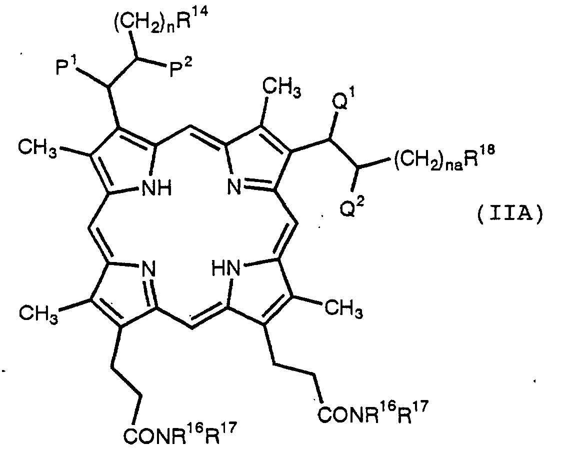 (wherein
n, na, P¹, P², Q¹, Q², R¹⁴, R¹⁶, R¹⁷ and R¹⁸ have the same meanings as described above),
*N*-lower alkyl-substituted compounds thereof (the lower alkyl of the *N*-lower alkyl-substituted compounds is present on the nitrogen atom of the pyrrole ring in the porphyrin ring) or metal-coordinated compounds thereof, or pharmaceutically acceptable salts thereof;
porphyrin derivatives represented by formula (IIB): 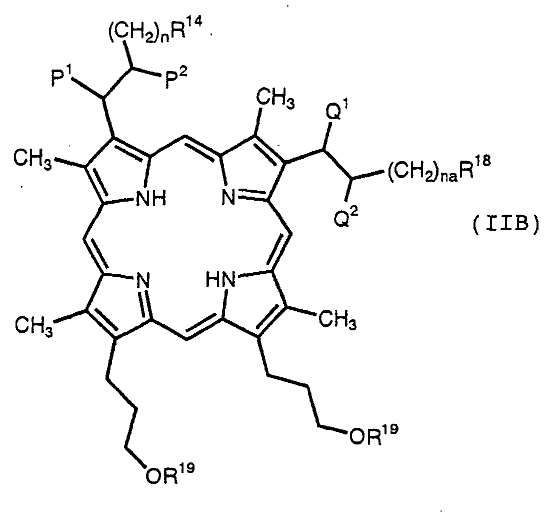 (wherein n, na, P¹, P², Q¹, Q², R¹⁴, R¹⁸ and R¹⁹ have the same meanings as described above),
*N*-lower alkyl-substituted compounds thereof (the lower alkyl of the *N*-lower alkyl-substituted compounds is present on the nitrogen atom of the pyrrole ring in the porphyrin ring) or metal-coordinated compounds thereof, or pharmaceutically acceptable salts thereof; and
porphyrin derivatives represented by formula (IIC): 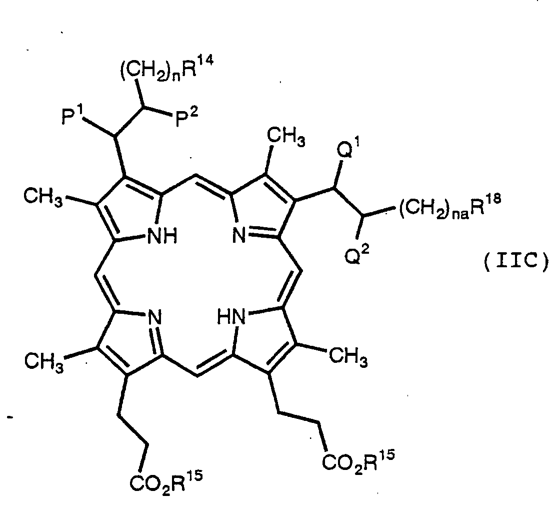 (wherein n, na, P¹, P², Q¹, Q², R¹⁴, R¹⁵ and R¹⁸ have the same meanings as described above),
*N*-lower alkyl-substituted compounds thereof (the lower alkyl of the *N*-lower alkyl-substituted compounds is present on the nitrogen atom of the pyrrole ring in the porphyrin ring) or metal-coordinated compounds thereof, or pharmaceutically acceptable salts thereof.

From further other aspect, among Compounds (I), the present invention relates to porphyrin derivatives represented by formula (III): 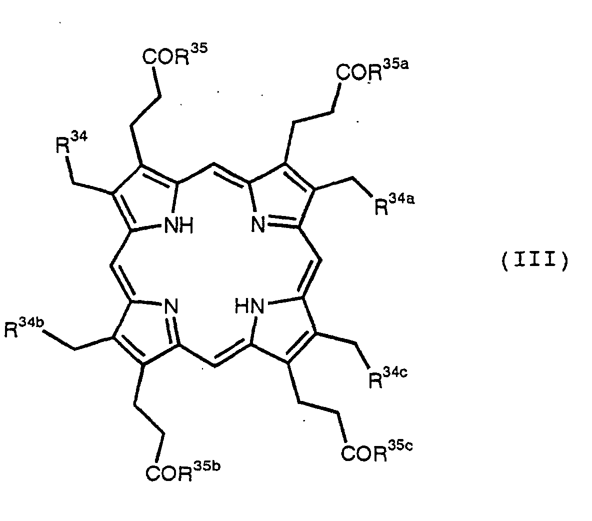 (wherein
R³⁴, R^{34a}, R^{34b} and R^{34c} are the same or different and each represents lower alkyl;
R³⁵, R^{35a}, R^{35b} and R^{35c} are the same or different and each represents OR³⁶ (wherein R³⁶ has the same meaning as R¹⁵ described above) or N(R³⁷)₂ (wherein R³⁷'s are the same or different and each represents hydrogen, substituted or unsubstituted lower alkyl, substituted or unsubstituted lower alkoxy, substituted or unsubstituted aralkyl or substituted or unsubstituted aryl, or two R³⁷'s are combined with the adjacent N to form a substituted or unsubstituted heterocyclic group)),
*N*-lower alkyl-substituted compounds thereof (the lower alkyl of the *N*-lower alkyl-substituted compounds is present on the nitrogen atom of the pyrrole ring in the porphyrin ring) or metal-coordinated compounds thereof, or pharmaceutically acceptable salts thereof.

Among Compounds (III), another embodiment includes porphyrin derivatives represented by formula (IIIA): 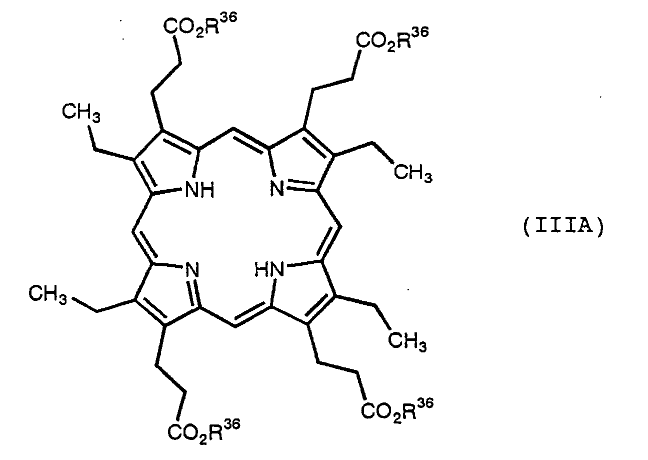 (wherein
R³⁶ has the same meaning as described above),
*N*-lower alkyl-substituted compounds thereof (the lower alkyl of the *N*-lower alkyl-substituted compounds is present on the nitrogen atom of the pyrrole ring in the porphyrin ring) or metal-coordinated compounds thereof, or pharmaceutically acceptable salts thereof.

In the above compounds, preferred are porphyrin derivatives represented by the following Compound Nos. 65 to 112, *N*-lower alkyl-substituted compounds thereof (the lower alkyl of each *N*-lower alkyl-substituted compounds is present on the nitrogen atom of the pyrrole ring in the porphyrin ring) or metal-coordinated compounds thereof, or pharmaceutically acceptable salts thereof.

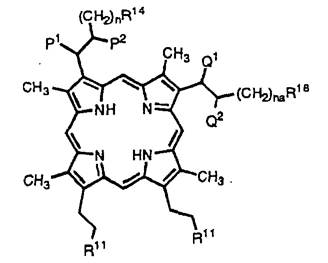

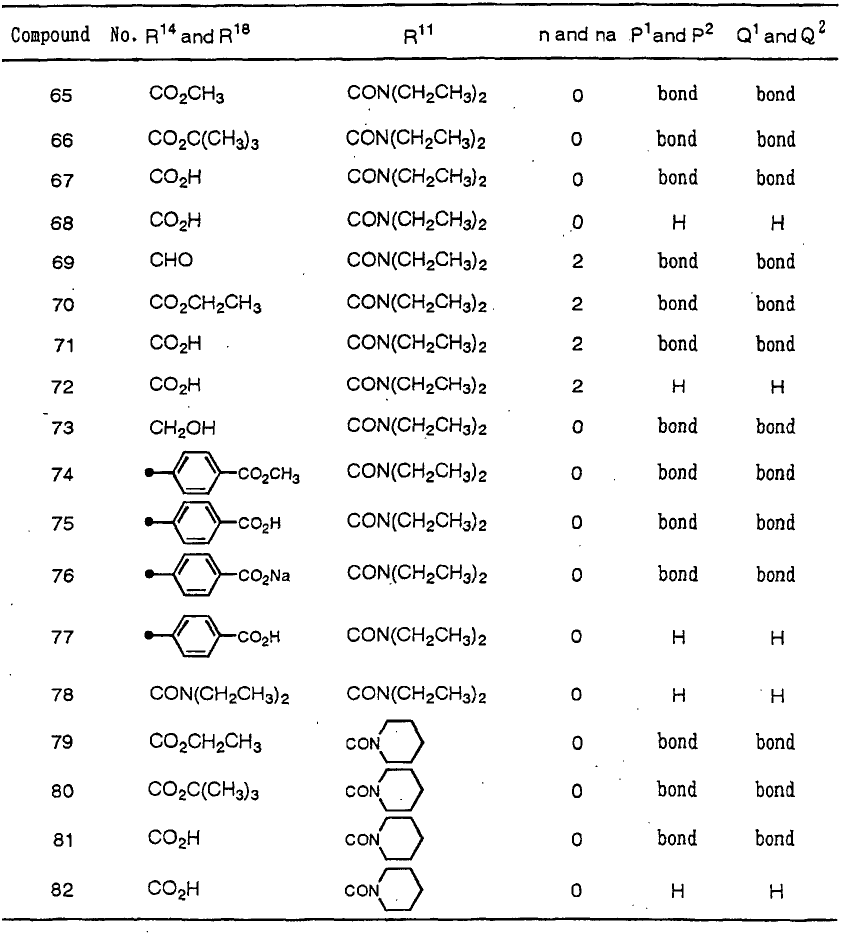

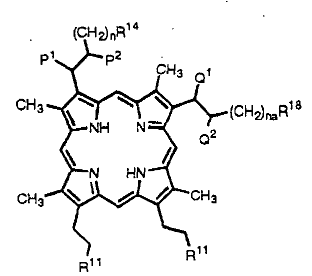

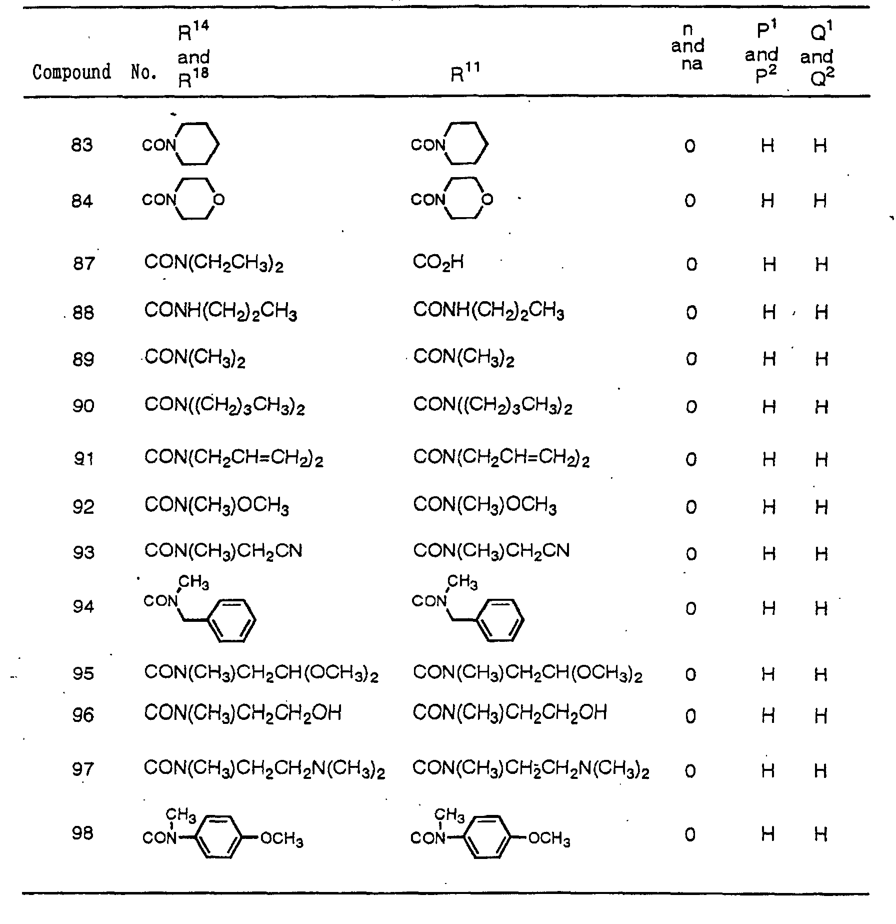

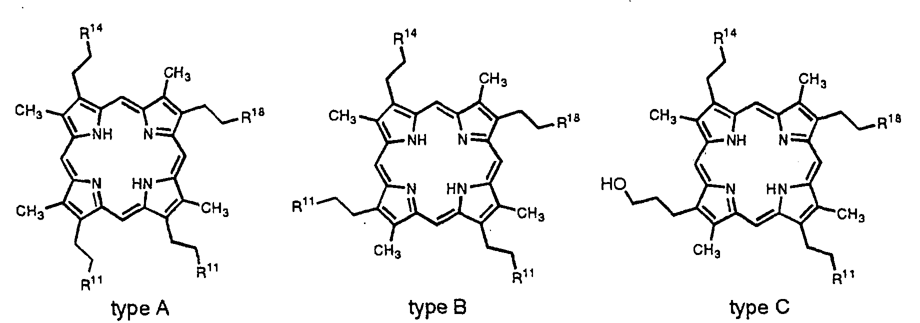

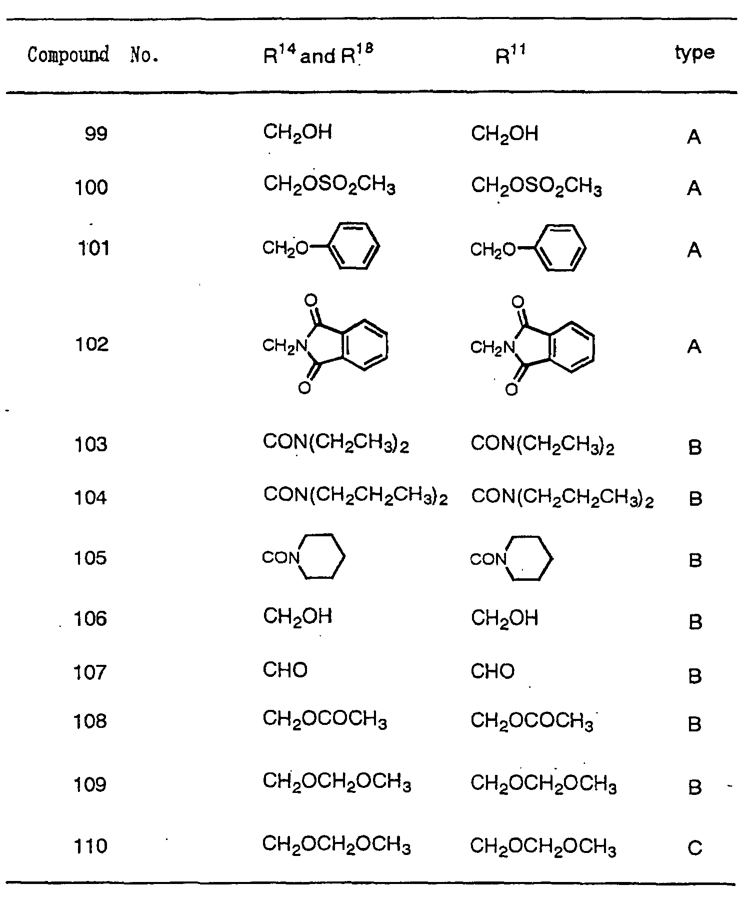

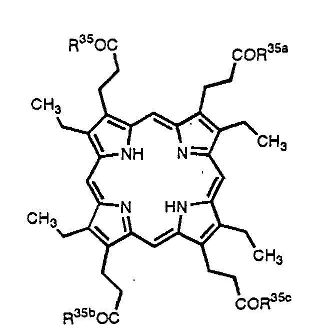

| Compound No. | R³⁵ and R^{25a} and R^{35b} and R^{35c} |
|---|---|
| 111 | OCH₃ |
| 112 | ONa |

The above inhibitor or the above antitumor agent is preferably provided in the form of a pharmaceutical composition comprising Compound (I), an *N*-lower alkyl-substituted compound thereof (the lower alkyl of the *N*-lower alkyl-substituted compound is present on the nitrogen atom of the pyrrole ring in the porphyrin ring) or metal-coordinated compound thereof, or a pharmaceutically acceptable salt thereof, and an additive for a formulation.

Moreover, the present invention relates to use of porphyrin derivatives represented by formula (I), *N*-lower alkyl-substituted compounds thereof (the lower alkyl of the *N*-lower alkyl-substituted compounds is present on the nitrogen atom of the pyrrole ring in the porphyrin ring) or metal-coordinated compounds thereof, or pharmaceutically acceptable salts thereof for the manufacture of the above telomerase inhibitor or the above antitumor agent, and a method for inhibiting telomerase, comprising a step of administering an effective amount of a porphyrin derivative represented by formula (I), an *N*-lower alkyl-substituted compound thereof (the lower alkyl of the *N*-lower alkyl-substituted compound is present on the nitrogen atom of the pyrrole ring in the porphyrin ring) or metal-coordinated compound thereof, or a pharmaceutically acceptable salt thereof to a mammalian animal including human.

Furthermore, the present invention relates to
porphyrin derivatives represented by formula (II), *N*-lower alkyl-substituted compounds thereof (the lower alkyl of the *N*-lower alkyl-substituted compounds is present on the nitrogen atom of the pyrrole ring in the porphyrin ring) or metal-coordinated compounds thereof, or pharmaceutically acceptable salts thereof;
porphyrin derivatives represented by formula (IIA), *N*-lower alkyl-substituted compounds thereof (the lower alkyl of the *N*-lower alkyl-substituted compounds is present on the nitrogen atom of the pyrrole ring in the porphyrin ring) or metal-coordinated compounds thereof, or pharmaceutically acceptable salts thereof;
porphyrin derivatives represented by formula (IIB), *N*-lower alkyl-substituted compounds thereof (the lower alkyl of the *N*-lower alkyl-substituted compounds is present on the nitrogen atom of the pyrrole ring in the porphyrin ring) or metal-coordinated compounds thereof, or pharmaceutically acceptable salts thereof;
porphyrin derivatives represented by formula (IIC), *N*-lower alkyl-substituted compounds thereof (the lower alkyl of the *N*-lower alkyl-substituted compounds is present on the nitrogen atom of the pyrrole ring in the porphyrin ring) or metal-coordinated compounds thereof, or pharmaceutically acceptable salts thereof;
porphyrin derivatives represented by formula (III), *N*-lower alkyl-substituted compounds thereof (the lower alkyl of the *N*-lower alkyl-substituted compounds is present on the nitrogen atom of the pyrrole ring in the porphyrin ring) or metal-coordinated compounds thereof, or pharmaceutically acceptable salts thereof; and
porphyrin derivatives represented by formula (IIIA), *N*-lower alkyl-substituted compounds thereof (the lower alkyl of the *N*-lower alkyl-substituted compounds is present on the nitrogen atom of the pyrrole ring in the porphyrin ring) or metal-coordinated compounds thereof, or pharmaceutically acceptable salts thereof.

In the definition of each group of Compound (I), the lower alkyl moiety of the lower alkyl, the lower alkoxycarbonyl and the lower alkoxysulfonyl may be a linear or branched one having 1 to 13 carbon atoms, and examples thereof include methyl, ethyl, propyl, isopropyl, butyl, isobutyl, *sec*-butyl, *tert*-butyl, pentyl, isoamyl, neopentyl, 1-ethylpropyl, hexyl, heptyl, octyl, nonyl, decyl, undecyl, dodecyl, tridecyl, and the like.

The lower alkenyl may be a linear or branched one having 2 to 13 carbon atoms and having 1 to 4 double bonds, and examples thereof include vinyl, allyl, isopropenyl, butenyl, isobutenyl, pentenyl, isopentenyl, isoprenyl, hexenyl, octenyl, decenyl, undecenyl, dodecenyl, tridecenyl, and the like.

In the definition of each group of Compound (I), the substituents of the substituted lower alkyl, the substituted lower alkenyl and the substituted lower alkoxycarbonyl, are 1 to 3 substituents, and include formyl, CO₂R^{15a} (wherein R^{15a} represents hydrogen, substituted or unsubstituted lower alkyl (the lower alkyl has the same meaning as the lower alkyl in the definition of Compound (II), Compound (IIA), Compound (IIB), Compound (IIC), Compound (III) and Compound (IIIA) described below (hereinafter, Compound (II), Compound (IIA), Compound (IIB), Compound (IIC), Compound (III) and Compound (IIIA) are sometimes referred to as Compounds (II, III)), and the substituent of the substituted lower alkyl has the same meaning as the substituent of the substituted lower alkyl in the definition of Compounds (II, III) described below), substituted or unsubstituted lower alkenyl (the lower alkenyl has the same meaning as the lower alkenyl in the definition of Compounds (II, III) described below, and the substituent of the substituted lower alkenyl has the same meaning as the substituent of the substituted lower alkenyl in the definition of Compounds (II, III) described below) or substituted or unsubstituted aralkyl (the aralkyl has the same meaning as the aralkyl in the definition of Compounds (II, III) described below, and the substituent of the substituted aralkyl has the same meaning as the substituent of the substituted aralkyl in the definition of Compounds (II, III) described below)), CONR^{16a}R^{17a} (wherein R^{16a} and R^{17a} are the same or different and each represents hydrogen, substituted or unsubstituted lower alkyl (the lower alkyl has the same meaning as the lower alkyl in the definition of Compounds (II, III) described below, and the substituent of the substituted lower alkyl has the same meaning as the substituent of the substituted lower alkyl in the definition of Compounds (II, III) described below), substituted or unsubstituted aralkyl (the aralkyl has the same meaning as the aralkyl in the definition of Compounds (II, III) described below, and the substituent of the substituted aralkyl has the same meaning as the substituent of the substituted aralkyl in the definition of Compounds (II, III) described below), substituted or unsubstituted lower alkoxy (the lower alkoxy has the same meaning as the lower alkoxy in the definition of Compounds (II, III) described below, and the substituent of the substituted lower alkoxy has the same meaning as the substituent of the substituted lower alkoxy in the definition of Compounds (II, III) described below), substituted or unsubstituted aryl (the aryl has the same meaning as the aryl in the definition of Compounds (II, III) described below, and the substituent of the substituted aryl has the same meaning as the substituent of the substituted aryl in the definition of Compounds (II, III) described below) or substituted or unsubstituted heteroaryl (the heteroaryl has the same meaning as the heteroaryl in the definition of Compounds (II, III) described below, and the substituent of the substituted heteroaryl has the same meaning as the substituent of the substituted heteroaryl in the definition of Compounds (II, III) described below), or R^{16a} and R^{17a} are combined with the adjacent N to form a substituted or unsubstituted heterocyclic group (the heterocyclic group formed together with the adjacent N has the same meaning as the heterocyclic group formed together with the adjacent N in the definition of Compounds (II, III) described below, and the substituent of the substituted heterocyclic group formed together with the adjacent N has the same meaning as the substituent of the substituted aryl in the definition of Compounds (II, III) described below)), CH₂OR^{19a} (wherein R^{19a} represents hydrogen, substituted or unsubstituted lower alkyl (the lower alkyl has the same meaning as the lower alkyl in the definition of Compounds (II, III) described below, and the substituent of the substituted lower alkyl has the same meaning as the substituent of the substituted lower alkyl in the definition of Compounds (II, III) described below), substituted or unsubstituted aralkyl (the aralkyl has the same meaning as the aralkyl in the definition of Compounds (II, III) described below, and the substituent of the substituted aralkyl has the same meaning as the substituent of the substituted aralkyl in the definition of Compounds (II, III) described below), substituted or unsubstituted lower alkanoyl (the lower alkanoyl has the same meaning as the lower alkanoyl in the definition of Compounds (II, III) described below, and the substituent of the substituted lower alkanoyl has the same meaning as the substituent of the substituted lower alkanoyl in the definition of Compounds (II, III) described below), substituted or unsubstituted aroyl (the aroyl has the same meaning as the aroyl in the definition of Compounds (II, III) described below, and the substituent of the substituted aroyl has the same meaning as the substituent of the substituted aroyl in the definition of Compounds (II, III) described below), SO₂R^{20a} (wherein R^{20a} represents substituted or unsubstituted lower alkyl (the lower alkyl has the same meaning as the lower alkyl in the definition of Compounds (II, III) described below, and the substituent of the substituted lower alkyl has the same meaning as the substituent of the substituted lower alkyl in the definition of Compounds (II, III) described below) or substituted or unsubstituted aryl (the aryl has the same meaning as the aryl in the definition of Compounds (II, III) described below, and the substituent of the substituted aryl has the same meaning as the substituent of the substituted aryl in the definition of Compounds (II, III) described below)) or Si(R^{21a})₃ (wherein R^{21a}'s are the same or different and each represents substituted or unsubstituted lower alkyl (the lower alkyl has the same meaning as the lower alkyl in the definition of Compounds (II, III) described below, and the substituent of the substituted lower alkyl has the same meaning as the substituent of the substituted lower alkyl in the definition of Compounds (II, III) described below) or substituted or unsubstituted aryl (the aryl has the same meaning as the aryl in the definition of Compounds (II, III) described below, and the substituent of the substituted aryl has the same meaning as the substituent of the substituted aryl in the definition of Compounds (II, III) described below)), OR^{19a1} (wherein R^{19a1} has the same meaning as R^{19a} described above) or substituted or unsubstituted aryl (the aryl has the same meaning as the aryl in the definition of Compounds (II, III) described below, and the substituent of the substituted aryl has the same meaning as the substituent of the substituted aryl in the definition of Compounds (II, III) described below), and the like.

In the definition of each group of Compounds (II, III), the lower alkyl and the lower alkyl moiety of the lower alkoxy, the lower alkanoyl, and the *N*-lower alkyl-substituted compounds may be a linear or branched one having 1 to 10 carbon atoms, and examples thereof include methyl, ethyl, propyl, isopropyl, butyl, isobutyl, *sec*-butyl, *tert*-butyl, pentyl, isoamyl, neopentyl, 1-ethylpropyl, hexyl, heptyl, octyl, nonyl, decyl, and the like.

The alkylene moiety of the aralkyl represents a group formed by removing one hydrogen from the above lower alkyl.

The lower alkenyl may be a linear or branched one having 2 to 10 carbon atoms and having 1 to 3 double bonds, and examples thereof include vinyl, allyl, isopropenyl, butenyl, isobutenyl, pentenyl, isopentenyl, isoprenyl, hexenyl, octenyl, decenyl, and the like.

The aryl and the aryl moiety of the aroyl may be aryl having 6 to 15 carbon atoms, and examples thereof include phenyl, naphthyl, anthranyl, and the like.

The aralkyl may be aralkyl having 7 to 15 carbon atoms, and examples thereof include benzyl, phenethyl, benzhydryl, naphthylmethyl, anthrylmethyl, and the like.

The heteroaryl is a monocyclic or polycyclic aromatic heterocyclic group, containing at least one heteroatom such as an oxygen atom, a sulfur atom or a nitrogen atom, wherein each ring is a 3 to 8-membered ring, and examples thereof include 5- or 6-membered nitrogen-containing aromatic heterocyclic groups such as imidazolyl, pyridyl, indolyl, quinolyl, isoquinolyl, quinoxalinyl, quinazolinyl, pyridazinyl, pyrimidinyl, pyrazinyl, and the like.

The heterocyclic group formed together with the adjacent N is a monocyclic or polycyclic heterocyclic group wherein each ring is a 3- to 8-membered ring, and may contain an oxygen atom, a sulfur atom, or the like in the ring other than the nitrogen atom. Preferred examples include 5- or 6-membered nitrogen-containing alicyclic heterocyclic group such as pyrrolidinyl, oxopyrrolidinyl, piperidinyl, piperazinyl, morpholino, thiomorpholino, homopiperidinyl, homopiperazinyl, tetrahydropyridinyl, phthalimido, and the like.

The metal in the metal-coordinated compound includes Fe, Mn, Sn, Mg, Cu, Co, Pd, Pt, Ni, Zn, and the like.

In the definition of each group of Compounds (II, III), the substituents of the substituted lower alkyl, the substituted lower alkenyl, the substituted lower alkoxy, the substituted lower alkanoyl, the substituted aryl, the substituted heteroaryl, the substituted aroyl and the substituted aralkyl, are 1 to 3 substituents, and include CO₂R³⁹ (wherein R³⁹ represents hydrogen, substituted or unsubstituted lower alkyl or aryl, or a group formed by removing one hydroxyl group from an polyglycol), COR⁴⁰ (wherein R⁴⁰ has the same meaning as R³⁹ described above), CONR⁴¹R⁴² (wherein R⁴¹ and R⁴² are the same or different and have the same meanings as R³⁹ described above, respectively), NR⁴³R⁴⁴ (wherein R⁴³ and R⁴⁴ are the same or different and have the same meanings as R³⁹ described above, respectively), OR⁴⁵ (wherein R⁴⁵ has the same meaning as R³⁹ described above), OCOR⁴⁶ (wherein R⁴⁶ has the same meaning as R³⁹ described above), SO₃R⁴⁷ (wherein R⁴⁷ has the same meaning as R³⁹ described above), SO₂R⁴⁸ (wherein R⁴⁸ has the same meaning as R³⁹ described above), SOR⁴⁹ (wherein R⁴⁹ has the same meaning as R³⁹ described above), SR⁵⁰ (wherein R⁵⁰ has the same meaning as R³⁹ described above), N⁺R⁵¹R⁵²R⁵³X¹⁻ (wherein X¹⁻ represents an anion, and R⁵¹, R⁵² and R⁵³ are the same or different and have the same meanings as R³⁹ described above, respectively), cyano, nitro, halogen, and the like. However, the substituent of the substituted lower alkyl in R¹⁶ does not include CO₂R³⁹, hydroxy and halogen.

In the above definitions of the substituents, the polyglycol includes polyethylene glycol, polypropylene glycol, and the like; the halogen means each atom of fluorine, chlorine, bromine, and iodine; and the anion includes a chlorine ion, a bromine ion, and the like. The lower alkyl and the aryl have the same meanings as the lower alkyl and the aryl described above, respectively. The substituents of the substituted lower alkyl in R³⁹ to R⁵³, are 1 to 3 substituents, and include CO₂R⁵⁵ (wherein R⁵⁵ represents hydrogen or lower alkyl), COR⁵⁶ (wherein R⁵⁶ has the same meaning as R⁵⁵ described above), CONR⁵⁷R⁵⁸ (wherein R⁵⁷ and R⁵⁸ are the same or different and have the same meanings as R⁵⁵ described above, respectively), NR⁵⁹R⁶⁰ (wherein R⁵⁹ and R⁶⁰ are the same or different and have the same meanings as R⁵⁵ described above, respectively), OR⁶¹ (wherein R⁶¹ has the same meaning as R⁵⁵ described above), OCOR⁶² (wherein R⁶² has the same meaning as R⁵⁵ described above), SO₃R⁶³ (wherein R⁶³ has the same meaning as R⁵⁵ described above), SO₂R⁶⁴ (wherein R⁶⁴ has the same meaning as R⁵⁵ described above), SOR⁶⁵ (wherein R⁶⁵ has the same meaning as R⁵⁵ described above), SR⁶⁶ (wherein R⁶⁶ has the same meaning as R⁵⁵ described above), N⁺R⁶⁷R⁶⁸R⁶⁹X²⁻ (wherein X²⁻ represents an anion, and R⁶⁷, R⁶⁸ and R⁶⁹ are the same or different and have the same meanings as R⁵⁵ described above, respectively), cyano, nitro, halogen, and the like. The lower alkyl, the anion and the halogen have the same meanings as described above, respectively.

The pharmaceutically acceptable salts of Compound (I), Compound (II), Compound (IIA), Compound (IIB), Compound (IIC), Compound (III), Compound (IIIA) and *N*-lower alkyl-substituted compounds thereof or metal-coordinated compounds thereof include pharmaceutically acceptable acid addition salts, metal salts, ammonium salts, organic amine addition salts, amino acid addition salts, and the like. The pharmaceutically acceptable acid addition salts include inorganic acid salts such as hydrochlorides, sulfates, phosphates, *etc.,* and organic acid salts such as acetates, maleates, fumarates, tartrates, citrates, lactates, *etc.* The pharmaceutically acceptable metal salts include alkali metal salts such as lithium salts, sodium salts, potassium salts, *etc.;* alkaline earth metal salts such as magnesium salts, calcium salts, *etc.;* aluminum salts; zinc salts; and the like. The pharmaceutically acceptable ammonium salts include salts such as ammonium, tetramethylammonium, and the like. The pharmaceutically acceptable organic amine addition salts include addition salts with morpholine, piperidine, and the like. The pharmaceutically acceptable amino acid addition salts include addition salts with glycine, phenylalanine, glutamic acid, lysine, and the like.

Compound (I), Compound (II), Compound (IIA), Compound (IIB), Compound (IIC), Compound (III), Compound (IIIA) and *N*-lower alkyl-substituted compounds thereof or metal-coordinated compounds thereof are commercially available, or can be produced by known methods *(Laboratory methods in porphyrin and metalloporphyrin research,* ELSEVIER, Kevin M. Smith *et al*., Netherlands (1975) and Japanese Published Unexamined Patent Application No. 238585/89) or according to the methods similar thereto, or by the following methods or according to the methods similar thereto.

### Production method 1

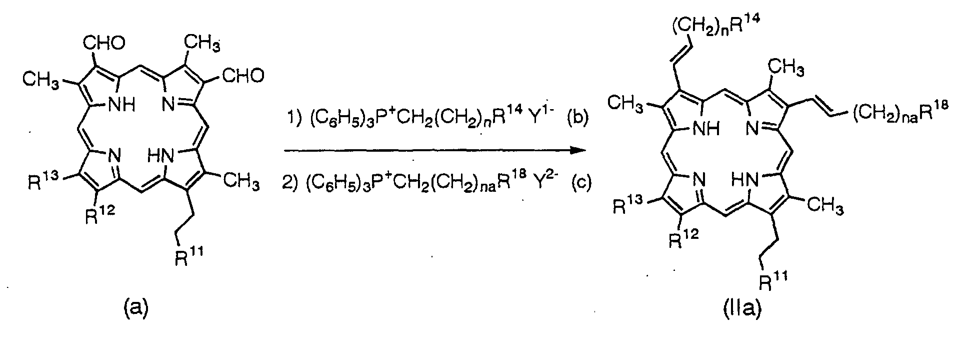

(In the formulae, Y¹ and Y² are the same or different and each represents halogen; and n, na, R¹¹, R¹², R¹³, R¹⁴ and R¹⁸ have the same meanings as described above, respectively.)

The halogen in the definition of Y¹ and Y² has the same meaning as described above.

Compound (IIa) wherein P¹ and P² are combined to represent a bond and Q¹ and Q² are combined to represent a bond among Compounds (II) can be obtained by reacting Compound (a) produced according to the method similar to that described in a literature (*J*. *Org. Chem., 62:* 1875 (1997)) or the like with Compound (b) in an inert solvent in the presence of a base, and then reacting with Compound (c).

The inert solvent includes chloroform, dichloromethane, ether, tetrahydrofuran, acetone, dimethylformamide, acetonitrile, and the like, and they are used alone or in combination. As the base, sodium hydride, n-butyl lithium, sodium methoxide, sodium hexamethyldisilazane, or the like may be used.

Compound (b), Compound (c) and the base each is used in an amount of usually 1 equivalent or more, preferably 1 to 200 equivalents to Compound (a). The reaction is usually carried out at 0 to 100°C and finished within 5 minutes to 24 hours.

Among Compound (IIa), the compound wherein n and na are the same and R¹⁴ and R¹⁸ are the same can be obtained using Compound (b) in an amount of 2 equivalents or more, preferably 2 to 200 equivalents to Compound (a). The reaction is usually carried out at 0 to 100°C and finished within 5 minutes to 24 hours.

### Production method 2

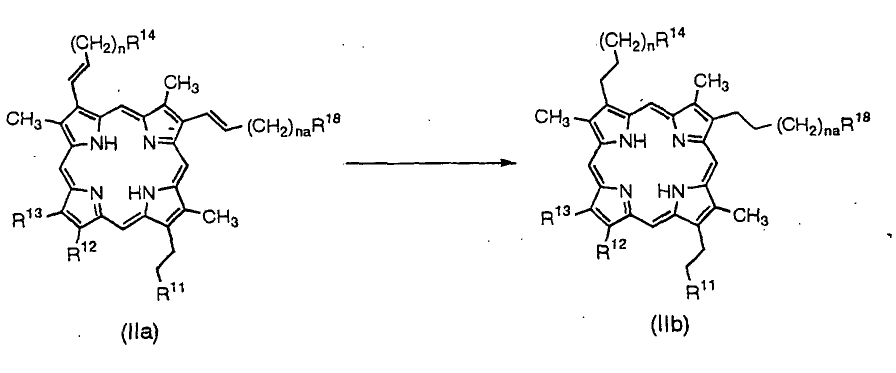

(In the formulae, n, na, R¹¹, R¹², R¹³, R¹⁴ and R¹⁸ have the same meanings as described above, respectively.)

Compound (IIb) wherein P¹, P², Q¹ and Q² each is a hydrogen atom among Compounds (II) can be obtained by subjecting Compound (IIa) to hydrogenation in an inert solvent in the presence of a catalyst.

As the inert solvent, methanol, ethanol, water or the like may be used. As the catalyst, for example, palladium-carbon, platinum oxide or the like may be used in an amount of usually 0.1 equivalent or more, preferably 0.1 to 200 equivalents to Compound (IIa). The reaction is usually carried out at 0 to 100°C and finished within 10 minutes to 24 hours.

### Production method 3

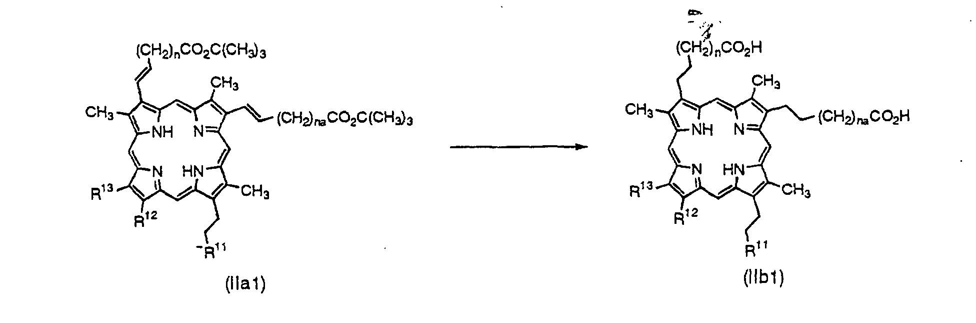

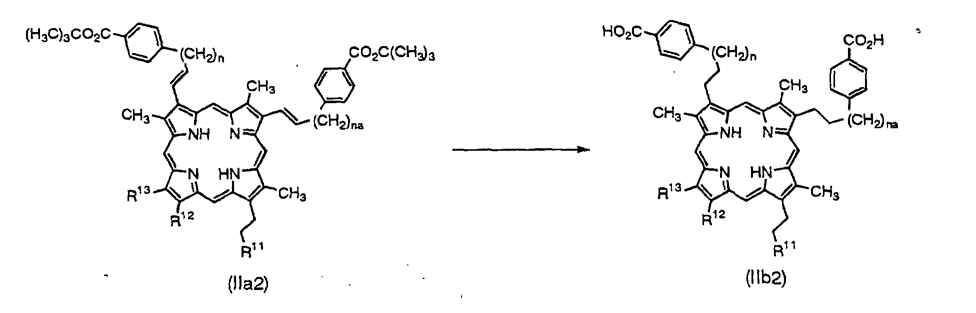

(In the formulae, n, na, R¹¹, R¹² and R¹³ have the same meanings as described above, respectively.)

Compound (IIb1) wherein R¹⁴ and R¹⁸ each is CO₂H among Compounds (IIb) can be obtained by hydrogenating Compound (IIa1) wherein R¹⁴ and R¹⁸ each is CO₂C(CH₃)₃ among Compounds (IIa) in an inert solvent in the presence of an acid and a catalyst.

As the inert solvent, any of methanol, ethanol, water and the like can be used. Alternatively, the reaction may be carried out without a solvent. As the acid, any of organic acids, such as formic acid, acetic acid, trifluoroacetic acid and the like, preferably formic acid, can be used in an amount of 1 equivalent or more, preferably 1 to 200 equivalents to Compound (IIa1). As the catalyst, for example, palladium-carbon, platinum oxide or the like may be used in an amount of usually 0.1 equivalent or more, preferably 0.1 to 200 equivalents to Compound (IIa1). The reaction is usually carried out at 0 to 150°C and finished within 1 to 24 hours.

Compound (IIb2) wherein R¹⁴ and R¹⁸ each is 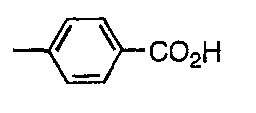 among Compounds (IIb) can also be produced from Compound (IIa2) wherein R¹⁴ and R¹⁸ each is 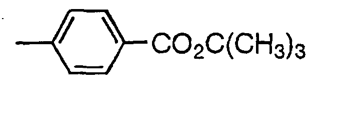 among Compounds (IIa), in a manner similar to the above.

### Production method 4

Compound (IId) wherein any of R¹¹, R¹⁴, R¹⁸, R²² and R²³ is CO₂H among Compounds (II) can be obtained by treating Compound (IIc) wherein any of R¹¹, R¹⁴, R¹⁸, R²² and R²³ is CO₂CH₃ or CO₂CH₂CH₃ with a base in an inert solvent.

Compound (IIc) can be produced according to the method similar to that described in a literature *(Laboratory methods in porphyrin and metalloporphyrin research,* ELSEVIER, Kevin M. Smith *et al.,* Netherlands (1975)).

As the inert solvent, any of the inert solvents described in Production method 3, may be used and they are used alone or in combination. As the base, an alkali metal hydroxide or an alkaline earth metal hydroxide such as sodium hydroxide, potassium hydroxide, etc., or the like may be used in an amount of usually 4 equivalents or more, preferably 4 to 400 equivalents to Compound (IIc). The reaction is usually carried out at 0 to 50°C and finished within 10 minutes to 24 hours.

### Production method 5

Compound (IIe) wherein any of R¹¹, R¹⁴, R¹⁸, R²² and R²³ is CONR¹⁶R¹⁷ (wherein R¹⁶ and R¹⁷ have the same meanings as described above, respectively) among Compounds (II) can be obtained by reacting Compound (IId) wherein any of R¹¹, R¹⁴, R¹⁸, R²² and R²³ is CO₂H with a chlorinating agent, and then reacting the obtained product with Compound (d) represented by HNR¹⁶R¹⁷ (wherein R¹⁶ and R¹⁷ have the same meanings as described above, respectively) in an inert solvent, or Compound (IIe) can be alternatively obtained by reacting Compound (IId) with Compound (d) in the presence of a condensing agent in an inert solvent.

As the inert solvent, any of the inert solvents described in Production method 1, may be used and they are used alone or in combination. As the chlorinating agent, thionyl chloride, phosphorus trichloride, oxalyl chloride or the like may be used. The chlorinating agent and Compound (d) each is used in an amount of usually 4 equivalents or more, preferably 4 to 400 equivalents to Compound (IId), respectively. The reaction with the chlorinating agent is usually carried out at 0 to 150°C and finished within 5 minutes to 24 hours, and the reaction with Compound (d) is usually carried out at 0 to 100°C and finished within 5 minutes to 24 hours. As the condensing agent, any of the agents usually used in the condensation of a carboxylic acid with an amine, for example, dicyclohexylcarbodiimide, 1-ethyl-3-(3-dimethylaminopropyl)carbodiimide·hydrochloride or the like may be used, and 1-hydroxybenzotriazole, N-hydroxysuccinimide or the like may be further added in an amount of 4 to 400 equivalent. Compound (d) and the condensing agent each is used in an amount of usually 4 equivalents or more, preferably 4 to 400 equivalents to Compound (IId). The reaction is usually carried out at 0 to 100°C and finished within 5 minutes to 24 hours.

### Production method 6

Compound (IIf) wherein any of R¹¹, R¹⁴, R¹⁸, R²² and R²³ is CH₂OH among Compounds (II) can be obtained by reacting Compound (IIc) with a reducing agent in a solvent.

As the solvent used in the reaction, any of the inert solvents described in Production method 1 may be used, and they are used alone or in combination. As the reducing agent, lithium aluminum hydride, sodium borohydride, or the like may be used in an amount of usually 4 equivalents or more, preferably 4 to 400 equivalents to Compound (IIc). The reaction is usually carried out at -100 to 100°C and finished within 5 minutes to 24 hours.

### Production method 7

Compound (IIg) wherein any of R¹¹, R¹⁴, R¹⁸, R²² and R²³ is CH₂OR^{19b} (wherein R^{19b} has the same meaning as R²⁶ described above) among Compounds (II) can be obtained by reacting Compound (IIf) with Compound (e) represented by R²⁶Y³ (wherein Y³ represents halogen and R²⁶ has the same meaning as described above) in an inert solvent in the presence of a base or by reacting Compound (IIf) with Compound (f) represented by (R³⁸)₂O (wherein R³⁸ represents substituted or unsubstituted lower alkanoyl or substituted or unsubstituted aroyl, and the substituted or unsubstituted lower alkanoyl and the substituted or unsubstituted aroyl have the same meaning as described above, respectively) in an inert solvent.

As the inert solvent, any of the inert solvents described in Production method 1 may be used, and they are used alone or in combination. Examples of the base include amines, such as pyridine, imidazole, triethylamine, diisopropyethylamine, etc., carbonates or bicarbonates of alkali metals or alkaline earth metals, such as sodium carbonate, potassium carbonate, calcium carbonate, sodium hydrogen carbonate, *etc.,* phosphates and the like. Examples of the acid include inorganic acids, such as hydrochloric acid, sulfuric acid, *etc.*, organic acids, such as p-toluenesulfonic acid, camphorsulfonic acid, pyridinium p-toluenesulfonate, trifluoroacetic acid, trifluoromethanesulfonic acid, *etc.,* and Lewis acids, such as titanium tetrachloride, a boron trifluoride · diethyl ether complex, *etc.* Compound (e), Compound (f), the base and the acid each is used usually in an amount of 4 equivalents or more, preferably 4 to 400 equivalents to Compound (IIf). The reaction is usually carried out at 0 to 100°C and finished within 5 minutes to 24 hours.

By combining optionally the methods described in the above or methods similar to them, Compound (I), Compound (II), Compound (IIA), Compound (IIB), Compound (IIC), Compound (III) and Compound (IIIA) having desired functional group(s) at desired position(s) can be obtained. Furthermore, by replacing the substituent(s), other derivatives of Compound (I), Compound (11), Compound (IIA), Compound (IIB), Compound (IIC), Compound (III) and Compound (IIIA) can also be produced.

The target compound in each production method can be isolated and purified by purification methods conventionally used in synthetic organic chemistry such as filtration, extraction, washing, drying, concentration, recrystallization, various kinds of chromatography, or the like.

In the case where a salt of *N*-lower alkyl-substituted compound (the lower alkyl of the *N*-lower alkyl-substituted compound is present on the nitrogen atom of the pyrrole ring in the porphyrin ring) or metal-coordinated compound of Compound (I), Compound (II), Compound (IIA), Compound (IIB), Compound (IIC), Compound (III) and Compound (IIIA) is desired, and the *N*-lower alkyl-substituted compound (the lower alkyl of the N-lower alkyl-substituted compound is present on the nitrogen atom of the pyrrole ring in the porphyrin ring) or metal-coordinated compound of Compound (I), Compound (II), Compound (IIA), Compound (IIB), Compound (IIC), Compound (III) and Compound (IIIA) is obtained in the form of the desired salt, it can be subjected to purification as it is. In the case where the product is obtained in a free form, it may be dissolved or suspended in an appropriate solvent by a usual method and a desired acid or base may be added to form a salt, which can be successively isolated and purified.

Moreover, Compound (I), Compound (II), Compound (IIA), Compound (IIB), Compound (IIC), Compound (III), Compound (IIIA), and *N*-lower alkyl-substituted compounds thereof (the lower alkyl of the *N*-lower alkyl-substituted compound is present on the nitrogen atom of the pyrrole ring in the porphyrin ring) or metal-coordinated compounds thereof, and the pharmaceutically acceptable salts thereof may be in the form of adducts with water or various solvents, which are also within the scope of the present invention.

Specific examples of Compound (I) and the *N*-lower alkyl-substituted compound thereof (the lower alkyl of the *N*-lower alkyl-substituted compound is present on the nitrogen atom of the pyrrole ring in the porphyrin ring) or metal-coordinated compound thereof are shown in Table 1.

Specific examples of Compound (II), Compound (IIA), Compound (IIB), Compound (IIC), Compound (III), Compound (IIIA), and the *N*-lower alkyl-substituted compounds thereof (the lower alkyl of the *N*-lower alkyl-substituted compound is present on the nitrogen atom of the pyrrole ring in the porphyrin ring) or metal-coordinated compounds thereof are shown in Table 2.

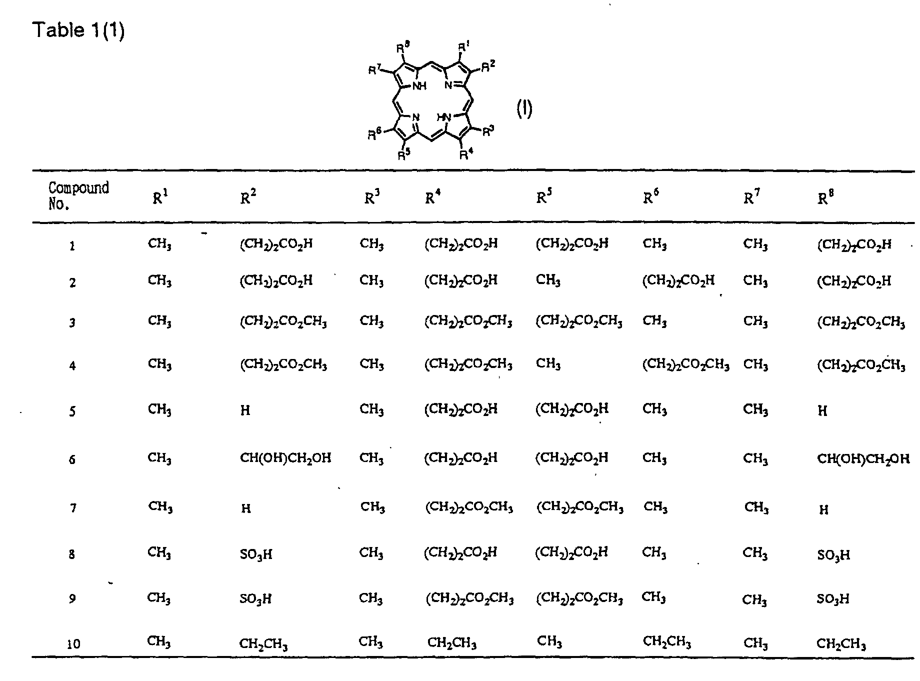

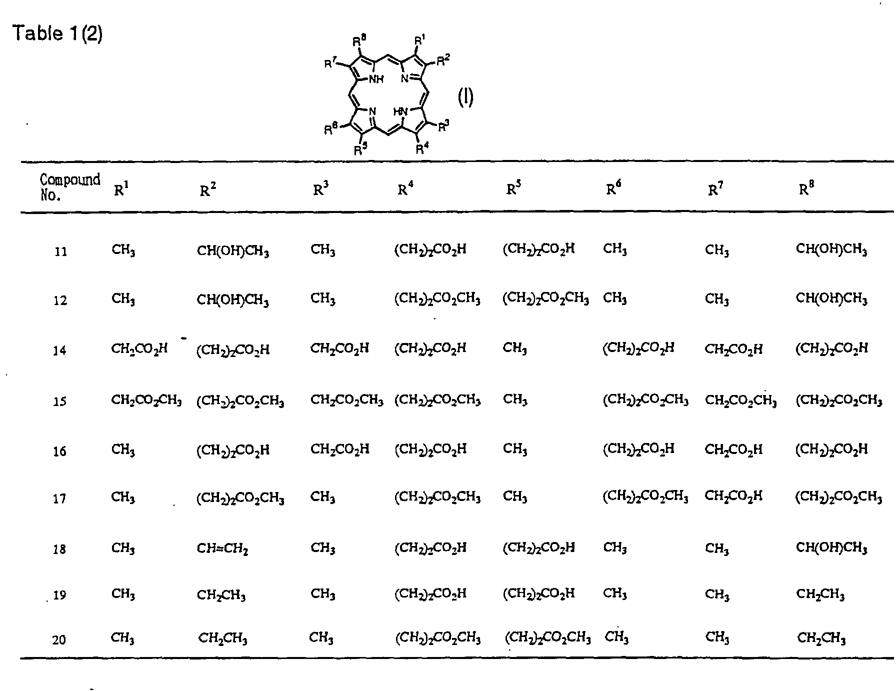

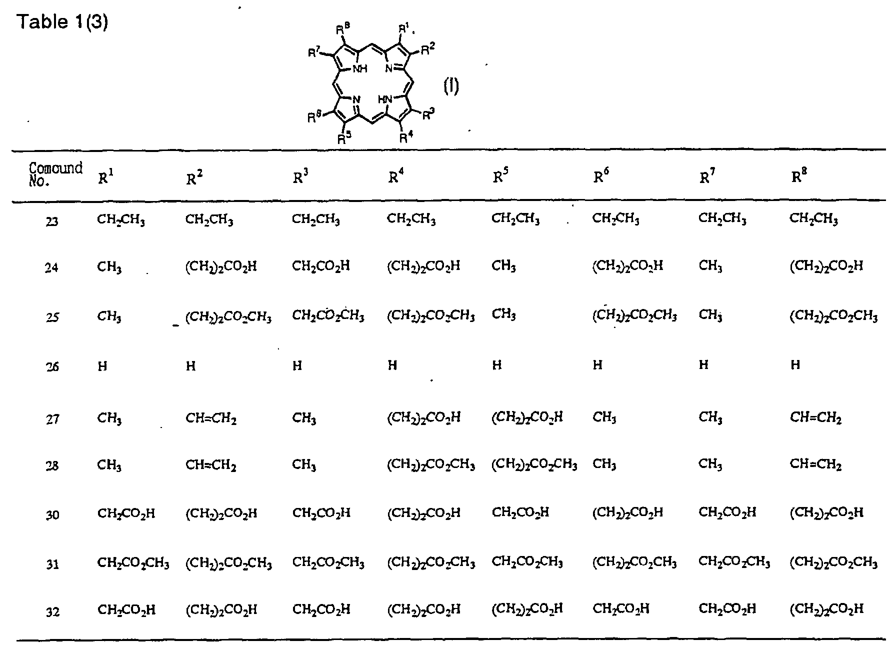

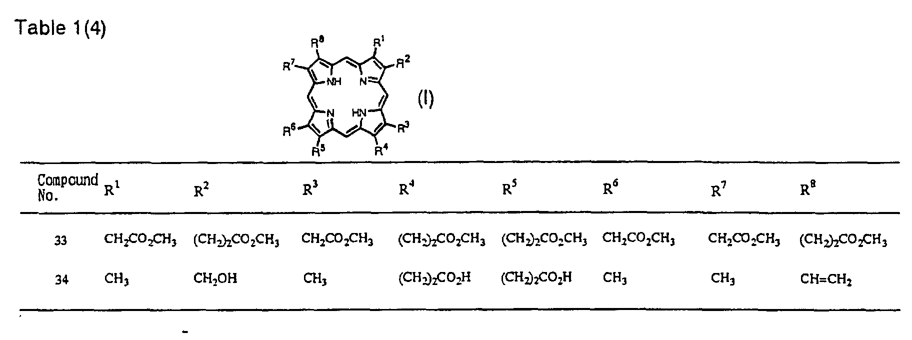

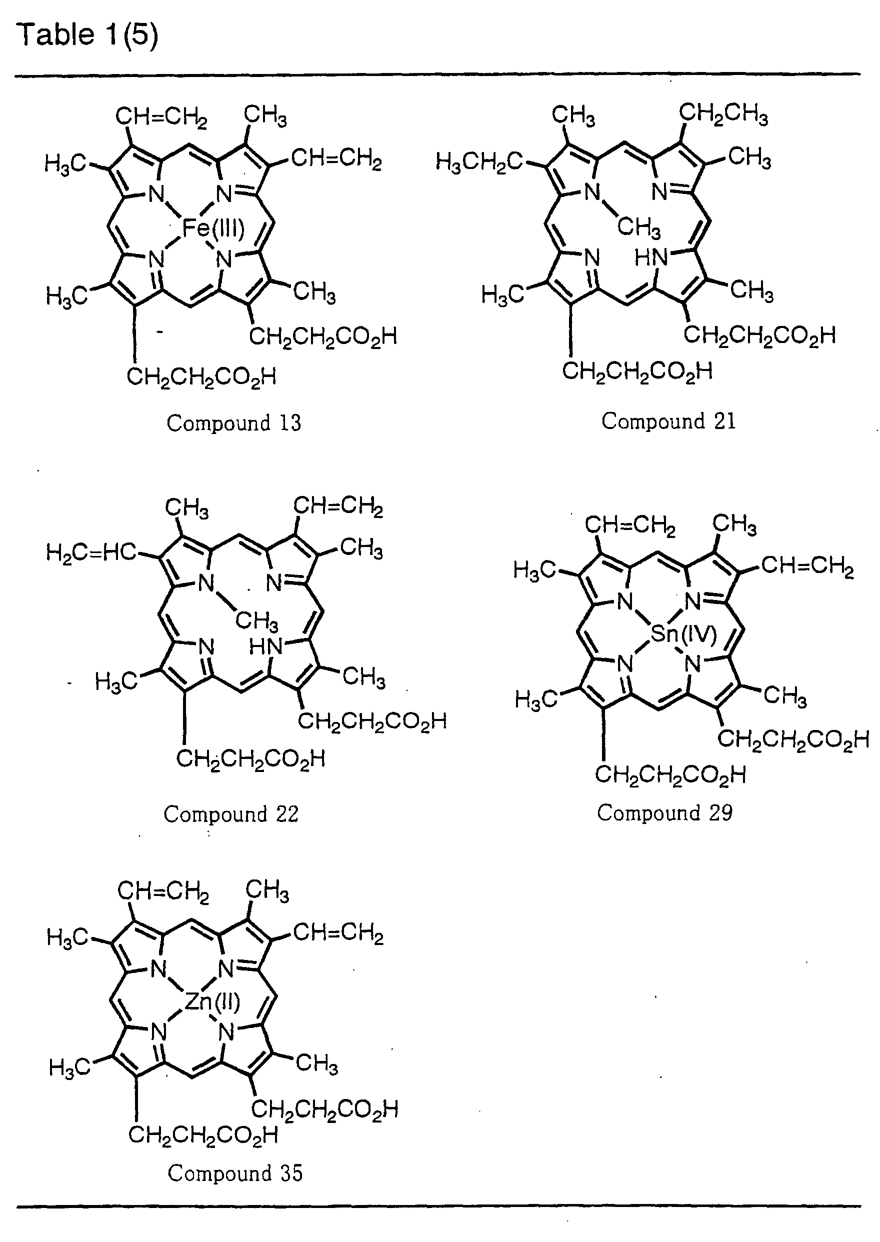

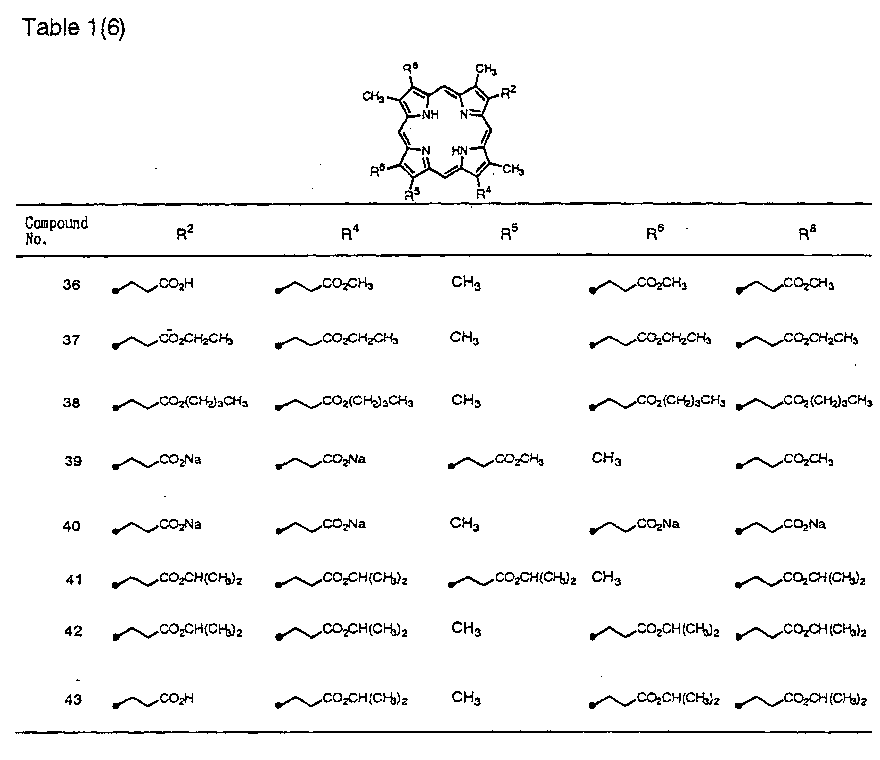

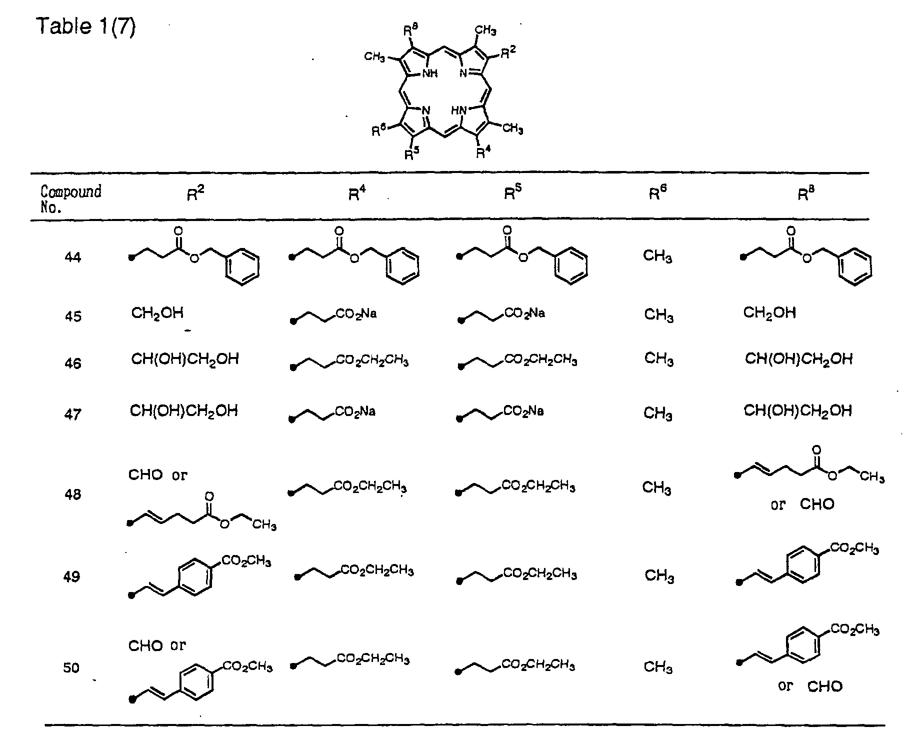

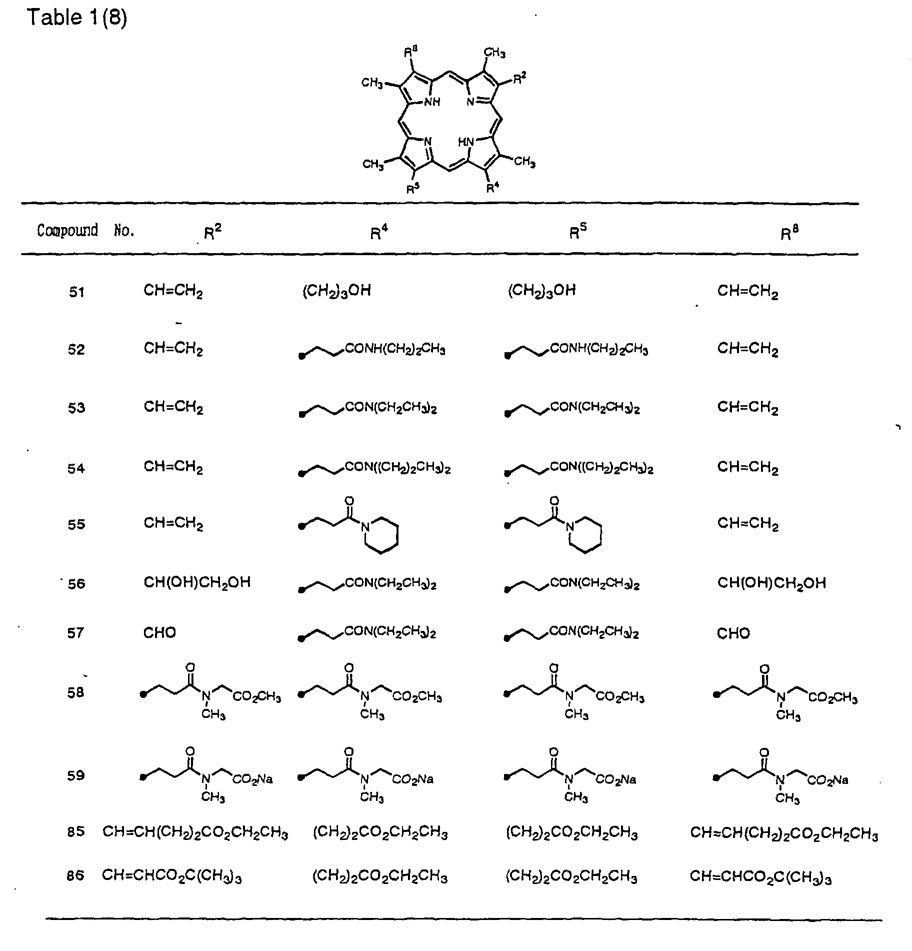

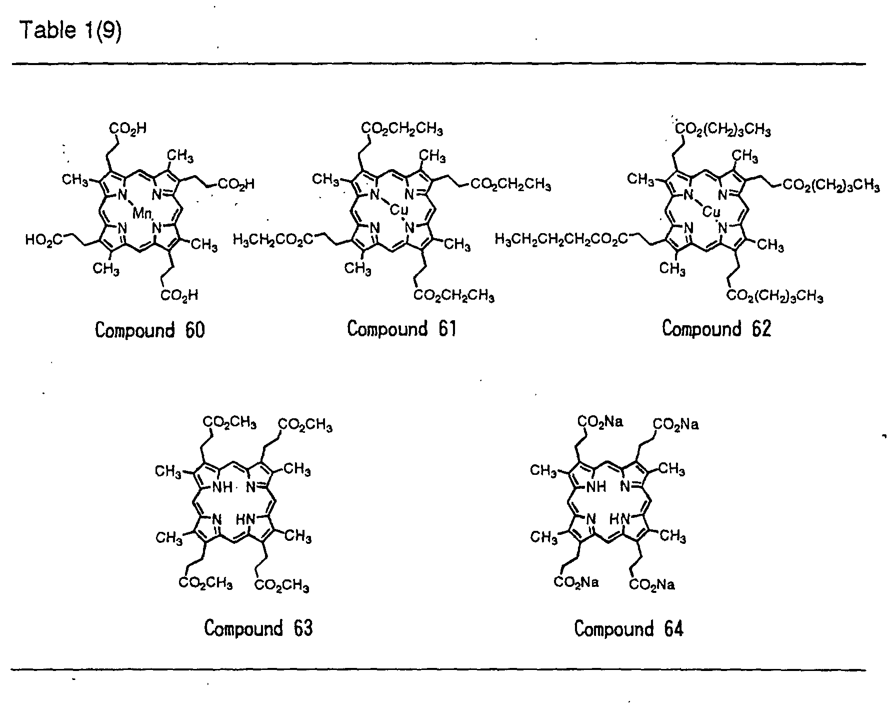

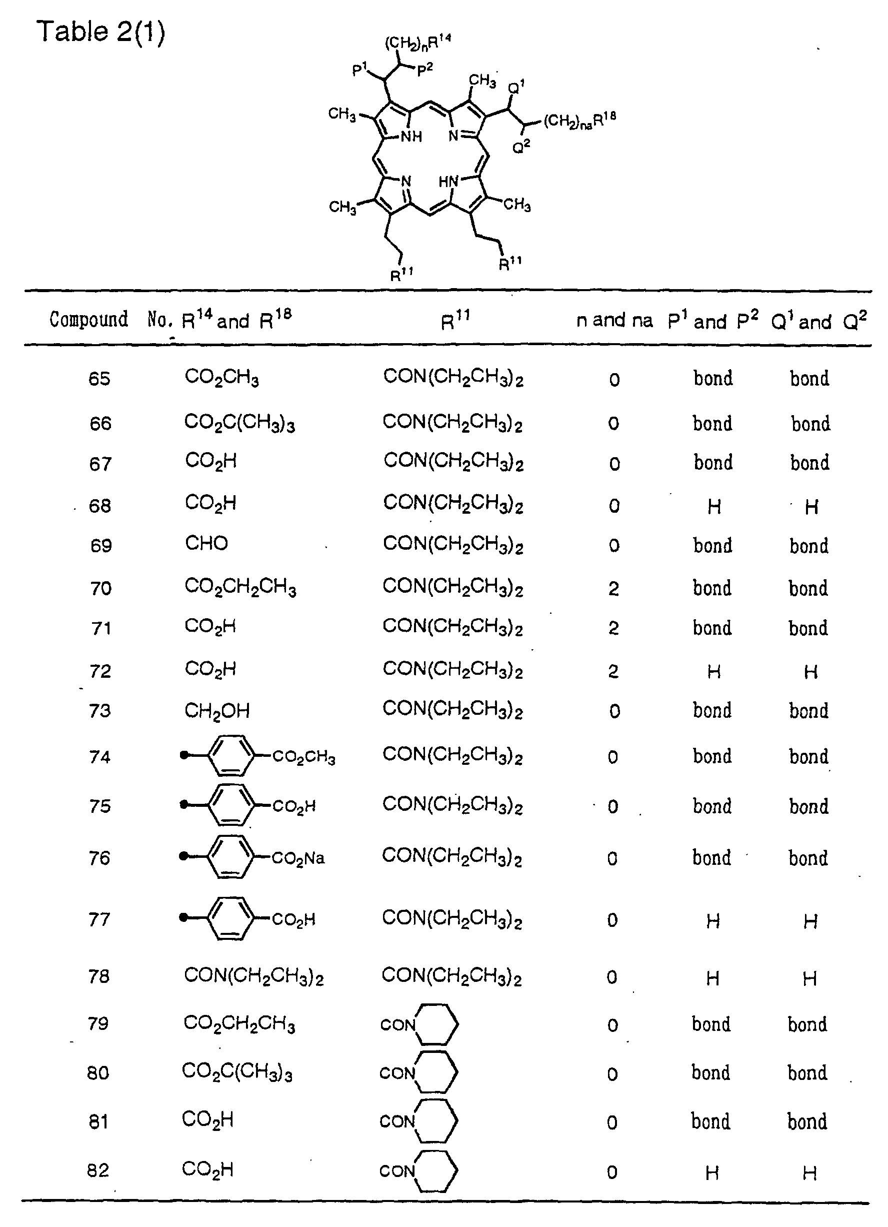

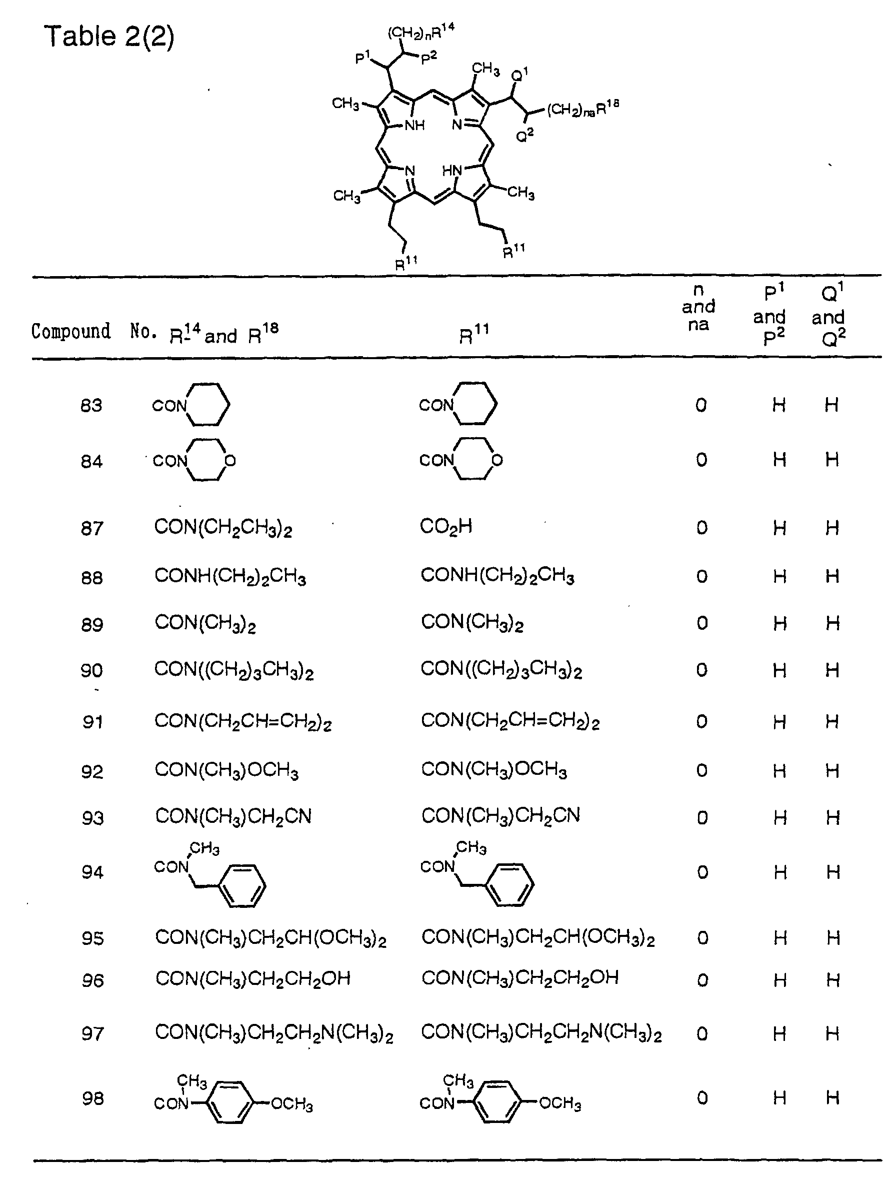

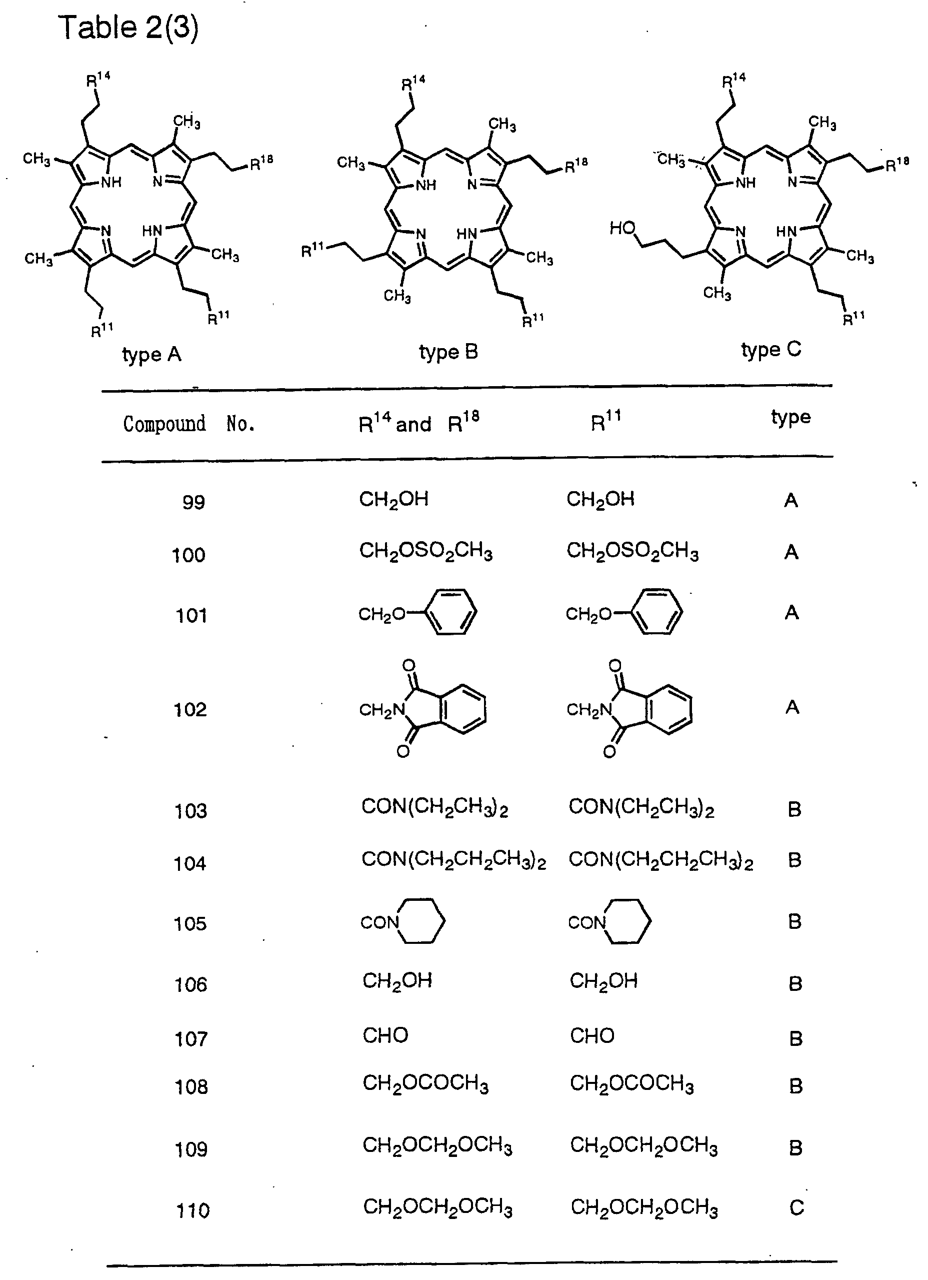

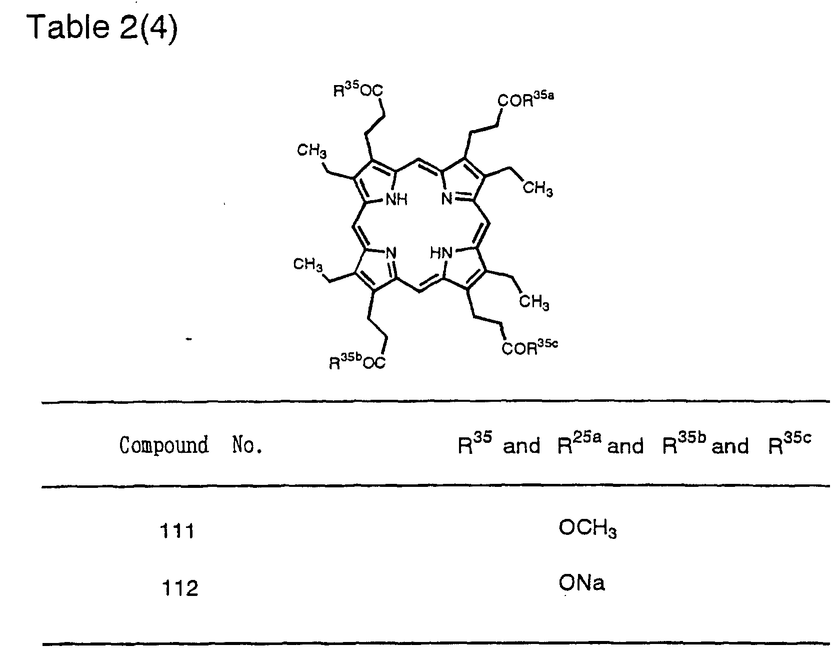

Next, pharmacological activities of the representative Compound (I), Compound (II), Compound (IIA), Compound (IIB), Compound (IIC), Compound (III), Compound (IIIA), or *N*-lower alkyl-substituted compounds (the lower alkyl of the *N*-lower alkyl-substituted compounds is present on the nitrogen atom of the pyrrole ring in the porphyrin ring) or metal-coordinated compounds thereof are shown specifically by Test Examples.

### Test Example 1

### Telomerase inhibitory activity in vitro:

The telomerase-inhibitory activity of Compound (I), and *N*-lower alkyl-substituted compound thereof (the lower alkyl of the *N*-lower alkyl-substituted compound is present on the nitrogen atom of the pyrrole ring in the porphyrin ring) or metal-coordinated compound thereof was measured in accordance with a known method (U.S. Patent 5,760,062).

That is, a dimethyl sulfoxide (DMSO) solution of a test compound was mixed with a nucleus extraction solution, wherein nucleus is derived from HEK293 cell, in the presence of a substrate, oligodeoxynucleotide, and deoxynucleotide triphospate, and the mixture was incubated.

The resulting reaction product (DNA having a telomere sequence) was adsorbed on a membrane, and hybridization was carried out by using a labeled oligonucleotide probe having a sequence complementary to the telomere sequence. Inhibitory ratio was calculated based on the ratio of the signal strength of the label on the membrane in the presence of the test compound relative to the signal strength in the absence of the test compound (control). The compound concentration inhibiting 50% of the enzyme activity relative to that of the control was determined as IC₅₀. The IC₅₀ value of TMPyP4 in the present assay system was found to be 60 µmol/L. Table 3 shows the results of the measurement of the inhibitory activity of various porphyrin derivatives.

**Table 3**

| Compound No. | IC₅₀ (µ mol/L) |
|---|---|
| 1 | 2.8 |
| 2 | 3.2 |
| 5 | 13 |
| 6 | 18 |
| 8 | 11 |
| 9 | 6 |
| 11 | 13 |
| 13 | 6.1 |
| 18 | 5 |
| 21 | 19 |
| 22 | 15 |
| 24 | 42 |
| 27 | 27 |
| 34 | 6.5 |
| 35 | 11 |

### Test Example 2

### Telomerase inhibitory activity in vitro:

The telomerase-inhibitory activity of Compound (I), Compound (II), Compound (IIA), Compound (IIB), Compound (IIC), Compound (III), Compound (IIIA), or *N*-lower alkyl-substituted compounds thereof (the lower alkyl of the *N*-lower alkyl-substituted compound is present on the nitrogen atom of the pyrrole ring in the porphyrin ring) or metal-coordinated compounds thereof was measured in accordance with a known method (U.S. Patent 5,760,062).

That is, a dimethyl sulfoxide (DMSO) solution of a test compound was mixed with telomerase obtained by partially purifying a nucleus extraction solution, wherein nucleus is derived from HEK293 cell, in the presence of a substrate, oligodeoxynucleotide, and deoxynucleotide triphospate, and the mixture was incubated. The resulting reaction product (DNA having a telomere sequence) was adsorbed on a membrane, and hybridization was carried out by using a labeled oligonucleotide probe having a sequence complementary to the telomere sequence. Inhibitory ratio was calculated based on the ratio of the signal strength of the label on the membrane in the presence of the test compound relative to the signal strength in the absence of the test compound (control). The compound concentration inhibiting 50% of the enzyme activity relative to that of the control was determined as IC₅₀. Table 4 shows the results of the measurement of the inhibitory activity of Compound (I), Compound (II), Compound (IIA), Compound (IIB), Compound (IIC), Compound (III), Compound (IIIA), or *N*-lower alkyl-substituted compounds thereof (the lower alkyl of the *N*-lower alkyl-substituted compound is present on the nitrogen atom of the pyrrole ring in the porphyrin ring) or metal-coordinated compounds thereof.

**Table 4(1)**

| Compound No. | IC₅₀ (µ mol/L) |
|---|---|
| 36 | 3.9 |
| 39 | 3.7 |
| 41 | 3.8 |
| 42 | 60 |
| 43 | 6.8 |
| 44 | 20 |
| 45 | 2.4 |
| 47 | <3 |
| 48 | 48 |
| 49 | 22 |
| 56 | 25 |
| 61 | 36 |
| 67 | 1.6 |

**Table 4(2)**

| Compound No. | IC₅₀ (µ mol/L) |
|---|---|
| 68 | 1.2 |
| 71 | 4.3 |
| 72 | 1.6 |
| 75 | 10.5 |
| 81 | 47 |
| 82 | 10 |
| 85 | 36 |
| 87 | 1.1 |
| 101 | 7.8 |
| 102 | 56 |
| 103 | 51 |
| 108 | 4.8 |

### Test Example 3

### Telomere inhibitory activity in cell system:

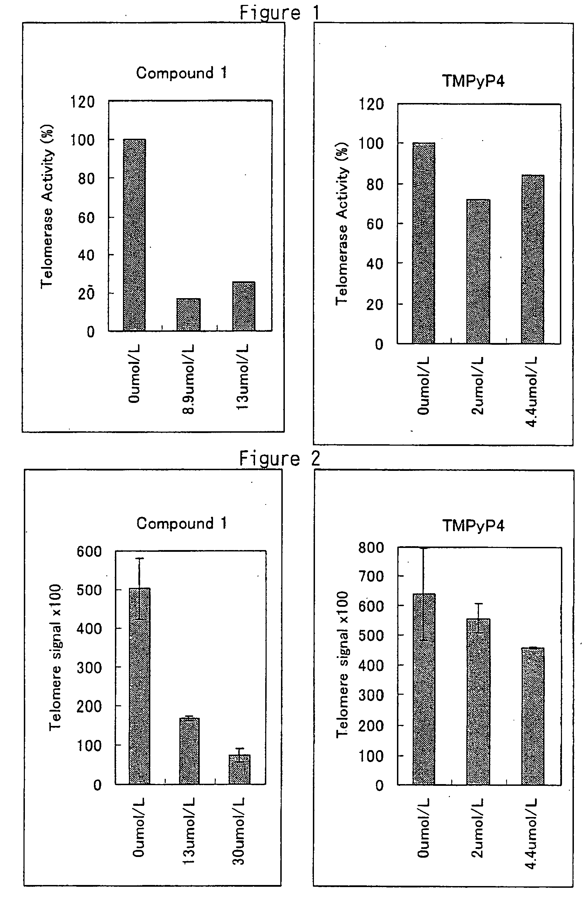

After a test compound was allowed to contact with human leukemia cell line CCRF-CEM for 7 days, a cell extraction solution was prepared and the enzyme activity was measured by a known method (U.S. Patent 5,629,154). That is, the cell extraction solution was prepared using a buffer solution containing 0.5% CHAPS {3-[(3-cholamidopropyl)dimethylammonio]-1-propanesulfonate}. using the extraction solution, TRAP (Telomeric Repeat Amplification Protocol) assay was carried out (TRAP_{EZE}™ ELISA Telomerase Detection Kit, manufactured by Oncor Co.) Inhibitory ratio was calculated based on the ratio of the enzyme activity in the extraction solution from the cells treated with the test compound relative to the enzyme activity in the extraction solution from the test compound-untreated cells. The results are shown in Fig. 1.

Compound 1 inhibited intracellular telomerase within the concentrations (8.9, 13 µmol/L) at which no remarkable cytotoxicity was shown. On the other hand, TMPyP4 did not inhibit telomerase within the concentrations (2, 4.4 µmol/L) at which no remarkable cytotoxicity was shown. Furthermore, in TMPyP4, no cellular protein was recovered at higher concentrations because of the cytotoxicity. Therefore, Compound 1 of the present invention is superior to TMPyP4 in view of the property of inhibiting telomerase within the concentrations at which no cytotoxicity is shown.

### Test Example 4

### Induction of the reduction of telomere in cell system:

After a test compound was. allowed to contact with human leukemia cell line CCRF-CEM for 21 days, a chromosomal DNA was extracted and the telomere length was measured by a known method (*Biotechniques, 23*: 476-484 (1997)). That is, the extracted chromosomal DNA was adsorbed on a membrane, and hybridization was carried out by using a labeled oligonucleotide probe having a sequence complementary to the telomere sequence. The signal of the label on the membrane proportional to the telomere DNA length was detected and digitalized. The results are shown in Fig. 2.

Compound 1 induced telomere reduction within the concentrations (13, 30 µmol/L) at which no remarkable cytotoxicity was shown. On the other hand, TMPyP4 did not induce telomere reduction within the concentrations (2, 4.4 µmol/L) at which no remarkable cytotoxicity was shown. Furthermore, in TMPyP4, no chromosomal DNA was recovered at higher concentrations because of the cytotoxicity.

From the above results, it was shown that Compound 1 was a substance which inhibited telomerase and also induced the telomere redction within the concentrations at which no cytotoxicity was shown. Because these phenomena were not shown in TMPyP4, it is concluded that Compound 1 of the present invention exhibits more excellent effects as a telomerase inhibitor.

Compound (I), Compound (II), Compound (IIA), Compound (IIB), Compound (IIC), Compound (III), Compound (IIIA), *N*-lower alkyl-substituted compounds thereof or metal-coordinated compounds thereof, or pharmaceutically acceptable salts thereof can be used as they are or in various pharmaceutical forms depending on the pharmacological activity and the purpose of administration. The pharmaceutical composition of the present invention comprising Compound (I), Compound (II), Compound (IIA), Compound (IIB), Compound (IIC), Compound (III), Compound (IIIA), an *N*-lower alkyl-substituted compound thereof or a metal-coordinated compound thereof, or a pharmaceutically acceptable salt thereof can be produced by homogeneously mixing an effective amount of Compound (I), Compound (II), Compound (IIA), Compound (IIB), Compound (IIC), Compound (III), Compound (IIIA), an *N*-lower alkyl-substituted compound thereof or a metal-coordinated compound thereof, or a pharmaceutically acceptable salt thereof with pharmaceutically acceptable carrier(s). Depending on the purpose and method of administration, these can be administered orally or parenterally in the form of tablets, granules, powders, capsules, syrups, injections, or the like which are prepared by the conventional manner. For example, in tablets, tablets containing 50 to 2000 mg of the active ingredient per one tablet is suitably used. In preparing the tablets, excipients (e.g., lactose, glucose, lactose, sucrose, mannitol, methyl cellulose, *etc.*), disintegrators (e.g., starch, sodium alginate, calcium carboxymethyl cellulose, crystalline cellulose, *etc.*), lubricants (e.g., magnesium stearate, talc, *etc*.), binders (e.g., gelatin, polyvinyl alcohol, polyvinyl pyrrolidone, hydroxypropyl cellulose, methyl cellulose, etc.), surfactants (e.g., sucrose fatty acid esters, sorbitol fatty acid esters, *etc.*), or the like can be used according to the conventional manner. In preparing the granules, excipients (e.g., lactose, sucrose, *etc.),* disintegrators (starch *etc.*), binders (gelatin *etc*.), or the like can be used according to the conventional manner.

In preparing the powders, excipients (e.g., lactose, mannitol, *etc.*) or the like can be used according to the conventional manner. In preparing the capsules, capsules containing 50 to 200 mg of the active ingredient per one capsule is suitably used. In preparing the capsules, gelatin, water, sucrose, gum arabic, sorbitol, glycerin, crystalline cellulose, magnesium stearate, talc, or the like can be used according to the conventional manner.

In preparing the syrups, sugars (e.g., olive oil, peanuts oil, ethyl oleate, propylene glycol, etc.), solubilizers (e.g., sodium benzoate, sodium salicylate, urethanes, *etc.),* isotonizing agent (e.g., sodium chloride, glucose, *etc.),* preservatives (e.g., phenol, cresol, p-hydroxybenzoic acid esters, chlorobutanol, *etc*.), antioxidants (e.g., ascorbic acid, sodium pyrosulfite, *etc.*), or the like can be used according to the conventional manner. The injections are used usually for intravenous administration, but intra-arterial administration, intraperitoneal administration, intrathoracic administration, and the like are also possible.

The dose varies depending on the administration mode, age, symptoms or the like, and the administration mode can also varies depending on the symptoms or the dose. For example, it is possible to administer an active ingredient in a dose range of 0.25 to 100 mg/kg in intravenous administration as injections, and in a dose range of 1 to 800 mg/kg in oral administration.

Next, the embodiments of the present invention will be explained with reference to Examples, Reference Examples, and Formulation Example.

The physicochemical data of each compound shown in Examples and Reference Examples described below were measured by the following instruments.
- MS: JEOL HX/HX110A
- ¹H NMR: JEOL Lambda300 (300 MHz)

In Examples and Reference Examples described below, in the physicochemical data of the compounds, "FABMS" means mass spectra by a "FAB" method, and "calculated" means theoretical values based on the molecular formulae. In proton nuclear magnetic resonance (¹H NMR) spectra, an exchangeable hydrogen is sometimes not clearly observed depending on the compounds or the measuring conditions. "br" means a broad signal.

In Examples and Reference Examples descried below, "usual post-treatment" means the following treatment after the reaction.

After the reaction at each step, water, an acid, a buffer solution or the like is optionally added to the reaction mixture, and the mixture is extracted with a water-insoluble solvent such as ethyl acetate, ether, chloroform, dichloromethane, or the like. The extract solution is washed with water, an aqueous sodium chloride solution, or the like and then dried over anhydrous sodium sulfate, and the solvent is removed by evaporation under reduced pressure.

### BEST MODE FOR CARRYING OUT THE INVENTION

### Example 1

### Synthesis of Compound 65:

Compound 57 (0.05 g, 0.07 mmol) obtained in Reference Example 17 was dissolved in chloroform (5 mL), and methyl (triphenylphosphoranylidene)acetate (0.062 g, 0.18 mmol) was added thereto. The mixture was stirred for 2 hours at room temperature and then concentrated. The concentrate was purified by preparative thin-layer chromatography (developed with chloroform : methanol = 25 : 1) to obtain Compound 65 (0.036 g, yield 61.4%).
¹H NMR (CDCl₃) δ ppm; 9.92, 9.85, 9.81, 9.75 (each s, 4H), 9.14 (dd, J = 16.1, 19.5 Hz, 2H), 6.97 (dd, J = 7.9, 16.1 Hz, 2H), 4.34 (m, 4H), 4.04 (s, 6H), 3.58, 3.52, 3.51, 3.50 (each s, 12H), 3.33 (q, J = 7.4 Hz, 4H), 3.16 (q, J = 7.4 Hz, 4H), 3.02 (q, J = 7.2 Hz, 4H), 0.99 (m, 6H), 0.78 (m, 6H), -4.05 (s, 2H)
FABMS m/z 789 (M+H)⁺ calculated for C₄₆H₅₆N₆O₆ = 788

### Example 2

### Synthesis of Compound 66:

Compound 57 (0.34 g, 0.5 mmol) obtained in Reference Example 17 was dissolved in chloroform (80 mL), and [(*tert*-butoxycarbonyl)methylene]triphenylphosphine (0.94 g, 2.5 mmol) was added thereto, followed by heating under reflux for 4 hours. The temperature was lowered to room temperature. After concentration, the concentrate was purified by silica gel column chromatography (eluted with chloroform : methanol = 100 : 0 to 99 : 1) and crystallized by using warm chloroform and hexane to obtain Compound 66 (0.30 g, yield 67.0%).
¹H NMR (CDCl₃) δ ppm; 10.32, 10.20, 10.15, 10.09 (each s, 4H), 9.31 (dd, J = 3.5, 16.1 Hz, 2H), 7.03 (d, J = 16.1 Hz, 2H), 4.36 (m, 4H), 3.76, 3.75, 3.58, 3.57 (each s, 12H), 3.33 (m, 4H), 3.18 (q, J = 7.2 Hz, 4H), 3.02 (t, J = 7.2 Hz, 4H), 1.69 (s, 18H), 1.07 (m, 6H), 0.84 (m, 6H), -3.52 (s, 2H)
FABMS m/z 873 (M+H)⁺ calculated for C₅₂H₆₈N₆O₆ = 872

### Example 3

### Synthesis of Compound 67:

Compound 57 (0.04 g, 0.58 mmol) obtained in Reference Example 17, pyridine (23 mL), and piperazine (0.007 mL) were heated under reflux for 10 minutes, and the temperature was lowered to 95°C. Then, a solution obtained by dissolving malonic acid (1.4 g) in pyridine (14 mL) and piperidine (0.023 mL) was added dropwise thereto. After the addition, the mixture was heated under reflux for 10 minutes, and the temperature was lowered to 95°C. Then, a solution obtained by dissolving malonic acid (0.46 g) in pyridine (4.65 mL) and piperidine (0.005 mL) was again added dropwise thereto. The mixture was cooled to room temperature and concentrated, and the concentrate was purified by preparative thin-layer chromatography (developed with chloroform : methanol = 8 : 3) to obtain Compound 67 (0.02 g, yield 45.3%).
FABMS m/z 761 (M+H)⁺ calculated for C₄₄H₅₂N₆O₆ = 760

### Example 4

### Synthesis of Compound 68:

Compound 66 (0.13 g, 0.15 mmol) obtained in Example 2 was dissolved in formic acid (20 mL), and 10% palladium-carbon (0.2 g) was added thereto. Hydrogen was added thereto and the mixture was heated under reflux for 3 hours. The temperature was lowered to room temperature. After the filtration through celite, the filtrate was concentrated, and the concentrate was purified by preparative thin-layer chromatography (developed with chloroform : methanol = 17 : 2) to obtain Compound 68 (0.045 g, yield 39.5%).
¹H NMR (CDCl₃+CD₃OD) δ ppm; 9.99 (m, 4H), 4.31 (m, 8H), 3.74 (m, 4H), 3.57, 3.55, 3.54, 3.53 (each s, 12H), 3.08 (m, 8H), 2.96 (m, 4H), 0.94 (m, 6H), 0.78 (m, 6H)
FABMS m/z 765 (M+H)⁺ calculated for C₄₄H₅₆N₆O₆ = 764

### Example 5

### Synthesis of Compound 69:

1,3-Dioxolan-2-ylmethyltriphenylphosphonium bromide (0.05 g, 0.11 mmol), potassium carbonate (0.005 g, 0.036 mmol) and 18-crown-6 (0.0008 g, 0.003 mmol) were dissolved in toluene (5 mL), followed by stirring at room temperature for 30 minutes. Compound 57 (0.02 g, 0.03 mmol) obtained in Reference Example 17 was added thereto, followed by stirring for another 20 hours. The temperature was raised to 90°C, the mixture was stirred for 2 hours, and then the temperature was lowered to room temperature. After the usual post-treatment, the mixture was dissolved in methanol (5 mL), and 1 mol/L hydrochloric acid (1.1 ml) was added thereto. The mixture was heated under reflux for 5 hours. Then, the temperature was lowered to room temperature, and the mixture was partitioned for extraction with chloroform and a saturated aqueous sodium chloride solution. The organic layer was dried over sodium sulfate and then concentrated. The concentrate was purified by preparative thin-layer chromatography (developed with chloroform : methanol = 9 : 1) to obtain Compound 69 (0.011 g, yield 48.9%).
¹H NMR (CDCl₃) δ ppm; 10.23, 10.21, 10.20, 10.19 (each s, 4H), 10.07 (s, 1H), 9.97 (s, 1H), 8.94 (dd, J = 7.1, 16.0 Hz, 2H), 7.47 (dd, J = 7.7, 16.0 Hz, 2H), 4.43 (m, 4H), 3.78, 3.73, 3.65, 3.63 (each s, 12H), 3.41 (q, J = 7.2 Hz, 4H), 3.26 (q, J = 6.6 Hz, 4H), 3.15 (q, J = 6.6 Hz, 4H), 1.07 (t, J = 7.2 Hz, 6H), 0.94 (t, J = 7.2 Hz, 6H), -3.34 (s, 2H)
FABMS m/z 729 (M+H)⁺ calculated for C₄₄H₅₂N₆O₄ = 728

### Example 6

### Synthesis of Compound 70:

Compound 70 (0.055 g, yield 85.1%) was obtained by carrying out reaction and treatment in a manner similar to that in Reference Example 11 using [3-(ethoxycarbonyl)propyl]triphenylphosphonium bromide (1.7 g, 3.2 mmol), tetrahydrofuran (10 mL), a 40% sodium hexamethyldisilazane/tetrahydrofuran solution (1.85 mL) and Compound 57 (0.05 g, 0.074 mmol) obtained in Reference Example 17, and purification by preparative thin-layer chromatography (developed with hexane : ethyl acetate = 1 : 1).
¹H NMR (CDCl₃) δ ppm; 10.11 (m, 4H), 8.02 (d, J = 16.1 Hz, 1H), 7.75 (m, 1H), 6.91 (m, 1H), 6.54 (m, 1H), 4.45 (m, 4H), 4.32 (m, 2H), 3.98 (m, 2H), 3.65, 3.63, 2.96, 2.88 (each s, 12H), 3.64 (m, 4H), 3.40 (m, 4H), 3.25 (m, 4H), 3.08 (m, 4H), 2.72 (m, 2H), 2.48 (m, 2H), 2.33 (m, 2H), 1.36 (t, J = 7.2 Hz, 2H), 1.23 (t, J = 7.2 Hz, 2H), 1.05 (m, 6H), 0.90 (m, 6H), -3.63 (s, 2H)
FABMS m/z 873 (M+H)⁺ calculated for C₅₂H₆₈N₆O₆ = 872

### Example 7

### Synthesis of Compound 71:

Compound 70 (0.029 g, 0.033 mmol) obtained in Example 6 was dissolved in methanol and a 1 mol/L aqueous sodium hydroxide solution (1 mL) was added thereto. The mixture was heated under reflux for 3 hours and the temperature was lowered to room temperature. After concentration, the concentrate was partitioned for extraction with chloroform and 1 mol/L hydrochloric acid. The organic layer was dried over sodium sulfate and then concentrated. The concentrate was purified by preparative thin-layer chromatography (developed with chloroform : methanol = 17 : 1) to obtain Compound 71 (0.012 g, yield 45.5%).
FABMS m/z 817 (M+H)⁺ calculated for C₄₈H₆₀N₆O₆ = 816

### Example 8

### Synthesis of Compound 72:

Compound 70 (0.05 g, 0.09 mmol) obtained in Example 6 was dissolved in methanol (10 mL). After the addition of 10% palladium-carbon (0.1 g), hydrogen was added thereto and the mixture was heated under reflux for 6 hours. The temperature was lowered to room temperature, and after the filtration through celite, the filtrate was concentrated. Compound 72 (0.0034 g, yield 4.6%) was obtained by carrying out reaction and treatment in a manner similar to that in Example 7 using the concentrate, methanol (1.2 mL) and a 1 mol/L aqueous sodium hydroxide solution (0.5 mL), and purification by preparative thin-layer chromatography (developed with chloroform : methanol = 9 : 1).
FABMS m/z 821 (M+H)⁺ calculated for C₄₈H₆₄N₆O₆ = 820

### Example 9

### Synthesis of Compound 73:

Compound 69 (0.02 g, 0.03 mmol) obtained in Example 5 was dissolved in tetrahydrofuran (3 mL), and after cooling to -78°C, lithium aluminum hydride (0.004 g, 0.11 mmol) was added thereto, followed by stirring for 2 hours. After the usual post-treatment, Compound 73 (0.008 g, yield 39.9%) was obtained by purification using preparative thin-layer chromatography (developed with chloroform : methanol = 9 : 1).
¹H NMR (CDCl₃) δ ppm; 9.94, 9.75, 9.74, 9.66 (each s, 4H), 7.77 (d, J = 16.0 Hz, 1H), 7.64 (d, J = 16.0 Hz, 1H), 6.81 (dt, J = 5.5, 16.0 Hz, 1H), 6.67 (dt, J = 5.5, 16.0 Hz, 1H), 4.63 (d, J = 5.2 Hz, 2H), 4.57 (d, J = 5.2 Hz, 2H), 4.19 (m, 4H), 3.56 (br s, 2H), 3.41, 3.33, 3.31, 3.28 (each s, 12H), 3.30 (m, 4H), 3.20 (m, 4H), 3.14 (m, 4H), 0.99 (t, J = 7.1 Hz, 6H), 0.79 (t, J = 7.1 Hz, 6H), -4.48 (s, 2H)
FABMS m/z 733 (M+H)⁺ calculated for C₄₄H₅₆N₆O₄ = 732

### Example 10

### Synthesis of Compound 74:

Compound 74 (0.03 g, yield 54.2%) was obtained by carrying out reaction and treatment in a manner similar to that in Reference Example 11 using [[4-(methoxycarbonyl)phenyl]methyl]triphenylphosphonium bromide (1.42 g, 2.9 mmol), tetrahydrofuran (8 mL), a 40% sodium hexamethyldisilazane/tetrahydrofuran solution (1.45 mL) and Compound 57 (0.04 g, 0.058 mmol) obtained in Reference Example 17, and purification by preparative thin-layer chromatography (developed with chloroform : methanol = 17 : 1).
¹H NMR (CDCl₃) δ ppm; 10.12, 10.11, 10.07, 10.01 (each s, 4H), 8.68 (t, J = 16.5 Hz, 2H), 8.25 (dd, J = 1.5, 8.3 Hz, 4H), 7.96 (t, J = 8.3 Hz, 4H), 7.69 (dd, J = 11.2, 16.5 Hz, 2H), 4.43 (t, J = 7.5 Hz, 4H), 4.03 (s, 6H), 3.70, 3.68, 3.62, 3.60 (each s, 12H), 3.41 (q, J = 6.1 Hz, 4H), 3.25 (m, 4H), 3.10 (q, J = 7.2 Hz, 4H), 1.08 (t, J = 7.1 Hz, 6H), 0.91 (t, J = 7.2 Hz, 6H), -3.61 (s, 2H)
FABMS m/z 941 (M+H)⁺ calculated for C₅₈H₆₄N₆O₆ = 940

### Example 11

### Synthesis of Compound 75:

Compound 75 (0.0036 g, yield 13.7%) was obtained by carrying out reaction and treatment in a manner similar to that in Example 7 using Compound 74 (0.03 g, 0.029 mmol) obtained in Example 10, methanol (3 mL), and a 1 mol/L aqueous sodium hydroxide solution (1 mL), and purification by preparative thin-layer chromatography (developed with chloroform : methanol = 8 : 3).
FABMS m/z 913 (M+H)⁺ calculated for C₅₆H₆₀N₆O₆ = 912

### Example 12

### Synthesis of Compound 76:

Compound 74 (0.05 g, 0.053 mmol) obtained in Example 10 was dissolved in methanol (5.5 mL), and a 1 mol/L aqueous sodium hydroxide solution (1.85 mL) was added thereto, followed by heating under reflux for 3 hours. Thereafter, the temperature was lowered to room temperature, and 5 mL of ethanol was added thereto. The precipitated salt was collected through filtration to obtain Compound 76 (0.051 g, yield 100%).
¹H NMR (CD₃OD) δ ppm; 9.72, 9.50 (each br s, 4H), 8.25 (m, 6H), 7.87 (m, 6H), 4.30 (m, 2H), 4.21 (m, 2H), 3.43 (m, 4H), 3.39 (m, 4H), 3.31, 3.30, 3.29, 3.28 (each s, 12H), 3.30 (m, 4H), 0.88 (m, 6H), 0.76 (t, J = 7.2 Hz, 6H)
FABMS m/z 913 (M-2Na+3H)⁺ calculated for C₅₆H₅₈N₆O₆ 2Na = 956

### Example 13

### Synthesis of Compound 77:

Compound 76 (0.04 g, 0.042 mmol) obtained in Example 12 was dissolved in methanol (4.5 mL). After the addition of 10% palladium-carbon (0.045 g), hydrogen was added thereto and the mixture was heated under reflux for 6 hours. After the temperature was lowered to room temperature, the mixture was filtered through celite, and the filtrate was concentrated. The concentrate was purified by preparative thin-layer chromatography (developed with chloroform : methanol = 8 : 2) to obtain Compound 77 (0.013 g, yield 32.8%).
¹H NMR (CD₃OD) δ ppm; 10.08, 9.94, 9.71, 9.70 (each s, 4H), 7.74 (t, J = 7.7 Hz, 4H), 7.16 (dd, J = 6.8, 22.2 Hz, 4H), 4.38 (m, 4H), 4.16 (m, 4H), 3.56 (m, 4H), 3.49 (m, 4H), 3.32, 3.31, 3.30, 3.29 (each s, 12H), 3.32 (m, 4H), 3.29 (m, 4H), 0.87 (t, J = 7.1 Hz, 6H), 0.73 (t, J = 6.2 Hz, 6H)
FABMS m/z 917 (M+H)⁺ calculated for C₅₆H₆₄N₆O₆= 916

### Example 14

### Synthesis of Compound 78:

Coproporphyrin III (21 mg, 0.032 mmol) was dissolved in *N,N*-dimethylformamide (4.2 mL). Diethylamine (0.98 mL, 0.95 mmol), 1-ethyl-3-(3-dimethylaminopropyl)carbodiimide monohydrochloride (180 mg, 0.95 mmol) and 1-hydroxybenzotriazole monohydrate (220 mg, 1.9 mmol) were added thereto, followed by stirring at 25°C for 2 hours. After the usual post-treatment, Compound 78 (22 mg, yield 80%) was obtained by purification using preparative thin-layer chromatography (developed with . chloroform : methanol = 20 : 0 to 20 : 1).
¹H NMR (CDCl₃) δ ppm; 10.15 (s, 2H), 10.14 (s, 1H), 10.11 (s, 1H), 4.47 (t, J = 7.5 Hz, 8H), 3.67 (d, J = 1.7 Hz, 12H), 3.37-3.50 (m, 8H), 3.20-3.30 (m, 8H), 3.04-3.17 (m, 8H), 1.05-1.16 (m, 12H), 0.87-1.00 (m, 12H), -3.76 (br s, 2H)
FABMS m/z 875 (M+H)⁺ calculated for C₅₂H₇₄N₄O₈ = 874

### Example 15

### Synthesis of Compound 79:

Compound C (0.1 g, 0.14 mmol) obtained in Reference Example 5, chloroform (10 mL), and [(ethoxycarbonyl)methylene]triphenylphosphine (0.24 g, 0.69 mmol) were allowed to react and treated in a manner similar to that in Example 1, and the resulting mixture was purified by silica gel column chromatography (eluted with chloroform : ethyl acetate = 1 : 4), and further purified by silica gel column chromatography (eluted with chloroform : methanol = 100 : 0 to 99 : 1) to obtain Compound 79 (0.07 g, yield 58.8%).
¹H NMR (CDCl₃) δ ppm; 9.97, 9.96, 9.94, 9.93 (each s, 4H), 9.26 (dd, J = 11.7, 16.1 Hz, 2H), 7.07 (dd, J = 3.3, 16.1 Hz, 2H), 4.57 (q, J = 7.2 Hz, 4H), 4.39 (m, 4H), 3.70, 3.64, 3.59, 3.58 (each s, 12H), 3.56 (m, 4H), 3.26 (q, J = 7.2 Hz, 4H), 3.12 (q, J = 6.1 Hz, 4H), 1.58 (m, 6H), 1.30 (m, 8H), 1.09 (m, 4H), -3.89 (s, 2H)
FABMS m/z 841 (M+H)⁺ calculated for C₅₀H₆₀N₆O₆ = 840

### Example 16

### Synthesis of Compound 80:

Compound 80 (0.3 g, yield 60.0%) was obtained by carrying out reaction and treatment in a manner similar to that in Example 1 using Compound C (0.35 g, 0.5 mmol) obtained in Reference Example 5, chloroform (80 mL), and [(*tert*-butoxycarbonyl)methylene]triphenylphosphine (0.47 g, 1.25 mmol), and crystallization using warm chloroform and hexane.
¹H NMR (CDCl₃) δ ppm; 10.25, 10.15, 10.10, 10.03 (each s, 4H), 9.33 (d, J = 16.0 Hz, 2H), 7.09 (d, J = 16.0 Hz, 2H), 4.41 (m, 4H), 3.81, 3.80, 3.64, 3.63 (each s, 12H), 3.57 (m, 4H), 3.25 (q, J = 7.2 Hz, 4H), 3.11 (q, J = 7.7 Hz, 4H), 1.77 (s, 18H), 1.36 (m, 8H), 1.10 (m, 4H), -3.54 (s, 2H)
FABMS m/z 897 (M+H)⁺ calculated for C₅₄H₆₈N₆O₆ = 896

### Example 17

### Synthesis of Compound 81:

Compound 79 (0.04 g, 0.05 mmol) obtained in Example 15, methanol (5 mL), and a lmol/L aqueous sodium hydroxide solution (2 mL) were allowed to react and treated in a manner similar to that in Example 7. The resulting mixture was purified by thin-layer chromatography (developed with chloroform : methanol = 8 : 3) to obtain Compound 81 (0.026 g, yield 66.8%).
FABMS m/z 785 (M+H)⁺ calculated for C₄₆H₅₂N₆O₆ = 784

### Example 18

### Synthesis of Compound 82:

Compound 79 (0.04 g, 0.05 mmol) obtained in Example 15 was dissolved in methanol (5 mL). After the addition of 10% palladium-carbon (0.05 g), hydrogen was added thereto and the mixture was heated under reflux for 6 hours. The temperature was lowered to room temperature, and after the filtration through celite, the filtrate was concentrated. Compound 82 (0.024 g, yield 57.5%) was obtained by carrying out reaction and treatment in a manner similar to that in Example 7 using the concentrate, methanol (4 mL) and a 1 mol/L aqueous sodium hydroxide solution (1.5 mL), and purification using preparative thin-layer chromatography (developed with chloroform : methanol = 8 : 1).
FABMS m/z 789 (M+H)⁺ calculated for C₄₆H₅₆N₆O₆ = 788

### Example 19

### Synthesis of Compound 83:

Compound 83 (25 mg, yield 91%) was obtained in a manner similar to that in Example 14 from coproporphyrin III (20 mg, 0.031 mmol), *N,N*-dimethylformamide (2.0 mL), piperidine (0.059 mL, 0.61 mmol), 1-ethyl-3-(3-dimethylaminopropyl)carbodiimide monohydrochloride (120 mg, 0.61 mmol) and 1-hydroxybenzotriazole monohydrate (140 mg, 1.2 mmol).
¹H NMR (CDCl₃) δ ppm; 10.01 (s, 2H), 9.98 (s, 2H), 4.43 (t, J = 7.5 Hz, 8H), 3.54-3.68 (m, 20H), 3.22-3.32 (m, 8H), 3.10-3.19 (m, 8H), 1.03-1.53 (m, 24H), -3.77 (br s, 2H)
FABMS m/z 923 (M+H)⁺ calculated for C₅₆H₇₄N₈O₄ = 922

### Example 20

### Synthesis of Compound 84:

Compound 84 (27 mg, yield 93%) was obtained in a manner similar to that in Example 14 from coproporphyrin III (20 mg, 0.031 mmol), *N,N*-dimethylformamide (1.0 mL), morpholine (0.053 mL, 0.61 mmol), 1-ethyl-3-(3-dimethylaminopropyl)carbodiimide monohydrochloride (120 mg, 0.61 mmol) and 1-hydroxybenzotriazole monohydrate (140 mg, 1.2 mmol).
¹H NMR (CDCl₃) δ ppm; 10.09 (s, 1H), 10.07 (s, 1H), 10.05 (s, 2H), 4.44 (t, J = 6.8 Hz, 8H), 3.64 (d, J = 4.8 Hz, 6H), 3.62 (d, J = 3.1 Hz, 6H), 3.50-3.67 (m, 12H), 3.19-3.40 (m, 16H), 2.78-3.18 (m, 12H), -3.83 (s, 2H)
FABMS m/z 931 (M+H)⁺ calculated for C₅₂H₆₆N₈O₈ = 930

### Example 23

### Synthesis of Compound 87:

From Compound D (0.037 g, 0.052 mmol) obtained in Reference 6, dichloromethane (5 mL) and thionyl chloride (0.011 mL, 0.13 mmol), the corresponding acid chloride was obtained in a manner similar to that in Reference Example 13, and then the acid chloride was reacted with diethylamine (0.03 mL, 0.26 mmol). After the usual post-treatment, Compound 87 (0.031 g, yield 87.1%) was obtained by carrying out reaction and treatment in a manner similar to that in Example 7 using the mixture obtained by the usual post-treatment, methanol (4.8 mL) and a 1 mol/L aqueous sodium hydroxide solution (1.6 mL), and purification using preparative thin-layer chromatography (developed with chloroform : methanol = 9 : 1).
FABMS m/z 765 (M+H)⁺ calculated for C₄₄H₅₆N₆O₆ = 764

### Example 24

### Synthesis of Compound 88:

Coproporphyrin III (19 mg, 0.027 mmol) was dissolved in *N,N*-dimethylformamide (3.8 mL), and thionyl chloride (0.040 mL, 0.54 mmol) was added thereto, followed by stirring at 25°C for 1 hour. After the reaction mixture was cooled to -20°C, n-propylamine monohydrochloride (100 mg, 1.1 mmol), triethylamine (0.23 mL, 1.6 mmol) and 4-(dimethylamino)pyridine (13 mg, 0.11 mmol) were added thereto, followed by stirring at 50°C for 1 hour. After the usual post-treatment, Compound 88 (7.2 mg, yield 32%) was obtained by purification using silica gel column chromatography (eluted with chloroform : methanol = 30 : 1 to 10 : 1).
¹H NMR (CDCl₃) δ ppm; 10.25 (s, 1H), 10.16 (s, 1H), 10.14 (s, 1H), 10.10 (s, 1H), 4.35-4.43 (m, 8H), 3.66 (t, J = 7.8 Hz, 12H), 2.99-3.20 (m, 16H), 1.17-1.34 (m, 8H), 0.54-0.65 (m, 12H)
FABMS m/z 819 (M+H)⁺ calculated for C₄₈H₆₆N₈O₄ = 818

### Example 25

### Synthesis of Compound 89:

Coproporphyrin III (20 mg, 0.031 mmol) was dissolved in *N,N*-dimethylformamide (1.0 mL). Dimethylamine monohydrochloride (50 mg, 0.61 mmol), 1-ethyl-3-(3-dimethylaminopropyl)carbodiimide monohydrochloride (120 mg, 0.61 mmol) and 1-hydroxybenzotriazole monohydrate (140 mg, 1.2 mmol) were added thereto, followed by stirring at 60°C for 2 hour.

After the usual post-treatment, Compound 89 (22 mg, yield 94%) was obtained by purification using silica gel column chromatography (eluted with chloroform : methanol = 30 : 0 to 30 : 1).
¹H NMR (CDCl₃) δ ppm; 11.00 (s, 2H), 10.08 (s, 1H), 10.07 (s, 1H), 4.42 (t, J = 7.3 Hz, 8H), 3.65 (s, 3H), 3.63 (s, 6H), 3.62 (s, 3H), 3.19-3.32 (m, 8H), 2.93 (d, J = 9.0 Hz, 6H), 2.90 (d, J = 7.8 Hz, 6H), 2.70 (d, J = 9.2 Hz, 6H), 2.65 (d, J = 9.0 Hz, 6H), -3.82 (s, 2H)
FABMS m/z 923 (M+H)⁺ calculated for C₄₄H₅₈N₈O₄ = 922

### Example 26

### Synthesis of Compound 90:

Compound 90 (27 mg, yield 82%) was obtained in a manner similar to that in Example 14 from coproporphyrin III (20 mg, 0.031 mmol), *N,N*-dimethylformamide (2.0 mL), di-n-butylamine (0.10 mL, 0.61 mmol), 1-ethyl-3-(3-dimethylaminopropyl)carbodiimide monohydrochloride (120 mg, 0.61 mmol) and 1-hydroxybenzotriazole monohydrate (140 mg, 1.2 mmol).
¹H NMR (CDCl₃) δ ppm; 10.15 (s, 1H), 10.14 (s, 2H), 10.12 (s, 1H), 4.46 (t, J = 8.0 Hz, 8H), 3.68 (s, 12H), 3.19-3.40 (m, 16H), 2.88-3.05 (m, 8H), 1.16-1.51 (m, 16H), 0.78-1.09 (m, 28H), 0.61 (t, J = 7.4 Hz, 3H), 0.58 (t, J = 7.5 Hz, 3H), 0.52 (t, J = 7.3 Hz, 3H), 0.48 (t, J = 7.3 Hz, 3H), -3.73 (s, 2H)
FABMS m/z 1099 (M+H)⁺ calculated for C₆₈H₁₀₆N₄O₈ = 1098

### Example 27

### Synthesis of Compound 91:

Compound 91 (27 mg, yield 90%) was obtained in a manner similar to that in Example 14 from coproporphyrin III (20 mg, 0.031 mmol), *N,N*-dimethylformamide (1.0 mL), diallylamine (0.075 mL, 0.61 mmol), 1-ethyl-3-(3-dimethylaminopropyl)carbodiimide monohydrochloride (120 mg, 0.61 mmol) and 1-hydroxybenzotriazole monohydrate (140 mg, 1.2 mmol).
¹H NMR (CDCl₃) δ ppm; 10.10 (s, 2H), 10.07 (s, 2H), 5.62-5.82 (m, 4H), 5.45-5.63 (m, 4H), 4.89-5.09 (m, 16H), 4.44 (t, J = 7.0 Hz, 8H), 3.99-4.10 (m, 8H), 3.59-3.69 (m, 20H), 3.27 (dd, J = 7.3, 15.0 Hz, 8H), -3.79 (br s, 2H)
FABMS m/z 971 (M+H)⁺ calculated for C₆₀H₇₄N₄O₈ = 970

### Example 28

### Synthesis of Compound 92:

Compound 92 (11 mg, yield 37%) was obtained in a manner similar to that in Reference Example 18 from coproporphyrin III (20 mg, 0.031 mmol), *N,N*-dimethylformamide (2.0 mL), *N,O*-dimethylhydroxylamine monohydrochloride (120 mg, 0.61 mmol), triethylamine (0.085 mL, 1.2 mmol), 1-ethyl-3-(3-dimethylaminopropyl)carbodiimide monohydrochloride (230 mg, 1.2 mmol) and 1-hydroxybenzotriazole monohydrate (290 mg, 2.4 mmol).
¹H NMR (CDCl₃) δ ppm; 10.18 (s, 1H), 10.16 (s, 1H), 10.15 (s, 1H), 10.01 (s, 1H), 4.43 (t, J = 7.0 Hz, 8H), 3.66 (s, 12H), 3.31-3.43 (m, 24H), 3.17-3.26 (m, 8H), -3.77 (s, 2H)
FABMS m/z 827 (M+H)⁺ calculated for C₄₄H₅₈N₈O₈ = 826

### Example 29

### Synthesis of Compound 93:

Compound 93 (3.6 mg, yield 14%) was obtained in a manner similar to that in Example 14 from coproporphyrin III (20 mg, 0.031 mmol), *N,N*-dimethylformamide (2.0 mL), (methylamino)acetonitrile (0.047 mL, 0.61 mmol), 1-ethyl-3-(3-dimethylaminopropyl)carbodiimide monohydrochloride (120 mg, 0.61 mmol) and 1-hydroxybenzotriazole monohydrate (140 mg, 1.2 mmol).
¹H NMR (CDCl₃) δ ppm; 10.10 (s, 1H), 10.07 (s, 1H), 10.04 (s, 1H), 10.02 (s, 1H), 4.38-4.48 (m, 8H), 4.31 (d, J = 3.5 Hz, 4H), 4.23 (d, J = 3.7 Hz, 4H), 3.64 (s, 6H), 3.62 (s, 6H), 3.22-3.38 (m, 8H), 2.83 (s, 3H), 2.80 (s, 3H), 2.76 (s, 3H), 2.69 (s, 3H), -3.86 (br s, 2H)
FABMS m/z 863 (M+H)⁺ calculated for C₄₈H₅₄N₁₂O₄ = 862

### Example 30

### Synthesis of Compound 94:

Compound 94 (31 mg, yield 94%) was obtained in a manner similar to that in Example 14 from coproporphyrin III (20 mg, 0.031 mmol), *N,N*-dimethylformamide (2.0 mL), (*N*-benzyl)methylamine (0.079 mL, 0.61 mmol), 1-ethyl-3-(3-dimethylaminopropyl)carbodiimide monohydrochloride (120 mg, 0.61 mmol) and 1-hydroxybenzotriazole monohydrate (140 mg, 1.2 mmol).
¹H NMR (CDCl₃) δ ppm; 10.01-10.23 (m, 4H), 6.74-7.16 (m, 20H), 4.39-4.62 (m, 12H), 4.19 (d, J = 11.5 Hz, 4H), 3.66 (s, 6H), 3.50-3.62 (m, 6H), 3.26-3.39 (m, 8H), 2.95 (s, 3H), 2.86 (s, 3H), 2.76 (s, 3H), 2.63 (s, 3H), -3.77 (m, 2H)
FABMS m/z 1067 (M+H)⁺ calculated for C₆₈H₇₄N₈O₄ = 1066

### Example 31

### Synthesis of Compound 95:

Compound 95 (33 mg, yield 100%) was obtained in a manner similar to that in Example 14 from coproporphyrin III (20 mg, 0.031 mmol), *N,N*-dimethylformamide (2.0 mL), methylaminoacetaldehyde dimethyl acetal (0.078 mL, 0.61 mmol), 1-ethyl-3-(3-dimethylaminopropyl)carbodiimide monohydrochloride (120 mg, 0.61 mmol) and 1-hydroxybenzotriazole monohydrate (140 mg, 1.2 mmol).
¹H NMR (CDCl₃) δ ppm; 10.17 (s, 1H), 10.12 (s, 1H), 10.11 (s, 1H), 10.08 (s, 1H), 4.38-4.49 (m, 8H), 3.69 (s, 3H), 3.68 (s, 3H), 3.67 (s, 3H), 3.66 (s, 3H), 3.41-3.52 (m, 4H), 3.07-3.38 (m, 28H), 2.79-3.03 (m, 24H), -3.81 (br s, 2H)
FABMS m/z 1059 (M+H)⁺ calculated for C₅₆H₈₂N₈O₁₂ = 1058

### Example 32

### Synthesis of Compound 96:

Compound 96 (8.5 mg, yield 32%) was obtained in a manner similar to that in Example 14 from coproporphyrin III (20 mg, 0.031 mmol), *N,N*-dimethylformamide (2.0 mL), 2-(methylamino)ethanol (0.049 mL, 0.61 mmol), 1-ethyl-3-(3-dimethylaminopropyl)carbodiimide monohydrochloride (120 mg, 0.61 mmol) and 1-hydroxybenzotriazole monohydrate (140 mg, 1.2 mmol).
¹H NMR (DMSO-d₆) δ ppm; 10.28 (s, 1H), 10.23 (s, 2H), 10.19 (s, 1H), 4.76-4.87 (m, 2H), 4.60-4.68 (m, 2H), 4.28-4.38 (m, 8H), 3.62 (s, 12H), 3.20-3.52 (m, 24H), 2.84-2.96 (m, 12H), -4.02 (m, 2H)
FABMS m/z 883 (M+H)⁺ calculated for C₄₈H₆₆N₈O₈ = 882

### Example 33

### Synthesis of Compound 97:

Coproporphyrin III (20 mg, 0.031 mmol) was dissolved in *N,N*-dimethylformamide (2.0 mL). Trimethylethylenediamine (44 mg, 0.61 mmol), 1-ethyl-3-(3-dimethylaminopropyl)carbodiimide monohydrochloride (120 mg, 0.61 mmol) and 1-hydroxybenzotriazole monohydrate (140 mg, 1.2 mmol) were added thereto, followed by stirring at 25°C for 2 hours. After the usual post-treatment, Compound 97 (10 mg, yield 37%) was obtained by purification using silica gel column chromatography (eluted with chloroform : methanol : triethylamine = 10 : 1 : 0 to 5 : 1 : 0.2).
¹H NMR (CDCl₃) δ ppm; 10.15 (s, 1H), 10.14 (s, 2H), 10.12 (s, 1H), 4.40-4.50 (m, 8H), 3.67 (s, 12H), 3.44-3.58 (m, 4H), 3.04-3.33 (m, 12H), 2.89-2.98 (m, 4H), 2.73-2.82 (m, 4H), 2.29 (s, 12H), 2.18 (s, 6H), 2.15 (s, 6H), 1.74-1.96 (m, 12H), -3.76 (br s, 2H)
FABMS m/z 991 (M+H)⁺ calculated for C₅₆H₈₆N₁₂O₄ = 990

### Example 34

### Synthesis of Compound 98:

Compound 98 (23 mg, yield 67%) was obtained in a manner similar to that in Example 14 from coproporphyrin III (20 mg, 0.031 mmol), *N,N*-dimethylformamide (2.0 mL), *N*-methyl-p-anisidine (84 mg, 0.61 mmol), 1-ethyl-3-(3-dimethylaminopropyl)carbodiimide monohydrochloride (120 mg, 0.61 mmol) and 1-hydroxybenzotriazole monohydrate (140 mg, 1.2 mmol).
¹H NMR (CDCl₃) δ ppm; 10.03 (s, 1H), 9.99 (s, 1H), 9.95 (s, 1H), 9.79 (s, 1H), 6.37 (dd, J = 8.8, 9.2 Hz, 4H), 6.25 (t, J = 9.0 Hz, 4H), 6.22 (d, J = 8.6 Hz, 2H), 6.09 (dd, J = 5.1, 8.6 Hz, 4H), 5.95 (d, J = 8.8 Hz, 2H), 4.25-4.40 (m, 8H), 3.51 (s, 3H), 3.47 (s, 6H), 3.42 (s, 3H), 3.35 (s, 3H), 3.28 (s, 3H), 3.25 (s, 3H), 3.15 (s, 3H), 3.10 (s, 3H), 3.09 (s, 3H), 3.05 (s, 6H), 2.99-3.11 (m, 8H), -3.81 (s, 2H)
FABMS m/z 1131 (M+H)⁺ calculated for C₆₈H₇₄N₈O₈ = 1130

### Example 35

### Synthesis of Compound 99:

Coproporphyrin III isopropyl ester (manufactured by Aldrich Chemical Company, Inc.) (15 mg, 0.018 mmol) was dissolved in tetrahydrofuran (7.6 mL), and lithium aluminum hydride (21 mg, 0.55 mmol) was added thereto, followed by stirring at 25°C for 4 hours. After the reaction mixture was passed through celite (R) 545, the solvent was removed by evaporation under reduced pressure to obtain Compound 99 (11 mg, yield 100%).
¹H NMR (DMSO-d₆) δ ppm; 10.38 (s, 1H), 10.36 (s, 2H), 10.21 (s, 1H), 4.82 (dd, J = 5.5, 10.5 Hz, 4H), 4.08-4.19 (m, 8H), 3.74-3.83 (m, 8H), 3.63 (s, 12H), 2.34-2.46 (m, 8H), -3.96 (br s, 2H)
FABMS m/z 599 (M+H)⁺ calculated for C₃₆H₄₆N₄O₄ = 598

### Example 36

### Synthesis of Compound 100:

Compound 99 (23 mg, 0.038 mmol) obtained in Example 35 was dissolved in *N,N*-dimethylformamide (4.6 mL), and methanesulfonyl chloride (0.072 mL, 0.92 mmol) and triethylamine (0.16 mL, 1.2 mmol) were added thereto, followed by stirring at 0°C for 1 hour. After the usual post-treatment, Compound 100 (7.5 mg, yield 21%) was' obtained by purification using silica gel column chromatography (eluted with chloroform : acetone = 20 : 0 to 20 : 1).
¹H NMR (CDCl₃) δ ppm; 9.99-10.09 (m, 4H), 4.55 (t, J = 6.0 Hz, 4H), 4.47 (t, J =6.1 Hz, 4H), 4.14-4.24 (m, 8H), 3.58-3.67 (m, 12H), 2.97 (s, 6H), 2.93 (s, 3H), 2.91 (s, 3H), 2.66-2.78 (m, 8H), -3.84 (br s, 2H)
FABMS m/z 911 (M+H)⁺ calculated for C₄₀H₅₄N₄O₁₂S₄ = 910

### Example 37

### Synthesis of Compound 101:

Compound 99 (23 mg, 0.038 mmol) obtained in Example 35 was dissolved in tetrahydrofuran (2.3 mL). Phenol (0.066 mL, 0.75 mmol), triphenylphosphine (200 mg, 0.75 mmol) and diisopropyl azodicarboxylate (0.15 mL, 0.75 mmol) were added thereto, followed by stirring at 70°C for 1.5 hours. After the usual post-treatment, Compound 101 (2.8 mg, yield 8.3%) was obtained by purification using silica gel column chromatography (eluted with n-hexane : ethyl acetate = 10 : 1 to 4 : 1).
¹H NMR (CDCl₃) δ ppm; 10.14 (s, 1H), 10.12 (s, 1H), 10.10 (s, 1H), 10.08 (s, 1H), 7.24-7.29 (m, 4H), 6.90-6.98 (m, 16H), 4.13-4.33 (m, 16H), 3.59 (d, J = 4.4 Hz, 6H), 3.47 (d, J = 7.2 Hz, 6H), 2.68-2.80 (m, 8H), -3.73 (s, 2H)
FABMS m/z 903 (M+H)⁺ calculated for C₆₀H₆₂N₄O₄ = 902

### Example 38

### Synthesis of Compound 102:

Compound 102 (9.6 mg, yield 24%) was obtained in a manner similar to that in Example 37 from Compound 99 (22 mg, 0.036 mmol) obtained in Example 35, tetrahydrofuran (2.2 mL), phthalimide (160 mg, 0.11 mmol), triphenylphosphine (280 mg, 0.11 mmol) and diisopropyl azodicarboxylate (0.21 mL, 0.11 mmol).
H NMR (CDCl₃) δ ppm; 10.01 (s, 1H), 9.99 (s, 1H), 9.93 (s, 1H), 9.92 (s, 1H), 7.18-7.59 (m, 16H), 4.04-4.21 (m, 16H), 3.62 (s, 6H), 3.57 (s, 6H), 2.67-2.89 (m, 8H), -4.13 (br s, 2H)
FABMS m/z 1115 (M+H)⁺ calculated for C₆₈H₅₈N₈O₈ = 1114

### Example 39

### Synthesis of Compound 103:

Coproporphyrin I dihydrochloride (manufactured by Aldrich Chemical Company, Inc.) (7.3 mg, 0.010 mmol) was dissolved in tetrahydrofuran (1.5 mL) and dichloromethane (1.5 mL), and thionyl chloride (0.0074 mL, 0.10 mmol) was added thereto, followed by heating under reflux for 1 hour. After the reaction mixture was cooled to room temperature, diethylamine (0.021 mL, 0.20 mmol) was added thereto, followed by stirring for 1 hour. After the usual post-treatment, Compound 103 (2.8 mg, yield 32%) was obtained by purification using preparative thin-layer chromatography (developed with chloroform : methanol = 9 : 1).
¹H NMR (DMSO-d₆) δ ppm; 10.1 (s, 4H), 4.47 (t, J = 7.1 Hz, 8H), 3.67 (s, 12H), 3.46 (q, J = 7.1 Hz, 8H), 3.24 (t, J = 8.0 Hz, 8H), 3.13 (q, J = 7.1 Hz, 8H), 1.12 (t, J = 7.1 Hz, 12H), 0.97 (t, J = 7.1 Hz, 12H), -3.73 (br s, 2H)
FABMS m/z 875 (M+H)⁺ calculated for C₅₂H₇₄N₈O₄ = 874

### Example 40

### Synthesis of Compound 104:

Thionyl chloride (1.0 mL, 14 mmol) was added to coproporphyrin I dihydrochloride (manufactured by Aldrich Chemical Company, Inc.) (10 mg, 0.014 mmol) and the mixture was heated under reflux for 1 hour. Thereafter, the reaction mixture was concentrated, the residue was dissolved in dichloromethane (1.0 mL), and di(n-propyl)amine (0.075 mL, 0.55 mmol) was added thereto, followed by stirring for 1 hour. After the usual post-treatment, Compound 104 (7.2 mg, yield 53%) was obtained by purification using preparative thin-layer chromatography (developed with chloroform : methanol = 5 : 1).
¹H NMR (CDCl₃) δ ppm; 10.14 (s, 4H), 4.46 (m, 8H), 3.66 (s, 12H), 3.36 (t, J = 7.7 Hz, 8H), 3.24 (t, J = 8.0 Hz, 8H), 3.03 (t, J = 7.7 Hz, 8H), 1.54 (q, J = 7.5 Hz, 8H), 1.43 (q, J = 7.5 Hz; 8H), 0.86 (t, J = 7.4 Hz, 12H), 0.65 (t, J = 7.4 Hz, 12H), -3.74 (s, 2H)
FABMS m/z 987 (M+H)⁺ calculated for C₆₀H₉₀N₈O₄ = 986

### Example 41

### Synthesis of Compound 105:

Compound 105 (9.4 mg, yield 74%) was obtained in a manner similar to that in Example 40 from coproporphyrin I dihydrochloride (manufactured by Aldrich Chemical Company, Inc.) (10 mg, 0.014 mmol), thionyl chloride (1.0 mL, 14 mmol), dichloromethane (1.0 mL) and piperidine (0.054 mL, 0.55 mmol).
¹H NMR (CDCl₃) δ ppm; 10.11 (s, 4H), 4.44 (m, 8H), 3.65 (s, 12H), 3.62 (m, 8H), 3.24 (t, J = 8.0 Hz, 8H), 3.17 (t, J = 5.5 Hz, 8H), 1.46 (m, 8H), 1.42 (m, 8H), 1.18 (m, 8H), -3.77 (s, 2H)
FABMS m/z 923 (M+H)⁺ calculated for C₅₆H₇₄N₈O₄ = 922

### Example 42

### Synthesis of Compound 106:

Compound 106 (3.8 mg, yield 100%) was obtained in a manner similar to that in Example 35 from coproporphyrin I isopropyl ester (manufactured by Aldrich Chemical Company, Inc.) (5.2 mg, 0.0063 mmol), tetrahydrofuran (2.6 mL) and lithium aluminum hydride (7.2 mg, 0.19 mmol).
¹H NMR (DMSO-d₆) δ ppm; 10.26 (s, 4H), 4.84 (br s, 4H), 4.15 (t, J = 6.4 Hz, 8H), 3.79 (t, J = 5.9 Hz, 8H), 3.63 (s, 12H), 2.33-2.45 (m, 8H), -3.97 (br s, 2H)
FABMS m/z 599 (M+H)⁺ calculated for C₃₆H₄₆N₄O₄ = 598

### Example 43

### Synthesis of Compound 107:

Compound 106 (8.6 mg, 0.014 mmol) obtained in Example 42 was dissolved in dimethyl sulfoxide (3 mL), and triethylamine (0.039 mL, 0.28 mmol) and sulfur trioxidepyridine (45 mg, 0.28 mmol) were added thereto, followed by stirring at room temperature for 25 minutes. After the usual post-treatment, Compound 107 (2.7 mg, yield 33%) was obtained by purification using preparative thin-layer chromatography (developed with chloroform : methanol = 6 : 1).
¹H NMR (CDCl₃) δ ppm; 10.1 (s, 4H), 10.0 (s, 4H), 4.40 (t, J = 7.7 Hz, 8H), 3.64 (s, 12H), 3.43 (t, J = 7.7 Hz, 8H), -3.76 (br s, 2H)
FABMS m/z 591 (M+H)⁺ calculated for C₃₆H₃₈N₄O₄ = 590

### Example 44

### Synthesis of Compound 108:

Compound 106 (0.006 g, 0.01 mmol) obtained in Example 42 was dissolved in acetic anhydride (3 mL), and concentrated sulfuric acid (one drop) was added thereto, followed by stirring at room temperature for 1 hour and then heating under reflux for 3 hours. The temperature was lowered to room temperature, and the mixture was neutralized with a saturated aqueous sodium hydrogen carbonate solution. After the usual post-treatment, Compound 108 (0.0045 g, yield 58.8%) was obtained by purification using preparative thin-layer chromatography (developed with hexane : ethyl acetate = 1 : 1) and further purification using preparative thin-layer chromatography (developed with chloroform : methanol = 9 : 1).
¹H NMR (CDCl₃) δ ppm; 10.06 (s, 4H), 4.43 (t, J = 6.2 Hz, 8H), 4.16 (t, J = 7.3 Hz, 8H), 3.63 (s, 12H), 2.64 (m, 8H), 2.19 (s, 12H), -3.76 (s, 2H)
FABMS m/z 767 (M+H)⁺ calculated for C₄₄H₅₄N₄O₈ = 766

### Example 45

### Synthesis of Compound 109 and Compound 110:

Compound 106 (4.8 mg, 0.0079 mmol) obtained in Example 42 was dissolved in *N,N*-dimethylformamide (0.24 mL), and bromomethyl methyl ether (0.039 mL, 0.47 mmol) and diisopropylethylamine (0.12 mL, 0.71 mmol) were added thereto, followed by stirring at 25°C for 13 hours. After the usual post-treatment, Compound 109 (2.7 mg, yield 44%) and Compound 110 (2.0 mg, yield 34%) were obtained by purification using preparative thin-layer chromatography (developed with n-hexane : ethyl acetate = 1 : 1). Compound 109
¹H NMR (CDCl₃) δ ppm; 10.13 (s, 4H), 4.78 (t, J = 2.6 Hz, 8H), 4.14-4.24 (m, 8H), 3.85 (t, J = 6.1 Hz, 8H), 3.63 (s, 12H), 3.47 (s, 12H), 2.52-2.67 (m, 8H), -3.75 (br s, 2H)
FABMS m/z 775 (M+H)⁺ calculated for C₄₄H₆₂N₄O₈ = 774

### Compound 110

¹H NMR (CDCl₃) δ ppm; 10.12-10.17 (m, 4H), 4.80 (s, 6H), 4.20 (t, J = 6.9 Hz, 8H), 3.95-4.01 (m, 2H), 3.86 (t, J = 6.1 Hz, 6H), 3.65 (s, 12H), 3.47 (s, 9H), 2.53-2.66 (m, 8H), -3.74 (br s, 2H)
FABMS m/z 731 (M+H)⁺ calculated for C₄₂H₅₈N₄O₇ = 730

### Example 46

### Synthesis of Compound 111:

In the presence of 1,8-diazabicyclo[5.4.0]-7-undecene (DBU), methyl 4-nitrobutyrate (2.2 mL, 17 mmol) was reacted with propionaldehyde (1.4 mL, 20 mmol) and the resulting product was allowed to react with acetic anhydride to obtain methyl 5-acetoxy-4-nitroheptanoate. Thereafter, the product was allowed to react with tert-butyl isocyanoacetate (2.32 g, 16.5 mmol) according to the method described in a literature (Japanese Published Unexamined Patent Application No. 238585/89) to obtain the corresponding pyrrole derivative, and Compound 111 (19 mg, overall yield 0.57%) was obtained via a dipyrromethane derivative.
¹H NMR (DMSO-d₆) δ ppm; 10.13 (s, 2H), 10.12 (s, 2H), 4.44 (t, J = 7.9 Hz, 8H), 4.12 (q, J = 7.5 Hz, 8H), 3.70 (s, 12H), 3.32 (t, J = 7.9 Hz, 8H), 1.93 (t, J = 7.5 Hz, 12H), -3.72 (s, 2H)
FABMS m/z 767 (M+H)⁺ calculated for C₄₄H₅₄N₄O₈ = 766

### Example 47

### Synthesis of Compound 112:

Compound 111 (5.0 mg, 0.0065 mmol) obtained in Example 46 was suspended in methanol (3 mL), and a 1 mol/L aqueous sodium hydroxide solution (0.5 mL) was added thereto, followed by heating under reflux for 30 minutes. The reaction mixture was cooled to room temperature and the precipitated crystals were collected through filtration to obtain Compound 112 (2.7 mg, yield 52%).
¹H NMR (D₂O) δ ppm; 10.1 (br s, 4H), 4.42 (br s, 8H), 4.13 (br s, 8H), 3.16 (br s, 8H), 1.91 (br s, 12H)
FABMS m/z 799 (M+H)⁺ calculated for C₄₀H₄₂N₄Na₄O₈ = 798

Reference Examples are shown below, and the structures of Compounds A, B, C and D in Reference Examples are shown in Table 5.

### Reference Example 1

### Synthesis of Compound A:

Compound 46 (163 mg, 0.248 mmol) obtained in Reference Example 10 described below was dissolved in tetrahydrofuran (100 mL), and periodic acid dihydrate (340 mg, 1.5 mmol) and water (5 mL) were added thereto, followed by stirring at room temperature for 1 hour. After the usual post-treatment, Compound A (135 mg, yield 88%) was obtained by trituration with hexane.
¹H NMR (CDCl₃) δ ppm; 11.1 (s, 1H), 11.0 (s, 1H), 10.1 (s, 1H), 9.66 (s, 2H), 9.37 (s, 1H), 4.30 (t, J = 7.9 Hz, 2H), 4.27 (t, J = 7.9 Hz, 2H), 4.17 (q, J = 7.2 Hz, 2H), 4.16 (q, J = 7.2 Hz, 2H), 3.57 (s, 3H), 3.47 (s, 3H), 3.46 (s, 3H), 3.31 (s, 3H), 3.23 (t, J = 7.5 Hz, 2H), 3.21 (t, J = 7.5 Hz, 2H); 1.18 (t, J = 7.2 Hz, 3H), 1.17 (t, J = 7.2 Hz, 3H),-4.85 (br s, 2H)
FABMS m/z 623 (M+H)⁺ calculated for C₃₆H₃₈N₄O₆ = 622

### Reference Example 2

### Synthesis of Compound 45:

Compound A (17 mg, 0.027 mmol) obtained in Reference Example 1 was dissolved in ethanol (5 mL), and sodium borohydride (5.0 mg, 0.13 mmol) was added thereto, followed by heating under reflux for 2 hours. After the usual post-treatment, a diol compound (11 mg, yield 65%) was obtained by purification using preparative thin-layer chromatography (developed with chloroform : methanol = 12 : 1).
¹H NMR (DMSO-d₆) δ ppm; 10.44 (s, 1H), 10.40 (s, 1H), 10.26 (s, 1H), 10.25 (s, 1H), 5.96 (d, J = 5.3 Hz, 4H), 5.85 (t, J = 5.4 Hz, 2H), 4.40 (t, J = 7.3 Hz, 4H), 4.02 (q, J = 7.3 Hz, 4H), 3.70 (s, 3H), 3.68 (s, 3H), 3.65 (s, 3H), 3.63 (s, 3H), 3.29 (t, J = 7.7 Hz, 4H), 1.01 (t, J = 7.2 Hz, 6H),-3.93 (s, 2H)
FABMS m/z 627 (M+H)⁺ calculated for C₃₆H₄₂N₄O₆ = 626

The diol compound (9.0 mg, 0.014 mmol) obtained above was dissolved in methanol (3 mL), and a 1 mol/L aqueous sodium hydroxide solution (0.5 mL) was added thereto, followed by stirring at room temperature for 17 hours. Ethanol (2 mL) was added to the reaction mixture and the precipitated crystals were collected through filtration to obtain Compound 45 (7.8 mg, yield 91%).
¹H NMR (D₂O) δ ppm; 9.60 (s, 1H), 9.21 (br s, 1H), 9.09 (br s, 1H), 8.46 (br s, 1H), 5.77 (br s, 2H), 5.58 (br s, 2H), 4.42 (br s, 4H), 3.59 (s; 3H), 3.55 (s, 3H), 3.40 (br s, 3H), 3.20 (br s, 3H), 3.09 (t, J = 7.5 Hz, 2H), 3.07 (t, J = 7.5 Hz, 2H)
FABMS m/z 571 (M+H)⁺ calculated for C₃₂H₃₄N₄O₆ = 570

### Reference Example 3

### Synthesis of Compound 64:

Coproporphyrin II tetramethyl ester (3.0 mg, 0.0042 mmol) known in a literature (Japanese Published Unexamined Patent Application No. 238585/89) was treated in a manner similar to the method in Example. 47 to obtain Compound 64 (2.2 mg, yield 71%).
¹H NMR (D₂O) δ ppm; 9.28 (br s, 4H), 4.28 (br s, 8H), 3.50 (br s, 12H), 2.92 (br s, 8H)
FABMS m/z 655 (M+H)⁺ calculated for C₃₆H₃₈N₄O₈ = 654

### Reference Example 4

### Synthesis of Compound B:

Compound B (1.3 g, yield 88.8%) was obtained by carrying out reaction and treatment in a manner similar to that in Reference Example 16 described below using Compound 55 (1.04 g, 1.5 mmol) obtained in Reference Example 15 described below, dioxane (315 mL), water (36 mL), osmium oxide (0.008 g, 0.32 mmol), 4-methylmorpholine *N*-oxide (0.53 g, 3.5 mmol) and sodium hydrogen sulfite (3 g, 28.5 mmol), and crystallization using warm chloroform and hexane.
¹H NMR (CDCl₃) δ ppm; 10.53, 10.46, 10.05, 9.93 (each s, 4H), 4.80 (m, 1H), 4.62 (m, 1H), 4.35 (m, 2H), 4.23 (m, 2H), 3.74, 3.73, 3.69, 3.63 (each s, 12H), 3.64 (m, 8H), 2.94 (m, 4H), 2.86 (m, 4H), 2.37 (br s, 2H), 2.21 (br s, 2H), 1.53 (m, 8H), 1.13 (m, 4H)
FABMS m/z 765 (M+H)⁺ calculated for C₄₄H₅₆N₆O₆ = 764

### Reference Example 5

### Synthesis of Compound C:

Compound C (0.77 g, yield 84.6%) was obtained by carrying out reaction and treatment in a manner similar to that in Reference Example 17 described below using Compound B (1.0 g, 1.3 mmol) obtained in Reference Example 4, tetrahydrofuran (845 mL), periodic acid (1.7 g, 7.5 mmol) and water (35 mL), and crystallization using warm chloroform and hexane.
¹H NMR (CDCl₃) δ ppm; 11.18, 11.14, 10.22, 9.97, 9.74, 9.53 (each s, 6H), 4.30 (m, 4H), 3.68, 3.51, 3.50, 3.49 (each s, 12H), 3.59 (m, 4H), 3.25 (m, 4H), 3.23 (m, 4H), 1.43 (m, 4H), 1.39 (m, 4H), 1.22 (m, 4H), -4.50 (s, 2H)
FABMS m/z 701 (M+H)⁺ calculated for C₄₂H₄₈N₆O₆ = 700

### Reference Example 6

### Synthesis of Compound D:

Compound D (0.037 g, yield 71.5%) was obtained by carrying out reaction and treatment in a manner similar to that in Example 4 using Compound 86 (0.06 g, 0.07 mmol) obtained in Reference Example 21 described below, formic acid (9 mL) and 10% palladium-carbon (0.09 g), and purification using preparative thin-layer chromatography (developed with chloroform : methanol = 9 : 1).
FABMS m/z 711 (M+H)⁺ calculated for C₄₀H₄₆N₄O₈ = 710

### Reference Example 7

### Synthesis of Compound 36:

Coproporphyrin I dihydrochloride (manufactured by Aldrich Chemical Company, Inc.) (20 mg, 0.027 mmol) was dissolved in methanol (4.0 mL), and a 10% hydrogen chloride-methanol solution was added thereto, followed by stirring at 25°C for 3 hours. After the usual post-treatment, Compound 36 (2.0 mg, yield 11%) was obtained by purification using silica gel column chromatography (eluted with chloroform : methanol : acetic acid = 95 : 25 : 5).
¹H NMR (CDCl₃) δ ppm; 9.99 (s, 1H), 9.96 (s, 1H), 9.90 (s, 2H), 7.18 (s, 9H), 4.24-4.34 (m, 8H), 3.48-3.63 (m, 12H), 3.13-3.26 (m, 8H), -3.34 (s, 2H)
FABMS m/z 697 (M+H)⁺ calculated for C₃₉H₄₄N₄O₈ = 696

### Reference Example 8

### Synthesis of Compound 43:

Compound 43 (4.0 mg, yield 24%) was obtained in a manner similar to that in Reference Example 7 from coproporphyrin I dihydrochloride (manufactured by Aldrich Chemical Company, Inc.) (15 mg, 0.021 mmol), 2-propanol (10 mL) and 12 mol/L hydrochloric acid.
¹H NMR (CDCl₃) δ ppm; 10.0 (br s, 1H), 9.99 (br s, 1H), 9.93 (br s, 1H), 9.89 (br s, 1H), 5.0-5.2 (m, 3H), 4.2-4.4 (m, 8H), 3.64 (s, 3H), 3.61 (s, 3H), 3.56 (s, 3H), 3.54 (s, 3H), 3.1-3.4 (m, 8H), 1.14 (d, J = 6.2 Hz, 12H), 1.13 (d, J = 6.2 Hz, 6H), -4.01 (br s, 2H)
FABMS m/z 781 (M+H)⁺ calculated for C₄₅H₅₆N₄O₈ = 780

### Reference Example 9

### Synthesis of Compound 44:

Coproporphyrin III (20 mg, 0.028 mmol) was dissolved in benzyl alcohol (1.0 mL), and concentrated sulfuric acid (0.020 mL, 0.21 mmol) was added thereto, followed by stirring at 25°C for 1 hour. After the usual post-treatment, Compound 44 (48 mg, yield 78%) was obtained by purification using silica gel column chromatography (eluted with n-hexane : ethyl acetate = 1 : 1).
¹H NMR (CDCl₃) δ ppm; 10.09 (s, 1H), 10.03 (s, 3H), 6.96-7.20 (m, 20H), 5.12 (s, 4H), 5.03 (d, J = 1.8 Hz, 4H), 4.42 (t, J = 6.6 Hz, 8H), 3.54-3.63 (m, 12H), 3.32 (t, J = 7.7 Hz, 8H), -3.78 (br s, 2H)
FABMS m/z 1014 (M+H)⁺ calculated for C₄H₈N₃O₄ = 1013

### Reference Example 10

### Synthesis of Compound 46:

Protoporphyrin IX diethyl ester (255 mg, 0.413 mmol) known in a literature (*Tetrahedron Lett., 29:* 1421 (1988)) was dissolved in 1,4-dioxane (100 mL). Water (10 mL), osmium tetraoxide (21 mg, 0.082 mmol), and N-methylmorpholine *N*-oxide (149 mg, 1.24 mmol) were added thereto, followed by stirring at room temperature for 19 hours. Sodium hydrogen sulfite (1.5 g) was added thereto, followed by stirring at 65°C for 20 minutes. The reaction mixture was filtered and the solvent was removed by evaporation under reduced pressure. Water (150 mL) was added thereto and the precipitated crystals were collected through filtration. Compound 46 (273 mg, yield 96%) was obtained by trituration of the crystals with isopropyl ether.
¹H NMR (DMSO-d₆) δ ppm; 10.7-10.8 (m, 2H), 10.243 (s, 1H), 10.237 (s, 1H), 6.2-6.4 (m, 4H), 5.2-5.3 (m, 2H), 4.3-4.5 (m, 6H), 4.1-4.3 (m, 2H), 4.03 (q, J = 7.2 Hz, 2H), 4.02 (q, J = 7.2 Hz, 2H), 3.72 (s, 3H), 3.68 (s, 3H), 3.65 (s, 3H), 3.61 (s, 3H), 3.29 (t, J = 7.5 Hz, 4H), 1.02 (t, J = 7.2 Hz, 3H), 1.00 (t, J = 7.2 Hz, 3H), -3.97 (br s, 2H)
FABMS m/z 687 (M+H)⁺ calculated for C₃₈H₄₆N₄O₈ = 686

### Reference Example 11

### Synthesis of Compound 48:

[3-(Ethoxycarbonyl)propyl]triphenylphosphonium bromide (0.085 g, 0.2 mmol) was dissolved in tetrahydrofuran (2 mL), followed by cooling to 0°C, and then a 40% sodium hexamethyldisilazane-tetrahydrofuran solution (0.094 mL) was added thereto, followed by stirring for 20 minutes. Compound A (0.02 g, 0.032 mmol) obtained in Reference Example 1 dissolved in tetrahydrofuran (1 mL) was added to the reaction solution, followed by stirring for another 30 minutes. The temperature was raised to room temperature and the mixture was concentrated. Thereafter, the mixture was partitioned for extraction with chloroform and water, and the organic layer was dried over sodium sulfate. Then, the organic layer was concentrated and the concentrate was purified by preparative thin-layer chromatography (developed with hexane : ethyl acetate = 1 : 1) to obtain Compound 48 (0.005 g, yield 21.2%).
FABMS m/z 721 (M+H)⁺ calculated for C₄₂H₄₈N₄O₇ = 720

### Reference Example 12

### Synthesis of Compounds 49 and 50:

Compound 49 (0.0052 g, yield 9.2%) and Compound 50 (0.0055 g, yield 11.4%) were obtained by carrying out reaction and treatment in a manner similar to that in Reference Example 11 using [[4-(methoxycarbonyl)phenyl]methyl]triphenylphosphonium bromide (0.18 g, 0.37 mmol), tetrahydrofuran (2 mL), a 40% sodium hexamethyldisilazane-tetrahydrofuran solution (0.19 mL) and Compound A (0.04 g, 0.064 mmol) obtained in Reference Example 1, and purification by preparative thin-layer chromatography (developed with chloroform : methanol = 50 : 1).
Compound 49
¹H NMR (CDCl₃) δ ppm; 10.22, 10.18, 10.09, 10.07 (each s, 4H), 8.76 (dd, J = 1.7, 16.4 Hz, 2H), 8.26 (d, J = 8.2 Hz, 4H), 8.00 (d, J = 8.2 Hz, 4H), 7.74 (d, 16.4 Hz, 2H), 4.41 (m, 4H), 4.15 (m, 4H), 4.03 (s, 6H), 3.78, 3.76, 3.65, 3.63 (each s, 12H), 1.15 (t, J = 7.2 Hz, 6H), 0.88 (m, 4H), -3.54 (s, 2H)
FABMS m/z 887 (M+H)⁺ calculated for C₅₄H₅₄N₄O₈ = 886
Compound 50
FABMS m/z 755 (M+H)⁺ calculated for C₄₅H₄₆N₄O₇ = 754

### Reference Example 13

### Synthesis of Compound 53:

Protoporphyrin IX disodium salt (manufactured by Aldrich Chemical Company, Inc.) (1.2 g, 2.0 mmol) was dissolved in dichloromethane (200 mL), and thionyl chloride (0.43 mL, 5.0 mmol) was added thereto, followed by heating under reflux for 1 hour. The temperature was cooled to room temperature and diethylamine (1.4 mL, 10 mmol) was added thereto, followed by stirring at room temperature for 2 hours. After the usual post-treatment, Compound 53 (1.3 g, yield 100%) was obtained by crystallization using warm chloroform and hexane.
¹H NMR (CDCl₃) δ ppm; 10.27, 10.10, 9.99, 9.85 (each s, 4H), 8.32 (m, 2H), 6.40 (dd, J = 5.1, 17.8 Hz, 2H), 6.21 (dd, J = 3.0, 12.2 Hz, 2H), 4.45 (t, J = 7.7 Hz, 4H), 3.75, 3.73, 3.54, 3.52 (each s, 12H), 3.40 (q, J = 7.2 Hz, 4H), 3.16 (t, J = 7.8 Hz, 4H), 3.04 (q, J = 7.2 Hz, 4H), 1.07 (t, J = 7.2 Hz, 6H), 0.87 (m, 6H), -3.61 (s, 2H)
FABMS m/z 673 (M+H)⁺ calculated for C₄₂H₅₂N₆O₂ = 672

### Reference Example 14

### Synthesis of Compound 54:

Protoporphyrin IX disodium salt (manufactured by Aldrich Chemical Company, Inc.) (0.061 g, 0.1 mmol) was dissolved in dichloromethane (10 mL), and thionyl chloride (0.02 mL, 0.25 mmol) was added thereto, followed by heating under reflux for 1 hour. The temperature was cooled to room temperature and di(n-propyl)amine (0.07 mL, 0.5 mmol) was added thereto, followed by stirring at room temperature for 2 hours. After the usual post-treatment, Compound 54 (0.037 g, yield 51%) was obtained by purification using preparative thin-layer chromatography (developed with chloroform : methanol = 9 : 1).
¹H NMR (CDCl₃) δ ppm; 10.27, 10.21, 10.13, 10.08 (each s, 4H), 8.32 (m, 2H), 6.39 (dd, J = 1.7, 2.8 Hz, 2H), 6.20 (dt, J = 1.9, 11.5 Hz, 2H), 4.44 (t, J = 7.8 Hz, 4H), 3.75, 3.74, 3.67, 3.64 (each s, 12H), 3.30 (t, J = 7.7 Hz, 4H), 3.25 (t, J = 7.8 Hz, 4H), 3.00 (t, J = 7.7 Hz, 4H), 1.35 (m, 8H), 0.83 (t, J = 7.3 Hz, 6H), 0.56 (t, J = 7.3 Hz, 6H), -3.58 (s, 2H)
FABMS m/z 729 (M+H)⁺ calculated for C₄₆H₆₀N₆O₂ = 728

### Reference Example 15

### Synthesis of Compound 55:

Protoporphyrin IX disodium salt (manufactured by Aldrich Chemical Company, Inc.) (1.2 g, 2.0 mmol) was dissolved in dichloromethane (200 mL), and thionyl chloride (0.43 mL, 5.0 mmol) was added thereto, followed by heating under reflux for 1 hour. The temperature was cooled to room temperature and piperidine (1.0 mL, 10 mmol) was added thereto, followed by stirring at room temperature for 2 hours. After the usual post-treatment, Compound 55 (1.4 g, yield 100%) was obtained by crystallization using warm chloroform and hexane.
¹H NMR (CDCl₃) δ ppm; 10.26, 10.18, 10.10, 10.01 (each s, 4H), 8.31 (m, 2H), 6.39 (d, J = 17.6 Hz, 2H), 6.20 (d, J = 11.7 Hz, 2H), 4.40 (t, J = 7.2 Hz, 4H), 3.74, 3.73, 3.63, 3.61 (each s, 12H), 3.56 (m, 4H), 3.26 (t, J = 7.2 Hz, 4H), 3.10 (t, J = 5.4 Hz, 4H), 1.42 (m, 4H), 1.33 (m, 4H), 1.07 (m, 4H), -3.66 (s, 2H)
FABMS m/z 697 (M+H)⁺ calculated for C₄₄H₅₂N₆O₂ = 696

### Reference Example 16

### Synthesis of Compound 56:

Compound 53 (1 g, 1.5 mmol) obtained in Reference Example 13 was dissolved in dioxane (315 mL) and water (36 mL). Osmium oxide (0.008 g, 0.32 mmol) and 4-methylmorpholine *N*-oxide (0.53 g, 3.5 mmol) were added thereto, followed by stirring at room temperature for 24 hours. Successively, sodium hydrogen sulfite (3 g, 28.5 mmol) was added thereto and the mixture was stirred at room temperature for 1 hour and then at 65°C for 15 minutes. The reaction mixture was cooled to room temperature and filtered, and the filtrate was partitioned for extraction with chloroform and water. The organic layer was dried over sodium sulfate and then concentrated, and the concentrate was crystallized using warm chloroform and hexane to obtain Compound 56 (0.8 g, yield 72.4%).
¹H NMR (CDCl₃) δ ppm; 10.52, 10.46, 10.01, 9.89 (each s, 4H), 4.78 (m, 1H), 4.59 (m, 1H), 4.30 (m, 2H), 4.21 (m, 2H), 3.72, 3.71, 3.67, 3.63 (each s, 12H), 3.44 (t, J = 7.2 Hz, 4H), 2.89 (m, 8H), 2.42 (br s, 2H), 2.24 (br s, 2H), 1.16 (t, J = 7.2 Hz, 4H), 0.83 (m, 6H), 0.76 (m, 6H)
FABMS m/z 741 (M+H)⁺ calculated for C₄₂H₅₆N₆O₆ = 740

### Reference Example 17

### Synthesis of Compound 57:

Compound 56 (0.74 g, 1.0 mmol) obtained in Reference Example 16 was dissolved in tetrahydrofuran (650 mL), and periodic acid (1.3 g, 5.8 mmol) dissolved in water (26 mL) was added thereto, followed by stirring at room temperature for 1 hour. After the usual post-treatment, Compound 56 (0.58 g, yield 86.1%) was, obtained by crystallization using warm chloroform and hexane.
¹H NMR (CDCl₃) δ ppm; 11.20, 11.14, 10.26, 9.98, 9.83, 9.55 (each s, 6H), 4.37 (q, J = 8.9 Hz, 4H), 3.68, 3.54, 3.53, 3.51 (each s, 12H), 3.42 (m, 4H), 3.22 (m, 8H), 1.15 (m, 6H), 0.98 (t, J = 7.2 Hz, 6H), -4.50 (s, 2H)
FABMS m/z 677 (M+H)⁺ calculated for C₄₀H₄₈N₆O₆ = 676

### Reference Example 18

### Synthesis of Compound 58:

Coproporphyrin III (54 mg, 0.082 mmol) was dissolved in *N,N*-dimethylformamide (11 mL). Sarcosine methyl ester monohydrochloride (250 mg, 1.6 mmol), triethylamine (0.23 mL, 1.6 mmol), 1-ethyl-3-(3-dimethylaminopropyl)carbodiimide monohydrochloride (320 mg, 1.6 mmol) and 1-hydroxybenzotriazole monohydrate (390 mg, 3.3 mmol) were added thereto, followed by stirring at 25°C for 2 hours. After the usual post-treatment, Compound 58 (64 mg, yield 54%) was obtained by purification using silica gel column chromatography (eluted with chloroform : methanol = 50 : 0 to 50 : 1).
¹H NMR (CDCl₃) δ ppm; 10.03-10.11 (m, 4H), 4.37-4.48 (m, 8H), 4.18 (d, J = 4.4 Hz, 4H), 4.14 (d, J = 3.5 Hz, 2H), 3.84 (d, J = 9.7 Hz, 1H), 3.78 (d, J = 11.0 Hz, 1H), 3.73 (d, J = 3.0 Hz, 4H), 3.58-3.68 (m, 20H), 3.25-3.46 (m, 8H), 2.86-2.94 (m, 12H), -3.85 (br s, 2H)
FABMS m/z 994 (M+H)⁺ calculated for C₅₂H₆₆N₁₂O₈ = 993

### Reference Example 19

### Synthesis of Compound 59:

Compound 58 (44 mg, 0.044 mmol) obtained in Reference Example 18 was dissolved in methanol (2.2 mL), and a 1 mol/L aqueous sodium hydroxide solution (0.88 mL) was added thereto, followed by stirring at 100°C for 2 hours. Then, the temperature was lowered to 25°C and stirring was continued for 13 hours. After ethanol was added to the reaction mixture to form powder, which was collected by filtration to obtain Compound 59 (21 mg, yield 52%).
¹H NMR (CD₃OD) δ ppm; 9.70-10.21 (m, 4H), 4.42 (br s, 8H), 3.86-4.02 (m, 4H), 3.45-3.73 (m, 16H), 3.15-3.42 (m, 8H), 2.78-2.99 (m, 12H)
FABMS m/z 937 (M-H)⁺ calculated for C₄₈H₅₈N₈O₁₂ = 938

### Reference Example 20

### Synthesis of Compound 85:

[3-(Ethoxycarbonyl)propyl]triphenylphosphonium bromide (0.085 g, 0.2 mmol) was dissolved in tetrahydrofuran (2 mL), and the solution was cooled to 0°C. Thereto was added a 40% sodium hexamethyldisilazane/tetrahydrofuran solution (0.094 mL) and the mixture was stirred for 20 minutes.

To the reaction solution was added Compound A (0.02 g, 0.032 mmol) obtained in Reference Example 1 dissolved in tetrahydrofuran (1 mL), followed by stirring for another 30 minutes. The temperature was raised to room temperature and the mixture was concentrated. Thereafter, the mixture was partitioned for extraction with chloroform and water, and the organic layer was dried over sodium sulfate. Then, the organic layer was concentrated and the concentrate was purified by preparative thin-layer chromatography (developed with hexane : ethyl acetate = 1 : 1) to obtain Compound 85 (0.004 g, yield 14.1%).
¹H NMR (CDCl₃) δ ppm; 10.11, 10.09, 10.02, 10.00 (each s, 4H), 8.00 (d, J = 16.8 Hz, 1H), 7.77 (d, J = 12.0 Hz, 1H), 6.55 (dd, J = 12.0, 16.8 Hz, 2H), 4.34 (m, 4H), 4.14 (m, 4H), 3.96 (m, 2H), 3.69 (m, 4H), 3.66, 3.65, 3.62, 3.58 (each s, 12H), 3.27 (t, J = 7.9 Hz, 4H), 3.09 (m, 1H), 2.90 (m, 1H), 2.69 (t, J = 7.9 Hz, 2H), 2.48 (t, J = 7.3 Hz, 2H), 1.11 (m, 6H), 1.01 (m, 6H), -3.66 (s, 2H)
FABMS m/z 819 (M+H)⁺ calculated for C₄₈H₅₈N₄O₈ = 818

### Reference Example 21

### Synthesis of Compound 86:

Compound 86 (0.038 g, yield 61.4%) was obtained by carrying out reaction and treatment in a manner similar to that in Example 1 using Compound A (0.05 g, 0.08 mmol) obtained in Reference Example 1, chloroform (7 mL), and [(*tert*-butoxycarbonyl)methylene]triphenylphosphine (0.076 g, 0.13 mmol), and purification by preparative thin-layer chromatography (developed with chloroform : ethyl acetate = 95 : 5).
¹H NMR (CDCl₃) δ ppm; 10.14, 10.08, 10.03, 9.99 (each s, 4H), 9.29 (d, J = 16.3 Hz, 2H), 7.07 (d, J = 16.3 Hz, 2H), 4.38 (m, 4H), 4.13 (q, J = 7.2 Hz, 4H), 3.78, 3.76, 3.62, 3.61 (each s, 12H), 3.25 (t, J = 7.5 Hz, 4H), 1.78 (s, 18H), 1.13 (t, J = 7.2 Hz, 6H), -3.73 (s, 2H)
FABMS m/z 819 (M+H)⁺ calculated for C₄₈H₅₈N₄O₈ = 818

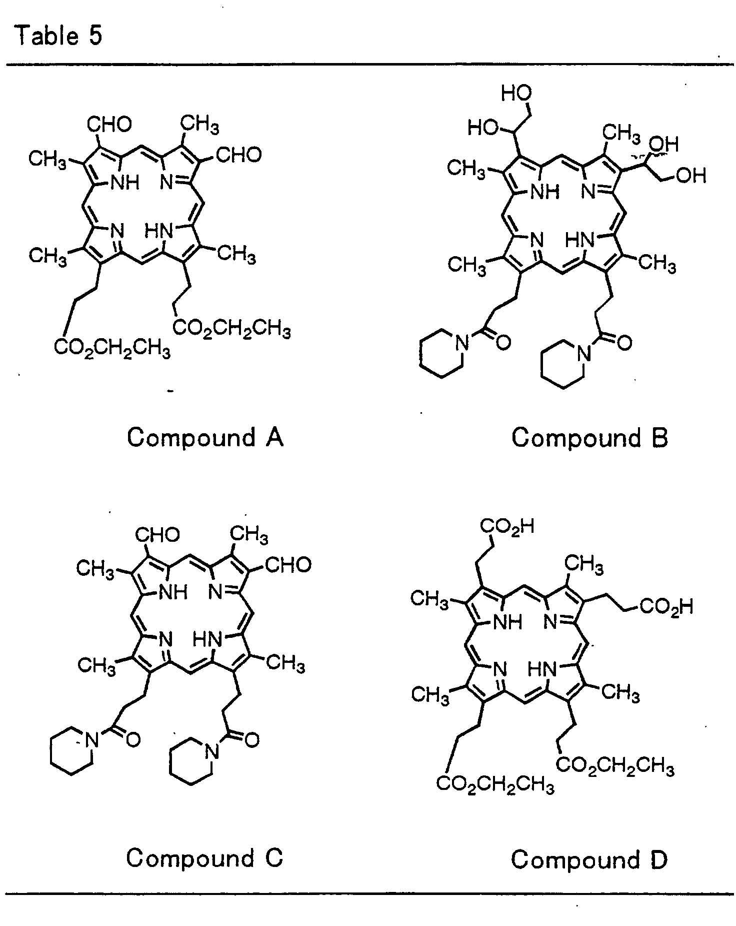

### Formulation Example

According to a usual method, tablets comprising the following composition are prepared.

### Composition per one tablet

| | |
|---|---|
| Compound 1 | 100 mg |
| Lactose | 300 mg |
| Potato starch | 83 mg |
| Hydroxypropyl cellulose | 15 mg |
| Magnesium stearate | 2 mg |
| | 500 mg |

### INDUSTRIAL APPLICABILITY

According to the present invention, there are provided telomerase inhibitors effective for suppressing malignant tumors and novel porphyrin derivatives.

## Claims

1. A telomerase inhibitor comprising, as an active ingredient, a porphyrin derivative represented by formula (I): 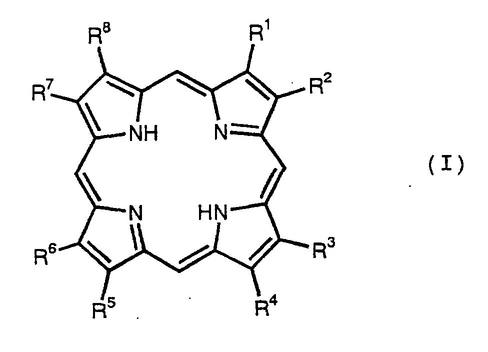 (wherein R¹, R², R³, R⁴, R⁵, R⁶, R⁷ and R⁸ are the same or different and each represents hydrogen, formyl, substituted or unsubstituted lower alkyl, substituted or unsubstituted lower alkenyl, carboxy, substituted or unsubstituted lower alkoxycarbonyl, CONR⁹R¹⁰ (wherein R⁹ and R¹⁰ are the same or different and each represents hydrogen or substituted or unsubstituted lower alkyl), sulfo, lower alkoxysulfonyl or cyano), an *N*-lower alkyl-substituted compound thereof (the lower alkyl of the *N*-lower alkyl-substituted compound is present on the nitrogen atom of the pyrrole ring in the porphyrin ring) or metal-coordinated compound thereof, or a pharmaceutically acceptable salt thereof.

2. An antitumor agent comprising, as an active ingredient, the porphyrin derivative according to claim 1, an N-lower alkyl-substituted compound thereof (the lower alkyl of the N-lower alkyl-substituted compound is present on the nitrogen atom of the pyrrole ring in the porphyrin ring) or metal-coordinated compound thereof, or a pharmaceutically acceptable salt thereof.

3. A telomerase inhibitor comprising, as an active ingredient, an *N*-lower alkyl-substituted compound (the lower alkyl of the *N*-lower alkyl-substituted compound is present on the nitrogen atom of the pyrrole ring in the porphyrin ring) or metal-coordinated compound of the porphyrin derivative according to claim 1, or a pharmaceutically acceptable salt thereof.

4. An antitumor agent comprising, as an active ingredient, an *N*-lower alkyl-substituted compound (the lower alkyl of the *N*-lower alkyl-substituted compound is present on the nitrogen atom of the pyrrole ring in the porphyrin ring) or metal-coordinated compound of the porphyrin derivative according to claim 1, or a pharmaceutically acceptable salt thereof.

5. A porphyrin derivative represented by formula (II): 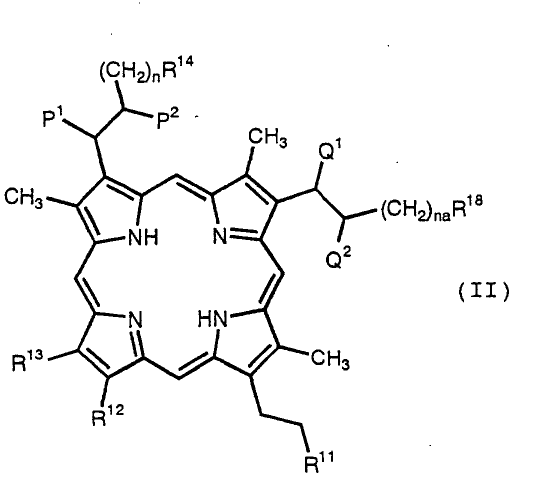 (wherein
n represents an integer of 0 to 10;
na has the same meaning as n described above;
P¹, P², Q¹ and Q² each represents a hydrogen atom, P¹ and P² are combined to represent a bond, or Q¹ and Q² are combined to represent a bond;
R¹¹ represents formyl, CO₂R¹⁵ (wherein R¹⁵ represents hydrogen, substituted or unsubstituted lower alkyl, substituted or unsubstituted lower alkenyl or substituted or unsubstituted aralkyl), CONR¹⁶R¹⁷ (wherein R¹⁶ represents hydrogen, substituted or unsubstituted lower alkyl, substituted or unsubstituted lower alkenyl, substituted or unsubstituted aralkyl, substituted or unsubstituted lower alkoxy, substituted or unsubstituted aryl or substituted or unsubstituted heteroaryl; R¹⁷ represents lower alkyl, substituted or unsubstituted lower alkenyl or substituted or unsubstituted aralkyl, or R¹⁶ and R¹⁷ are combined with the adjacent N to form a heterocyclic group), CH₂NR^{16b}R^{17b} (wherein R^{16b} and R^{17b} are combined with the adjacent N to form a heterocyclic group) or CH₂OR¹⁹ (wherein R¹⁹ represents hydrogen, substituted or unsubstituted lower alkyl, substituted or unsubstituted aralkyl, substituted or unsubstituted lower alkanoyl, substituted or unsubstituted aryl, substituted or unsubstituted aroyl, SO₂R²⁰ (wherein R²⁰ represents substituted or unsubstituted lower alkyl or substituted or unsubstituted aryl) or Si(R²¹)₃ (wherein R²¹'s are the same or different and each represents substituted or unsubstituted lower alkyl or substituted or unsubstituted aryl));
R¹² represents methyl and R¹³ represents (CH₂)₂R²² (wherein R²² has the same meaning as R¹¹ described above), or R¹² represents (CH₂)₂R²³ (wherein R²³ has the same meaning as R¹¹ described above) and R¹³ represents methyl;
R¹⁴ represents formyl, CONR²⁴R²⁵ (wherein R²⁴ and R²⁵ have the same meanings as R¹⁶ and R¹⁷ described above, respectively) or CH₂OR²⁶ (wherein R²⁶ represents substituted or unsubstituted lower alkyl, substituted or unsubstituted aralkyl, substituted or unsubstituted lower alkanoyl, substituted or unsubstituted aryl, substituted or unsubstituted aroyl, SO₂R²⁷ (wherein R²⁷ has the same meaning as R²⁰ described above) or Si(R²⁸)₃ (wherein R²⁸ has the same meaning as R²¹ described above));
R¹⁸ has the same meaning as R¹⁴ described above; and
1) when R¹¹ represents CONR¹⁶R¹⁷ or CH₂OR¹⁹, and R²² represents CONR¹⁶R¹⁷ or CH₂OR¹⁹, or
when R¹¹ represents CONR¹⁶R¹⁷ or CH₂OR¹⁹, and R²³ represents CONR¹⁶R¹⁷ or CH₂OR¹⁹,
R¹⁴ and R¹⁸ are the same or different, and may represent CH₂OH, CO₂R³² (wherein R³² has the same meaning as R¹⁵ described above) or substituted or unsubstituted aryl, and
2) when R¹¹ represents CO₂R¹⁵, and P¹, P², Q¹ and Q² each represents hydrogen, and R²² represents CO₂R¹⁵, or
when R¹¹ represents CO₂R¹⁵, and P¹, P², Q¹ and Q² each represents hydrogen, and R²³ represents CO₂R¹⁵,
R¹⁴ and R¹⁸ are the same or different, and may represent substituted or unsubstituted aryl),
an *N*-lower alkyl-substituted compound thereof (the lower alkyl of the *N*-lower alkyl-substituted compounds is present on the nitrogen atom of the pyrrole ring in the porphyrin ring) or metal-coordinated compound thereof, or a pharmaceutically acceptable salts thereof.

6. A porphyrin derivative represented by formula (IIA): 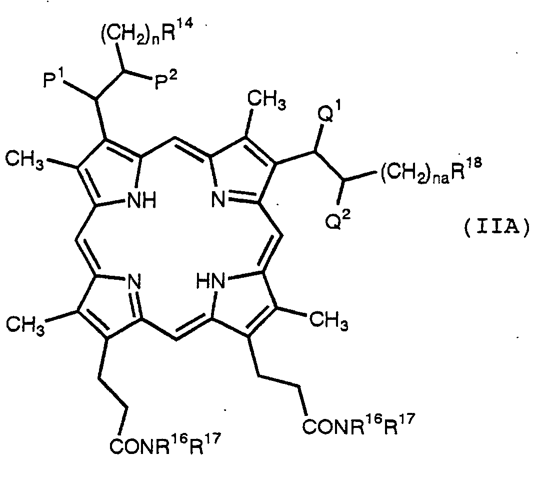 (wherein
n, na, P¹, P², Q¹, Q², R¹⁴, R¹⁶, R¹⁷ and R¹⁸ have the same meanings as described above, respectively)
an *N*-lower alkyl-substituted compound thereof (the lower alkyl of the *N*-lower alkyl-substituted compounds is present on the nitrogen atom of the pyrrole ring in the porphyrin ring) or metal-coordinated compound thereof, or a pharmaceutically acceptable salt thereof.

7. A porphyrin derivative represented by formula (IIB): 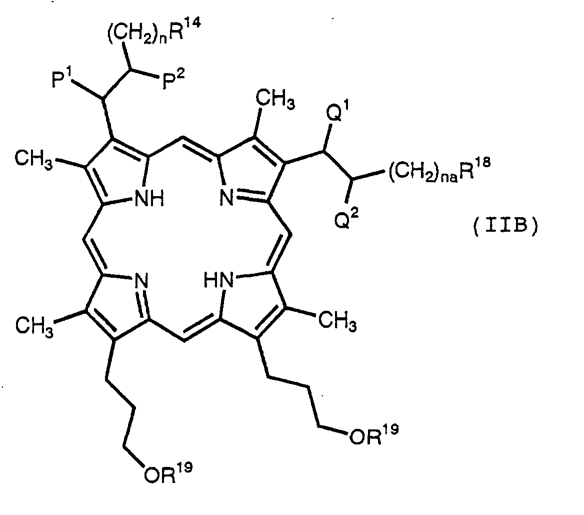 (wherein
n, na, P¹, P², Q¹, Q², R¹⁴, R¹⁸ and R¹⁹ have the same meanings as described above, respectivley)
an *N*-lower alkyl-substituted compound thereof (the lower alkyl of the *N*-lower alkyl-substituted compounds is present on the nitrogen atom of the pyrrole ring in the porphyrin ring) or metal-coordinated compound thereof, or a pharmaceutically acceptable salt thereof.

8. A porphyrin derivative represented by formula (IIC): 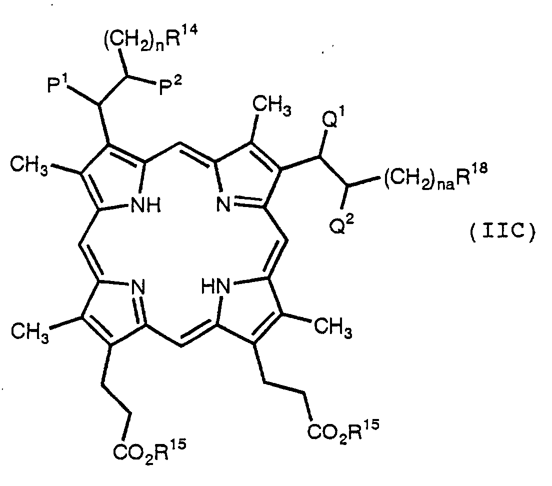 (wherein
n, na, P¹, P², Q¹, Q², R¹⁴, R¹⁵ and R¹⁸ have the same meanings as described above, respectively)
an *N*-lower alkyl-substituted compound thereof (the lower alkyl of the *N*-lower alkyl-substituted compounds is present on the nitrogen atom of the pyrrole ring in the porphyrin ring) or metal-coordinated compound thereof, or a pharmaceutically acceptable salt thereof.

9. A porphyrin derivative represented by formula (III): 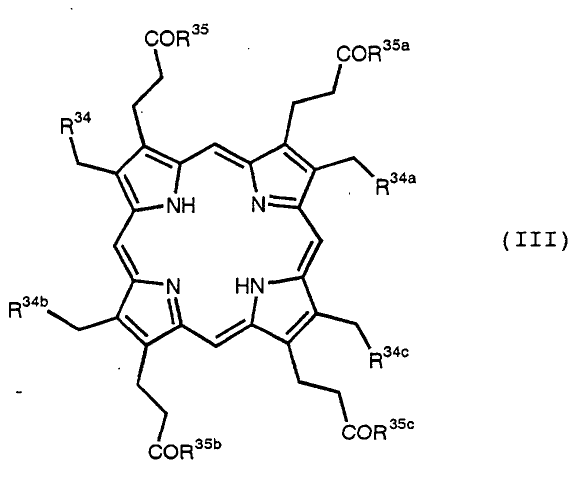 (wherein
R³⁴, R^{34a}, R^{34b} and R^{34c} are the same or different and each represents lower alkyl;
R³⁵, R^{35a}, R^{35b} and R^{35c} are the same or different and each represents OR³⁶ (wherein R³⁶ has the same meaning as R¹⁵ described above) or N(R³⁷)₂ (wherein R³⁷'s are the same or different and each represents hydrogen, substituted or unsubstituted lower alkyl, substituted or unsubstituted lower alkoxy, substituted or unsubstituted aralkyl or substituted or unsubstituted aryl, or two R³⁷'s are combined with the adjacent N to form a substituted or unsubstituted heterocyclic group)),
an *N*-lower alkyl-substituted compound thereof (the lower alkyl of the *N*-lower alkyl-substituted compounds is present on the nitrogen atom of the pyrrole ring in the porphyrin ring) or metal-coordinated compound thereof, a or pharmaceutically acceptable salt thereof.

10. A porphyrin derivative represented by formula (IIIA): 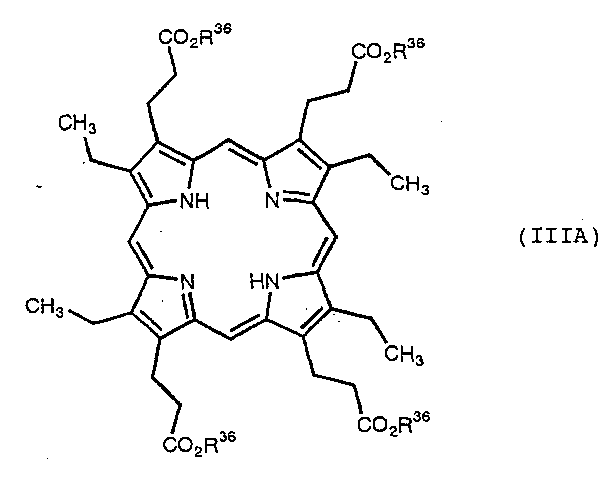 (whereinR³⁶ has the same meaning as described above),
an *N*-lower alkyl-substituted compound thereof (the lower alkyl of the *N*-lower alkyl-substituted compounds is present on the nitrogen atom of the pyrrole ring in the porphyrin ring) or metal-coordinated compound thereof, or a pharmaceutically acceptable salt thereof.

11. A porphyrin derivative represented by any of the following Compound Nos. 65 to 112, an *N*-lower alkyl-substituted compound thereof (the lower alkyl of each *N*-lower alkyl-substituted compounds is present on the nitrogen atom of the pyrrole ring in the porphyrin ring) or metal-coordinated compound thereof, or a pharmaceutically acceptable salt thereof. 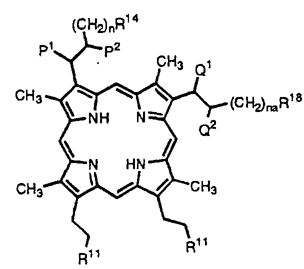 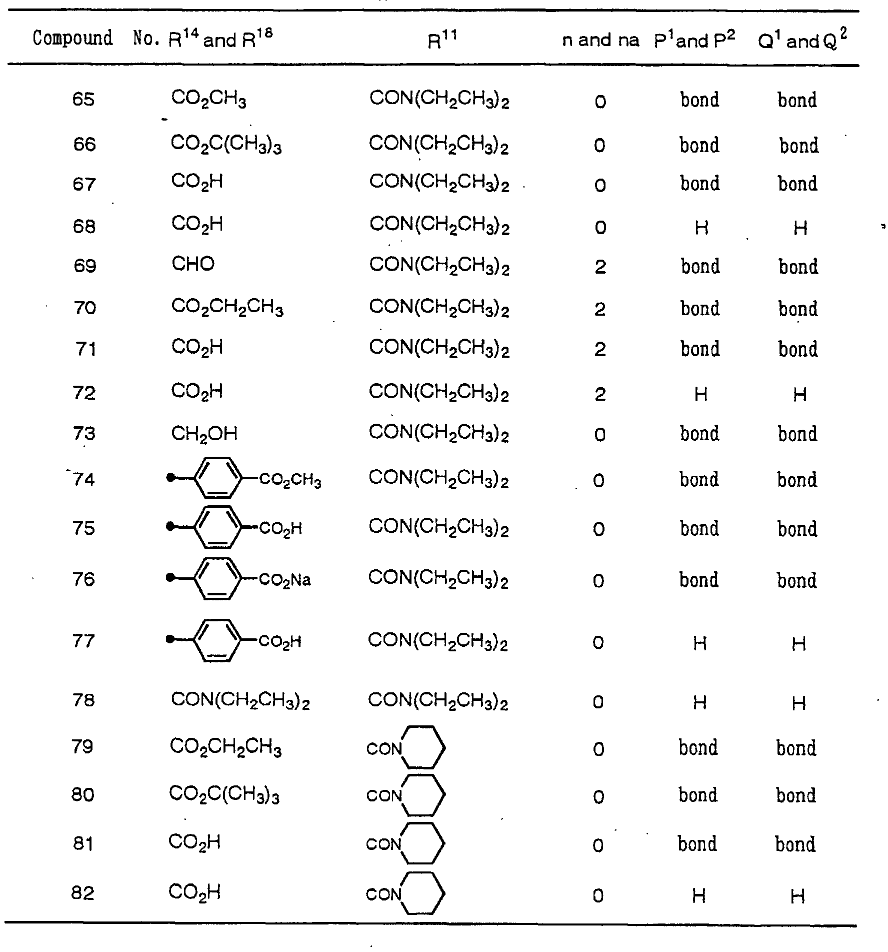 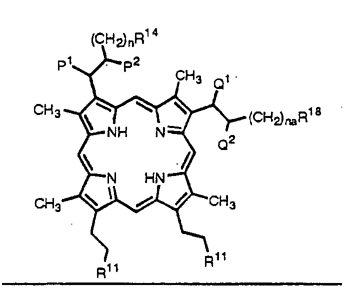 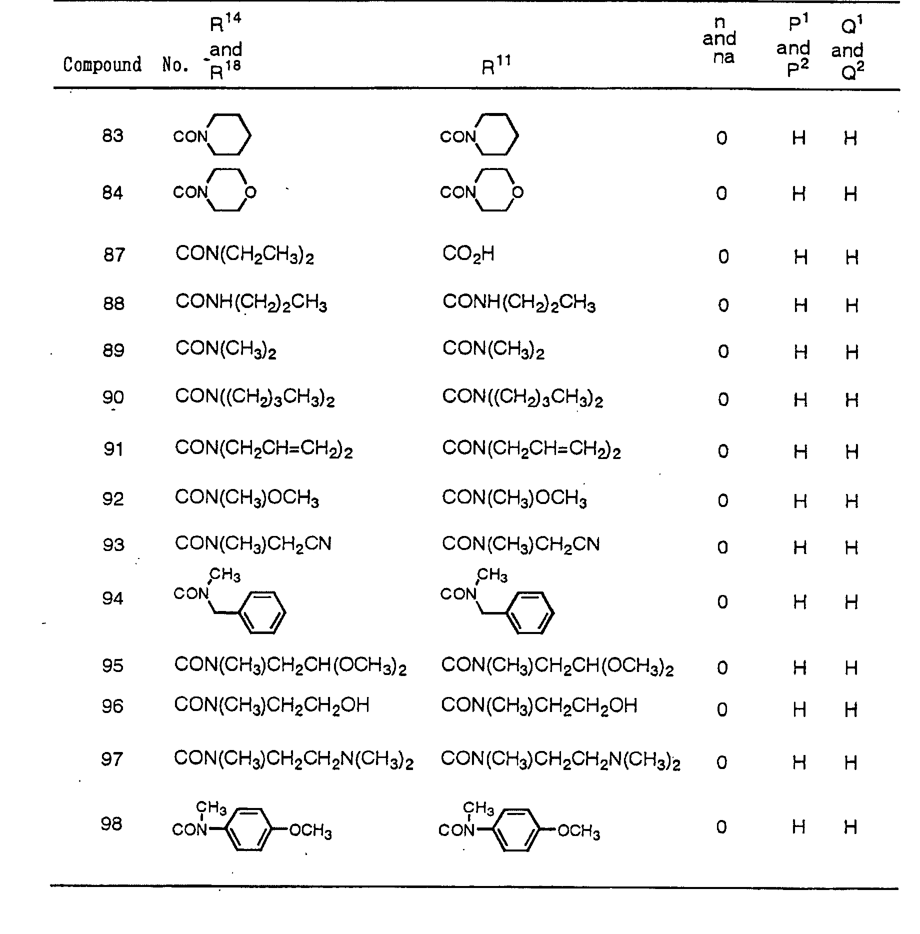 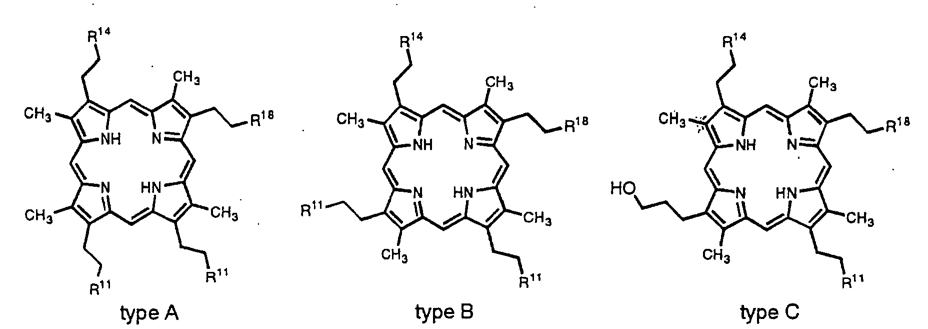 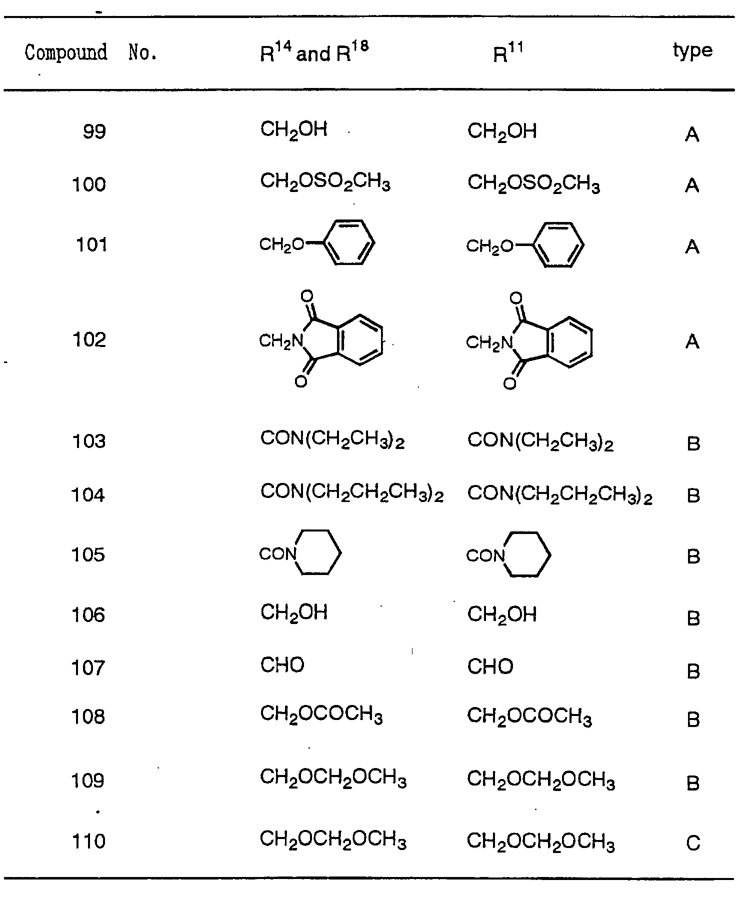 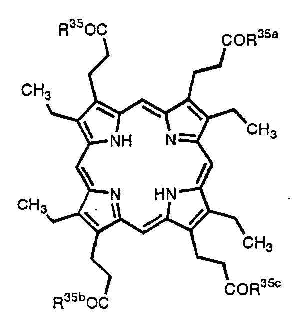
| Compound No. | R³⁵ and R^{25a} and R^{35b} and R^{35c} |
|---|---|
| 111 | OCH₃ |
| 112 | ONa |

12. A pharmaceutical composition comprising at least one of the porphyrin derivative according to any one of claims 5 to 11, an *N*-lower alkyl-substituted compound thereof (the lower alkyl of the *N*-lower alkyl-substituted compound is present on the nitrogen atom of the pyrrole ring in the porphyrin ring) or metal-coordinated compound thereof, or a pharmaceutically acceptable salt thereof.

13. Use of a porphyrin derivative represented by formula (I) according to claim 1, an *N*-lower alkyl-substituted compound thereof (the lower alkyl of the *N*-lower alkyl-substituted compounds is present on the nitrogen atom of the pyrrole ring in the porphyrin ring) or metal-coordinated compound thereof, or a pharmaceutically acceptable salt thereof for the manufacture of a telomerase inhibitor.

14. Use of a porphyrin derivative represented by formula (I) according to claim 1, an *N*-lower alkyl-substituted compound thereof (the lower alkyl of the *N*-lower alkyl-substituted compounds is present on the nitrogen atom of the pyrrole ring in the porphyrin ring) or metal-coordinated compound thereof, or a pharmaceutically acceptable salt thereof for the manufacture of an antitumor agent.

15. A method for inhibiting telomerase, comprising a step of administering an effective amount of a porphyrin derivative represented by formula (I), an *N*-lower alkyl-substituted compound thereof (the lower alkyl of the *N*-lower alkyl-substituted compound is present on the nitrogen atom of the pyrrole ring in the porphyrin ring) or metal-coordinated compound thereof, or a pharmaceutically acceptable salt thereof to a mammalian animal including human.
